(19) **Europäisches Patentamt**

**European Patent Office**

**Office européen des brevets**

(11) **EP 1 477 571 A1**

(12) **EUROPEAN PATENT APPLICATION**

(43) Date of publication:
**17.11.2004 Bulletin 2004/47**

(51) Int Cl.⁷: $C12Q\ 1/68$

(21) Application number: **04010205.5**

(22) Date of filing: **29.04.2004**

(84) Designated Contracting States:
**AT BE BG CH CY CZ DE DK EE ES FI FR GB GR HU IE IT LI LU MC NL PL PT RO SE SI SK TR**
Designated Extension States:
**AL HR LT LV MK**

(30) Priority: **16.05.2003 US 439767**

(71) Applicant: **Eppendorf AG**
**22339 Hamburg (DE)**

(72) Inventors:
• **Remacle, José**
  **5020 Malonne (BE)**

• **de Longueville, Francoise**
  **5360 Natoye (BE)**
• **Zammatteo, Nathalie**
  **5024 Gelbressée (BE)**
• **Toussaint, Olivier**
  **5020 Vedrin (BE)**
• **Van Huffel, Christophe**
  **1170 Watermael-Boitsfort (BE)**

(74) Representative: **Becker Kurig Straus**
**Patentanwälte**
**Bavariastrasse 7**
**80336 München (DE)**

(54) **Determination of a general three-dimensional status of a cell by multiple gene expression analysis on micro-arrays**

(57) The invention provides a tool for the easy interpretation of the changes occurring in a cell, being a three dimensional complex and control system, by analyzing a limited number of data obtained by quantifying the intensity of the signals present on spots distributed in a two dimensional surface. These signals intensities are related to the level of genes or gene products present in the cells and after processing and data analysis, they provide an absolute or relative quantification of these genes and gene products present in the analyzed cell or tissue or organisms. The invention also provides a list of cellular functions, which are essential in order to obtain an overview of the modifications occurring in the vital or specific cellular functions under specific biological conditions.

**EP 1 477 571 A1**

**Description**

**Field of the invention**

The present invention relates to the field of analyzing gene expression changes occurring in cells under particular conditions which allows to obtain a global overview of the modifications occurring in cells main vital cellular functions optionally in combination with some specific functions. In particular, this invention pertains to a method and a kit for the quantitative determination of the overall cellular status of a cell. In particular, this invention relates to a method for the determination of the three dimensional status of a cell, wherein an array containing nucleic acids or proteins belonging to or being representative for at least 9 specific vital cellular functions, which functions being represented on the array by at least 4 genes or proteins, is contacted with a sample derived from a particular cell of interest, wherein the pattern obtained by the binding of the sample to the spots is indicative of the cellular status.

**Description of the related art**

Biological Research in general tries to obtain a general overview of the cells performance/status either in a given time period and/or under specific conditions. Since cells represent three dimensional structures it is difficult to obtain such an overview.

So far use of confocal microscopes allowed to obtain a two dimensional picture of a cellular macroscopic condition only. When obtaining several pictures/representations at different levels of the cellular structure, a rough three-dimensional structural picture could be obtained by reconstituting 10 to 30 sections. Proceeding accordingly, however, only allows to study a restricted number of cell components.

Another two-dimensional representation of cellular functions is presented in terms of cellular pathways, which are consecutive chains of reactions linked to each other in a complex and well regulated network. The pathways show a particular cellular process indicating the proteins/factors involved. So far a number of metabolic pathways and other specific cell functions like apoptosis, cell cycle or signal transduction activation have been elucidated.

With the development of molecular biology, research has focused more and more on the genetic level, which eventually leads to the sequencing of genomes of a number of different organisms. Cells of a given organism contain in their genome the information for all the genes which are necessary for the activity and various functions either specific or non specific of a given cell or tissue. In general, the genes encode proteins having specific activities in the cells, such as enzymes. These enzymes are normally part of cellular pathways in the cells, which cells are three dimensional structures composed of various compartments, e.g. the cytoplasm, the nucleus, the mitochondria, lysososmes, endoplasmic reticulum, the Golgi apparatus, the peroxisomes, the chloroplasts. The network of reactions is embricated into this three dimensional structure.

Yet, not all of the genes present are actually expressed or used by the cells. Usually, cells having the same differentiation pattern express the same genes, but the expression changes with the time and also with the cellular environment. E.g., in cells some genes are activated or expressed only at a specific time, at a specific level, at a specific developmental stage, and/or in a specific cellular, physiologic, and/or tissue context. In eukaryotic organisms (i. e., those having a nucleus) the number of individual genes transcribed is typically in the range of between ten and twenty thousands of genes.

For this reason, determining when a gene is expressed, and what causes the gene to be expressed, may be a key issue in better understanding the effects of various stimuli on cellular responses. In addition, determining at which time point or during which time period a gene is expressed may yield a better understanding of the effects of various normal or variant genes on disease pathogenesis.

It is known that many biological functions are accomplished by altering the expression of genes through transcriptional (e.g. through control of initiation, provision of RNA precursors, RNA processing, etc.) and/or translational control. E.g., fundamental biological processes such as cell cycle, cell differentiation and cell death, are often characterized by variations in the expression levels of groups of genes. Gene expression is also known to be associated with pathogenesis. For example, the lack of sufficient expression of functional tumor suppressor genes and/or the over expression of oncogene/protooncogenes could lead to oncogenesis (Marshall, Cell, 64: 313-326 (1991); Weinberg, Science, 254: 1138-1146 (1991), incorporated herein by way of reference). Thus, changes in the expression levels of particular genes (e.g. oncogenes or tumor suppressors) serve as signposts for the presence and progression of various diseases.

So far, the study of gene expression generally focused on regulatory regions of the gene of interest and on the relation among a limited number of genes only. However, the expression of a particular gene is in most of the cases controlled by the expression of a large number of other genes. The expression of those regulatory genes may also be under the control of additional genes creating a complex network within the cell.

Therefore, there is a need in the art to develop a systematic approach to understand the complex regulatory relationships among large numbers of genes.

A contemporary methodology for analyzing simultaneously a plurality of genes for gene expression levels utilizes nucleic acid arrays (or micro-arrays or macro-arrays, hereinafter collectively referred to as arrays). DNA-arrays typically consist of hundreds to thousands of immobilized DNA sequences present on a surface of an object the size of a business card or smaller. Although many different micro-array systems have been developed, the most commonly used systems today can be divided into two groups, according to the arrayed material: complementary DNA (cDNA) and oligo-nucleotide micro-arrays. The probes in a cDNA-array are long polynucleotides usually synthesized as PCR products generated from cDNA libraries. They are printed with a robot onto glass slides, nylon filters, glass, plastic, as spots at defined locations. Spots are typically in the range of 100-400 μm in size and are spaced about the same distance apart. Labeled probe samples are prepared from RNA from biological samples. The probes are hybridized to the immobilized nucleic acids on the arrays, and a detector instrument collects the intensities of hybridization of the bound labeled probe sample to the individual gene sequences.

Oligonucleotide arrays are a series of small nucleotides present on the arrays either by physical spotting or by *in situ* chemical synthesis directly on the support. The gene expression analysis on such oligo-arrays is possible after cutting the cDNA or corresponding amplified RNA into pieces and analysis of these pieces on several oligonucleotides. For each gene, a positive detection is the result of a pattern of positive answers on several oligonucleotides together with negative results on the corresponding control oligonucleotides. Arrays are either developed as low (or medium) density micro-arrays having different spot number lower than 1000 (or 3000) or as high density micro-arrays.

Some low density micro-arrays have been developed for the analysis of specific changes in cells especially associated with cancer prognosis and diagnostics. Low density arrays bearing selected genes have been used so far in very specific applications.

In this respect, WO0246467 describes a polynucleotide library representing 176 genes useful in the molecular characterization of primary breast carcinomas. Also, US-P-6,183,968 describes an array comprising 134 polynucleotide probes encoding receptors and proteins associated with cell proliferation. This micro-array is described to be usable in the diagnosis and treatment of cancer, in immuno-pathology and neuropathology.

High-density arrays may be used to investigate problems in cell biology and to cover a much larger range of cellular changes in one particular condition. Even though high density arrays were used to generate long lists of genes with altered expression, they did not provide information, as to which of these changes are important or meaningful in establishing a given phenotype.

Many studies have emerged from experiments on high-density arrays.

In WO0228999 a high density array is used to identify the changes in gene expression associated with activation of granulocytes. Also WO0229103 describes such an array to identify the changes in gene expression associated with liver cancer by examining gene expression in tissue from normal liver, metastatic malignant liver and hepatocellular carcinoma.

In WO0177389 another high density array is used to select polynucleotides, which are differentially expressed during foam cell development and which are associated with atherosclerosis.

WO0194629 describes a process for assaying potential anti-tumor agents based on the modulation of the expression of specified genes on micro-array bearing 8447 polynucleotide sequences.

US-P-02048763 16,834 unique human genome-derived single exon probes have been identified, useful for gene expression analysis by micro-array. The derived micro-array has been used to determine genes expressed at significant levels in various tissues like brain, heart, liver, fetal liver, placenta, lung, bone marrow.

One of the objective of using high density gene arrays is to reconstitute the interconnection or communication diagram between all genes in a given cell or sample ("the genes wiring diagram"). While this is a fundamental question of greatest scientific interest, practically, this approach alone can only detect the changes for a relatively limited number of genes whose expression (and therefore level of mRNA) changed during the biological process (treatment or disease progression). Additionally, the degree of complexity of such a pieced together network (of typically more than 30.000 genes for mammals) poses a great problem given the large dimensional space imposed by such a large number of genes.

Practically, such experimentally derived "gene wiring diagram" is only reflecting the interrelations or interactions between transcriptionally regulated genes and is not taking into account the vast knowledge base derived from biochemistry (enzyme activity and regulation) and structural biochemistry (macromolecular interactions) and offers therefore only a blurry representation of the integration of vital and/or specific functions within a cell or sample.

At present, there is no current tool for providing researchers, clinicians, pharmacists and in a simple process with the essential information about the changes occurring in a cell in a particular condition. Thus, a problem of the present invention is to provide a means or tool, which generates a clear and reliable picture of the cell behavior in a particular condition by quantitative analysis of specific genes or gene products expressed in a particular biological condition.

## Description of the invention

The above problem has been solved by providing a method for a quantitative determination of the overall status of cell(s), which method comprises the steps of providing an array containing on predetermined locations thereof a maximum of 2999 nucleic acids or proteins belonging to or being representative for at least 9 of the following vital cellular functions: apoptosis, cell adhesion, cell cycle, growth factors and cytokines, cell signaling, chromosomal processing, DNA repair/synthesis, intermediate metabolism, extracellular matrix, cell structure, protein metabolism, oxidative metabolism, transcription and house keeping genes, said functions being represented by at least 4 genes or proteins, contacting a sample derived from a particular cell of interest with the array, detecting whether and where binding of the sample to any of the predetermined locations on the array occurred, and optionally quantifying the intensity of the spots detected, wherein the pattern and/or the intensity of the binding events is indicative for a particular cellular status.

In effect, the present invention allows to obtain a quantitative overview of the modifications occurring in cells via an analysis on a two dimensional surface of an array of the intensity of a limited number of signals correlated with gene expression or its products involved in or characteristic for at least 9 of the above mentioned main vital cellular functions. It has been found that this overall assessment of the at least 9 vital functions allows to reconstitute the relationships of these essential functions either between themselves or related to other specific functions.

According to another embodiment the present invention provides a method for a quantitative determination of a general cellular status of cell(s) comprising the steps of, providing an array, containing on predetermined locations thereof nucleic acids or proteins belonging to or representative for at least 5 of the following vital cellular functions: apoptosis, cell adhesion, cell cycle, growth factors and cytokines, cell signaling, chromosomal processing, DNA repair/synthesis, intermediate metabolism, extracellular matrix, cell structure, protein metabolism, oxidative metabolism, transcription and house keeping genes and at least one nucleic acid or protein, belonging to or representative for at least one of the following specific functions: cell differentiation, oncogene/tumor suppressor, stress response, lipid metabolism, proteasome, circulation, wherein the array comprises at least 20 different spot compositions and a maximum of 2999 different spots, contacting a sample derived from a cell of interest with the array, detecting and/or quantifying the intensity of spots present on an array specific of the expression of, said method comprising the steps of comparing the transcriptome of cells or tissues in the given biological condition with at least one reference or control condition.

## Description of the drawings

Fig. 1 is a schematic presentation of the pattern of a micro-array (Generalchip) for gene expression analysis of the main vital cellular functions (202 genes) with the appropriate controls.
Fig. 2 is a schematic presentation of the pattern of a micro-array (Senechip) for gene expression analysis of the vital cellular functions together with the genes associated with aging and stresses (239 genes) with the appropriate controls.
Fig. 3 shows a general pathway of apoptosis.
Fig. 4 shows a general pathway of the cell cycle.
Fig. 5 shows genes with statistically significant transcript level changes (circled).

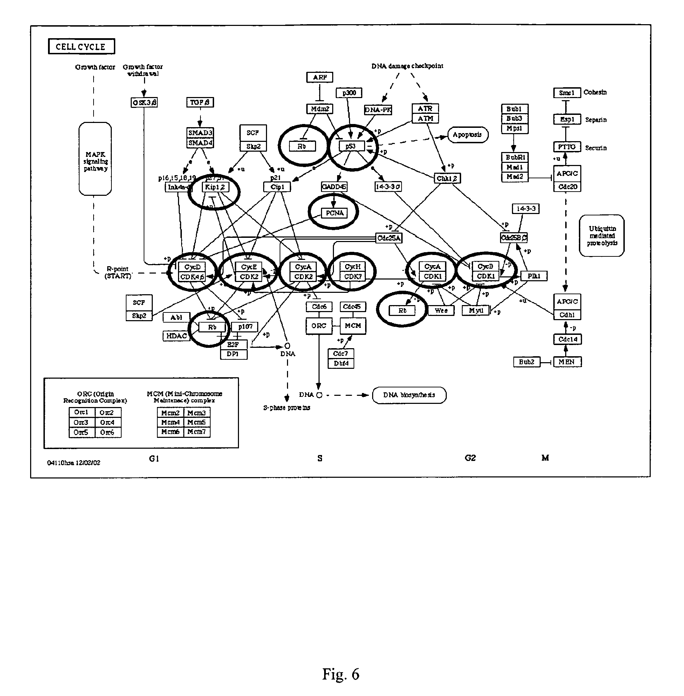

Fig. 6 shows the integration of this information in the cell cycle genes.

Table 1 is a list of the genes on the 2D array classified according to their vital or specific functions. The table also provides the Genbank accession number and one reference for each gene.
Table 2 presents the values of genes expression which are statistically significant in the study of either the cell vital functions in the "Generalchip" or in association with the stress and aging process on the Senechip.

## Definitions

In the present invention, the term cell "vital function" or "vital cellular function" designates a cellular function which is essential for the life, division and growth of cells. Examples of such vital functions are in general selected from the group comprising apoptosis, cell adhesion, cell cycle, growth factors and cytokines, cell signaling, chromosomal processing, DNA repair/synthesis, intermediate metabolism, extracellular matrix, cell structure, protein metabolism, oxidative metabolism, transcription and house keeping genes. More detailed examples for vital functions are listed in table 1.
The term "specific function" refers to any cellular function which is present in some mammalian cell types only but not in others. General examples for such functions are functions determining/controlling the differentiation status of a cell, or functions which are activated or expressed under given conditions only, including pathological conditions, drug treatment, chemical or physical modification of the environment. Illustrative examples are functions for cell differentiation, oncogene/tumor suppressor, stress response, lipid metabolism, proteasome, circulation, with more specific examples may be derived from table 1.

The term "expressed genes" are those regions of the genomic DNA which are transcribed into mRNA and optionally then translated into (poly-)peptides or proteins. According to the present invention the determination of expressed genes may be performed on either molecules, specifically via detection of the mRNA or of the (poly-)peptide or protein (which latter terms will be used hereinafter interchangeably). The determination may also be based on a specific property of the protein, e.g. its enzymatic activity.

The terms "nucleic acid, array, probe, target nucleic acid, bind substantially, hybridizing specifically to, background, quantifying" are as described in WO97/27317, which is incorporated herein by way of reference. In particular, the term "array" means a given number of capture probes being immobilized on support. The most common arrays are composed of capture probes being present in predetermined locations on a single support being or not a substrate for their binding. However, capture probes being present on multiple supports are also considered as arrays, as long as the different target molecules can be individually detected and/or quantified.

The term "nucleotide triphosphate" refers to nucleotides present in either DNA or RNA and thus includes nucleotides which incorporate adenine, cytosine, guanine, thymine and uracil as bases, the sugar moieties being deoxyribose or ribose. Other modified bases capable of base pairing with one of the conventional bases adenine, cytosine, guanine, thymine and uracil may be employed. Such modified bases include for example 8-azaguanine and hypoxanthine.

The term "nucleotide" as used herein refers to nucleosides present in nucleic acids (either DNA or RNA) combined with the bases of said nucleic acid, and includes nucleotides comprising usual or modified bases as above described.

References to nucleotide(s), polynucleotide(s) and the like include analogous species wherein the sugarphosphate backbone is modified and/or replaced, provided that its hybridization properties are not destroyed. By way of example the backbone may be replaced by an equivalent synthetic peptide, called Peptide Nucleic Acid (PNA).

"Polynucleotide" sequences that are complementary to one or more of the genes described herein, refer to polynucleotides that are capable of hybridizing under stringent conditions to at least part of the nucleotide sequence of said genes. Polynucleotides also include oligonucleotides which can be used in particular conditions. Such hybridizable polynucleotides will typically exhibit at least about 75% sequence identity at the nucleotide level to said genes, preferably about 80% or 85% sequence identity or more preferably about 90% or 95% or more nucleotide sequence identity to said genes. They are composed of either small sequences typically 15-30 base long or longer ones being between 30 and 100 or even longer between 100 and 300 base long.

The terms "capture probe" relates to a molecule capable to specifically bind to a given polynucleotide or polypeptide. Polynucleotide binding is obtained through base pairing between two polynucleotides, one being the immobilized capture probe and the other one the target to be detected. Polypeptide binding is best performed using antibodies specific of the polypeptide for the capture of a given polypeptide or protein. Part of the antibodies, or recombinant proteins incorporating part of the antibodies, typically the variable domains, or even proteins being able to specifically recognize the peptide can also be used as capture probe. The term "capture probes" in the sense of the present invention shall designate genes or parts of genes of different lengths, e.g. between 10 and 1500 nucleotides, which are either synthesized chemically in situ on the surface of the support or laid down thereon. Moreover, this term shall also designate polypeptides or fragments thereof, or antibodies directed to particular polypeptides, which terms are used interchangeably, attached or adsorbed on the support.

The term "single capture nucleotide species" is a composition of related nucleotides for the detection of a given sequence by base pairing hybridization; nucleotides are synthesized either chemically or enzymatically but the synthesis is not always perfect and the main sequence is contaminated by other related sequences like shorter ones or sequences differing by one or a few nucleotides. The essential characteristic of one nucleotide species for the invention is that the overall species can be used for capture of a given sequence belonging to a given gene.

The "hybridized nucleic acids" are typically detected by detecting one or more "labels" attached to the sample nucleic acids. The labels may be incorporated by any of a number of means well known to those skilled in the art, such as detailed in WO 99/32660, which is incorporated herein by way of reference.

## Detailed description of the invention

Accordingly, the present invention provides a tool for the analysis of an overall status/performance of a cell on the basis of the changes occurring in at least 9 cellular vital functions under the respective biological condition.

In another embodiment, the present inventors provide a tool for the analysis of an overall status/performance of a cell on the basis of changes occurring in at least 5 vital functions considering the change in at least 1 specific function of the particular cell.

According to another embodiment the present invention also provides a kit for carrying out the present methods, while the method and the kit being suitable for analyzing gene expression changes occurring in particular conditions a cell is subjected. These conditions may be for example overall or cellular physiological events, such as stress, ageing, stem cell differentiation, haematopoiesis, a particular neuronal functional status, diabetes, obesity, transformation process such as carcinogenesis, protein turnover or circulatory disorders as atherosclerosis.

Accordingly, it has been found that in order to obtain a picture of a cellular status/performance essentially not all of the cellular genes/gene-products have to be investigated but only some genes or gene products associated with vital functions of the cell which direct cell behavior and optionally together with genes associated with specific functions of the cell. The main vital and specific functions and the characteristic genes which have to be analyzed are describe here after

## A) Vital functions

## 1. Apoptosis

Life requires death. Elimination of unwanted cells is vital for embryogenesis, metamorphosis and tissue turnover, as well as for the development and function of the immune system. For this reason, mammalian development is tightly regulated not only by the proliferation and differentiation of cells but also by cell death. The cell death that occurs during development or tissue turnover is called programmed cell death, most of which proceeds via apoptosis. Apoptosis is morphologically distinguished from necrosis, which occurs during the accidental cell death caused by physical or chemical agents. During apoptosis, the cytoplasm of the affected cells condenses, and the nucleus also condenses and becomes fragmented. At the final stage of apoptosis, the cells themselves are fragmented (apoptotic bodies) and are phagocytosed by neighboring macrophages and granulocytes.

Apoptosis or programmed cell death is triggered by a variety of stimuli, including cell surface receptors like FAS, the mitochondrial response to stress, and factors released from cytotoxic T cells. It constitutes a system for the removal of unnecessary, aged, or damaged cells that are regulated by the interplay of proapoptotic and antiapoptotic proteins of the Bcl-2 family. The proapoptotic proteins Bax, Bad, Bid, Bik, and Bim contain an $\alpha$-helical BH3 death domain that fits the hydrophobic BH3 binding pocket on the antiapoptotic proteins Bcl-2 and Bcl-$X_L$, forming heterodimers that block the survival-promoting activity of Bcl-2 and Bcl-$X_L$. Thus, the relative abundance of proapoptotic and antiapoptotic proteins determines the susceptibility of the cell to programmed death. The proapoptotic proteins act at the surface of the mitochondrial membrane to decrease the mitochondrial trans-membrane potential and promote leakage of cytochrome c. In the presence of dATP cytochrome c complexes with and activates Apaf-1. Activated Apaf-1 binds to downstream caspases, such as procaspase-9, and processes them into proteolytically active forms. This begins a caspase cascade resulting in apoptosis.

The caspases comprise a class of cysteine proteases many members of which are involved in apoptosis. The caspases convey the apoptotic signal in a proteolytic cascade, with caspases cleaving and activating other caspases that subsequently degrade cellular targets that lead to cell death. The activating caspases include caspase-8 and caspase-9. Caspase-8 is the initial caspase activated in response to receptors with a death domain that interacts with FADD. The mitochondrial stress pathway begins with the release of cytochrome c from mitochondria, which then interacts with Apaf-1, causing self-cleavage and activation of caspase-9. The effector caspases, caspase-3, -6 and-7 are downstream of the activator caspsases and act to cleave various cellular targets. Granzyme B and perform, proteins released by cytotoxic T cells, induce apoptosis in target cells by forming transmembrane pores and triggering apoptosis, perhaps through cleavage of caspases. Caspase independent mechanisms of granzyme B-mediated apoptosis have been suggested.

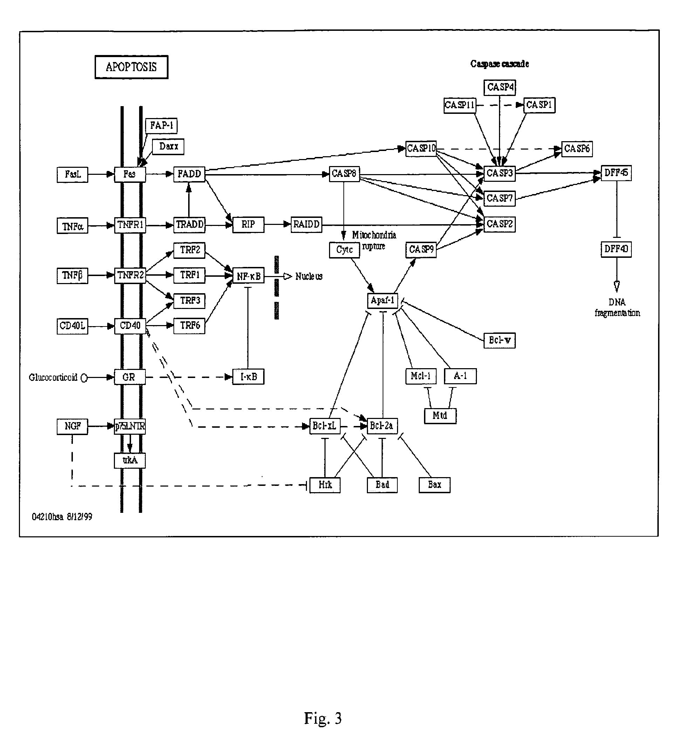

A general pathway of apoptosis is illustrated in Fig. 3.

According to the present invention 10 genes (or proteins, respectively) found to represent checkpoint genes in apoptosis were selected as preferred representatives of the apoptosis pathway. They comprise: bad, bax, bcl2, bclx, bid, casp2, casp3, casp7, casp8 and casp9 and are listed in table 1.

## 2. Cell adhesion

Direct interactions between cells, as well as between cells and the extracellular matrix, are critical for the development and function of multicellular organisms. Some cell-cell interactions are transient, such as the interactions between cells of the immune system and the interactions that direct white blood cells to sites of tissue inflammation. In other cases, stable cell-cell junctions play a key role in the organization of cells in tissues. For example, several different types of stable cell-cell junctions are critical to the maintenance and function of epithelial cell sheets.

To form an anchoring junction, cells must first adhere. A bulky cytoskeletal apparatus must then be assembled around the molecules that directly mediate the adhesion. The result is a well-defined structure - a desmosome, a hemidesmosome, or an adherents or septate junction. In the early stages of development of a cell junction, however, before the cytoskeletal apparatus has assembled, and especially in embryonic tissues, the cells often adhere to one another without clearly displaying these characteristic structures. Many simple tissues, including most epithelia, derive from precursor cells whose progeny are prevented from wandering away by being attached to the extracellular matrix or to other cells or to both. But the cells, as they accumulate, do not simply remain passively stuck together as a disorderly pile; instead, the tissue architecture is actively maintained by selective adhesions that the cells make and progressively adjust. Thus, if cells of different embryonic tissues are artificially mingled, they will often spontaneously sort out to restore a more normal adhesion organization.

Cell-cell adhesion is a selective process, such that cells adhere only to other cells of specific types. Such selective cell-cell adhesion is mediated by transmembrane proteins called cell adhesion molecules, which can be divided into four major groups: the selectins, the integrins, the immunoglobulin (Ig), and the cadherins.

The selectins mediate transient interactions between leukocytes and endothelial cells or blood platelets. There are three members of the selectin family: L-selectin, which is expressed on leukocytes; E-selectin, which is expressed on endothelial cells; and P-selectin, which is expressed on platelets. The selectins recognize cell surface carbohydrates. One of their critical roles is to initiate the interactions between leukocytes and endothelial cells during the migration of leukocytes from the circulation to sites of tissue inflammation. The selectins mediate the initial adhesion of leukocytes to endothelial cells. This is followed by the formation of more stable adhesions, in which integrins on the surface of leukocytes bind to intercellular adhesion molecules (ICAMs), which are members of the Ig superfamily expressed on the surface of endothelial cells. The firmly attached leukocytes are then able to penetrate the walls of capillaries and enter the underlying tissue by migrating between endothelial cells.

The major cell surface receptors responsible for the attachment of cells to the extracellular matrix are the integrins. More than 20 different integrins, formed from combinations of subunits, have been identified. The integrins bind to short amino acid sequences present in multiple components of the extracellular matrix, including collagen, fibronectin, and laminin.

In addition to attaching cells to the extracellular matrix, the integrins serve as anchors for the cytoskeleton. The resulting linkage of the cytoskeleton to the extracellular matrix is responsible for the stability of cell-matrix junctions. Distinct interactions between integrins and the cytoskeleton are found at two types of cell-matrix junctions, focal adhesions and hemidesmosomes. Focal adhesions attach a variety of cells, including fibroblasts, to the extracellular matrix.

Cells dissociated from various tissues of vertebrate embryos preferentially reassociate with cells from the same tissue when they are mixed. This tissue-specific recognition process in vertebrates is mainly mediated by a family of $Ca^{2+}$-dependent cell-cell adhesion proteins called cadherins, which hold cells together by a homophilic interaction between transmembrane cadherin proteins on adjacent cells. In order to hold cells together, the cadherins must be attached to the cortical cytoskeleton. Most animal cells also have $Ca^{2+}$-independent cell-cell adhesion systems that mainly involve members of the immunoglobulin superfamily, which includes the neural cell adhesion molecule N-CAM. As even a single cell type uses multiple molecular mechanisms in adhering to other cells (and to the extracellular matrix), the specificity of cell-cell adhesion seen in embryonic development must result from the integration of a number of different adhesion systems, some of which are associated with specialized cell junctions while others are not.

In this invention, 21 genes (or proteins) are included into the array analysis as characteristic of the cell adhesion pathway (table 1). They comprise: CATB1, CD36, CDH1, CDH5, CDH11, CDH13, DSG1, ICAM1, ITGA4, ITGA5, ITGA6, ITGB1, ITGB2, ITGB3, PECAM1, SELE, SELL, RANTES, TSP1, TSP2 and VCAM1.

### 3. The Cell Cycle

Cell division is the fundamental process by which all living organism grow, repair, and reproduce. In unicellular organisms, each cell division doubles the number of organisms; and in multicellular species, many rounds of cell division are required to produce a new organism or to replace cells lost by wear and tear or by programmed cell death.

In proliferating cells, the cell cycle consists of four phases. Gap 1 (G1) is the interval between mitosis and DNA replication that is characterized by cell growth. The transition that occurs at the restriction point (R) in G1 commits the cell to the proliferative cycle. If conditions that signal this transition are not present, the cell exits the cell cycle and enters G0, a non-proliferative phase during which growth, differentiation and apoptosis occur. Replication of DNA occurs during the synthesis (S) phase, which is followed by a second gap phase (G2) during which growth and preparation for cell division occurs. Mitosis and the production of two daughter cells occur in M phase.

Passage through the four phases of the cell cycle is controlled by a family of cyclins that act as regulatory subunits for cyclin-dependent kinases (cdks). The activity of the various cyclin/cdk complexes that regulate the progression through G1 -S-G2 phases of the cell cycle is controlled by the synthesis of the appropriate cyclins during a specific phase of the cell cycle. The cyclin/cdk complex is then activated by the sequential phosphorylation and dephosphorylation of the key residues of the complex, located principally on the cdk subunits.

The cyclin cdk complex of early G1 is either cdk2, cdk4, or cdk6 bound to a cyclin D isoform. There are several proteins that may inhibit the cell cycle in G1. If DNA damage occurred, p53 accumulates in the cell and induces the p21-mediated inhibition of cyclin D/cdk. Mdm2, by facilitating the nuclear export/inactivation of p53, becomes part of an inhibitory feedback loop that inactivates p21-mediated G1 arrest. Similarly, activation of TGF-$\beta$ receptors induces the inhibition of cyclin D/cdk by p15, while cyclic-AMP inhibits the cyclin D/cdk complex via p27. If the cyclin D/cdk complex is inhibited, retinoblastoma protein (Rb) is in a state of low phosphorylation and is tightly bound to the transcription factor E2F, inhibiting its activity.

Passage through the restriction point and transition to S phase is triggered by the activation of the cyclin D/cdk complex, which phosphorylates Rb. Phoshporylated Rb dissociates from E2F, which is then free to initiate DNA replication. Cyclin E/cdk2 accumulates during late G phase and triggers the passage into S phase. The entire genome is replicated during S phase. The synthesis and accumulation of cyclin B/cdc2 also begins during S phase, but the complex is phosphorylated at $Thr^{14}$ -$Tyr^{15}$ and is inactive. Cyclin A/cdk2 accumulates during S phase and its activation triggers the transition to $G_2$, a phase characterized by the accumulation of cyclin B/cdc2, the inhibition of DNA replication, cell growth and new protein synthesis.

The transition from the $G_2$ phase to mitosis is triggered by the Cdc25-mediated activation (dephosphorylation) of the cyclin B/cdc2 complex (MPF). The activation of cyclin B/cdc2 that is necessary for G/M progression is currently the most well characterized step in the cell cycle. CyclinB/cdc2 is activated by phosphorylation of $Thr^{160}$ and the dephosphorylation of $Thr^{14}$ -$Tyr^{15}$. $Thr^{160}$ is phosphorylated by cyclin activating kinase (CAK), following the activation of CAK by a cyclin activating kinase activating kinase (CAKAK). However, the complex is kept in an inactive state due to the phosphorylation of $Thr^{15}$, which is catalyzed by the Weel kinase. Cyclin B/cdc2 activation is triggered when Cdc25, a phospatase, dephosphorylates $Thr^{15}$. In turn, the activity of Cdc25 is regulated by both activating and inhibitory phosphorylations. Phosphorylation of Ser by Chk1 (a check point activated kinase that participates in the $G_2$-arrest of cells with damaged DNA) leads to the inactivation of Cdc25, while phosphorylation by an M-phase activated kinase creates a positive feedback loop leading to the rapid activation of the cyclin B/cdc2 complex.

MPF catalyzes the phosphorylation of lamins and histone 1, and is involved in the regulation of events preceding cell division, such as spindle formation, chromatin condensation, and fragmentation of the nuclear envelope and of organelles such as the Golgi and endoplasmic reticulum. The metaphase to anaphase transition is triggered by inactivation of MPF and the degradation of cyclin B. This induces the separation of chromatids and their movement to the poles of the mitotic spindle, after which the mitotic apparatus disappears, the nuclear membranes reform and the nucleoli reappear. During cytokinesis, the cytoplasm divides and the resulting daughter cells enter G1.

When cells traverse the $G_0$ to $G_1$ phase to the S-phase transition, a series of cyclin-dependent kinases is activated. The addition of serum growth factors to quiescent cells promotes transcription of the cyclin $D_1$ gene. Cyclin $D_1$ then associates with pre-existing cdk4 to form an active complex. The kinase activity associated with this complex can phosphorylate specific sites on the retinoblastoma protein (pRb), leading to inactivation of pRb and the activation cyclin E transcription by E2F. Activation of the cyclin E gene can be blocked by the cdk inhibitor p16. Cyclin E associates with existing cdk2 and this active complex regulates the function of several sets of target proteins. First, cyclin E/cdk2 complexes associate with E2F/p107 complexes to activate expression of the cyclin a gene. Also, cyclin E/cdk2 complexes cooperate with cyclin D1 to amplify the phosphorylation of pRb. Cyclin A associates with cdk2 to form a kinase complex that phosphorylates downstream targets involved in the initiation of DNA replication.

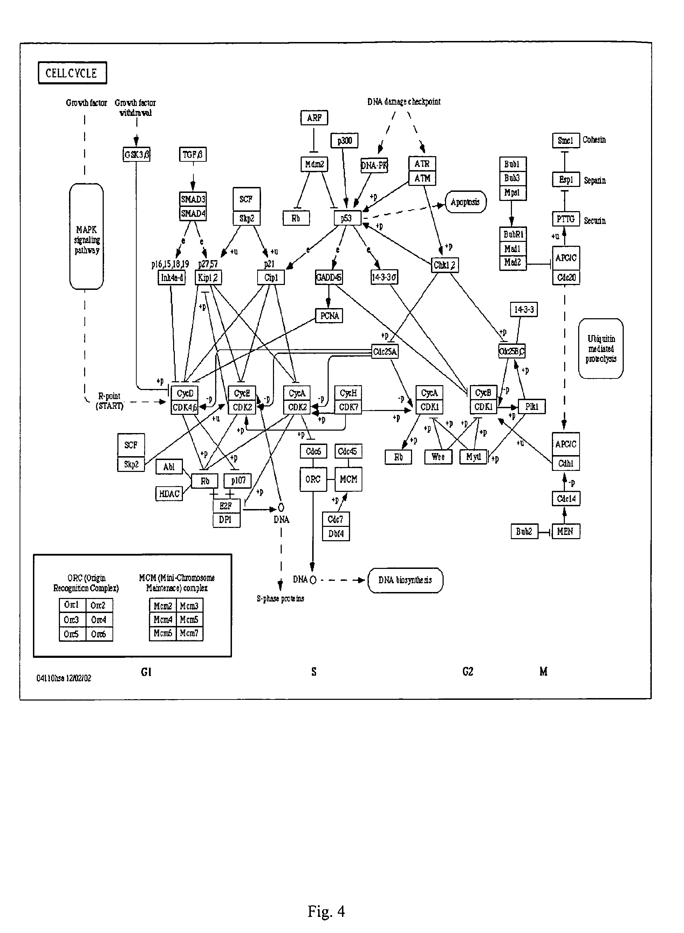

The general pathway of the cell cycle is illustrated in Fig. 4.

In this invention, 37 genes (or proteins, respectively) have been selected as being good representatives of the cell cycle pathway being a vital function of the cell (table 1). They comprise : ATM, CAV1, CCNA1, CCNB1, CCND1, CCND2, CCND3, CCNE1, CCNF, CCNG, CCNH, CDK2, CDK4, CDK6, DHFR, FE65, GRB2, HLF, MCM2, MDM2, MKI67, p16, p21, p27, p35, p53, p57, PCNA, RB1, SMAD1, SMAD2, SMAD3, SMAD4, S100A10, S100A4, S100A8, TK1.

### 4. Growth factors and cytokines

Growth factors are proteins that bind to receptors on the cell surface, with the primary result of activating cellular proliferation and/or differentiation. Many growth factors are quite versatile, stimulating cellular division in numerous different cell types, while others are specific to a particular cell-type.

Cytokines are a unique family of growth factors. Secreted primarily from leukocytes, cytokines stimulate both the humoral and cellular immune responses, as well as the activation of phagocytic cells. Cytokines that are secreted from lymphocytes are termed lymphokines, whereas those secreted by monocytes or macrophages are termed monokines. A large family of cytokines are produced by various cells of the body. Many of the lymphokines are also known as interleukins (ILs), since they are not only secreted by leukocytes but also able to affect the cellular responses of leukocytes. Specifically, interleukins are growth factors targeted to cells of hematopoietic origin.

EGF, like all growth factors, binds to specific high-affinity, low-capacity receptors on the surface of responsive cells. EGF has proliferative effects on cells of both mesodermal and ectodermal origin, particularly keratinocytes and fibroblasts. EGF exhibits negative growth effects on certain carcinomas as well as hair follicle cells. Growth-related responses to EGF include the induction of nuclear proto-oncogene expression, such as Fos, Jun and Myc.

Proliferative responses to PDGF action are exerted on many mesenchymal cell types. Other growth-related responses to PDGF include cytoskeletal rearrangement and increased polyphosphoinositol turnover. Again, like EGF, PDGF induces the expression of a number of nuclear localized proto-oncogenes, such as Fos, Myc and Jun.

There are at least 19 distinct members of the FGF family of growth factors. Studies of human disorders as well as gene knockout studies in mice show the prominent role for FGFs is in the development of the skeletal system and nervous system in mammals. Additionally, several members of the FGF family are potent inducers of mesodermal differentiation in early embryos. Non-proliferative effects include regulation of pituitary and ovarian cell function. TGFs-b have proliferative effects on many mesenchymal and epithelial cell types. Under certain conditions TGFs-b will demonstrate anti-proliferative effects on endothelial cells, macrophages, and T- and B-lymphocytes. Such effects include decreasing the secretion of immunoglobulin and suppressing hematopoiesis, myogenesis, adipogenesis and adrenal steroidogenesis. Several members of the TGF-b family are potent inducers of mesodermal differentiation in early embryos, in particular TGF-b and activin A.

The predominant sources of TGF-$\alpha$ are carcinomas, but activated macrophages and keratinocytes (and possibly other epithelial cells) also secrete TGF-$\alpha$. In normal cell populations, TGF-$\alpha$ is a potent keratinocyte growth factor. The Mullerian inhibiting substance (MIS) is also a TGF-$\beta$-related protein, as are members of the bone morphogenetic protein (BMP) family of bone growth-regulatory factors.

IGF-I is a growth factor structurally related to insulin. IGF-I is the primary protein involved in responses of cells to growth hormone (GH): that is, IGF-I is produced in response to GH and then induces subsequent cellular activities, particularly on bone growth.

The predominant function of IL-1 is to enhance the activation of T-cells in response to antigen. The activation of T-cells, by IL-1, leads to increase T-cell production of IL-2 and of the IL-2 receptor, which in turn augments the activation of the T-cells in an autocrine loop. IL-1 also induces expression of interferon-g (IFN-$\gamma$) by T-cells. There are 2 distinct IL-1 proteins, termed IL-1-a and -1-b, that are 26% homologous at the amino acid level. The IL-1s are secreted primarily by macrophages but also from neutrophils, endothelial cells, smooth muscle cells, glial cells, astrocytes, B- and T-cells, fibroblasts and keratinocytes. Production of IL-1 by these different cell types occurs only in response to cellular stimulation. In addition to its effects on T-cells, IL-1 can induce proliferation in non-lymphoid cells.

IL-2, produced and secreted by activated T-cells, is the major interleukin responsible for clonal T-cell proliferation. IL-2 also exerts effects on B-cells, macrophages, and natural killer (NK) cells. The production of IL-2 occurs primarily by CD4+ T-helper cells. In contrast to T-helper cells, NK cells constitutively express IL-2 receptors and will secrete TNF-$\alpha$, IFN-g and GM-CSF in response to IL-2, which in turn activate macrophages. IL-6 is produced by macrophages, fibroblasts, endothelial cells and activated T-helper cells. IL-6 acts in synergy with IL-1 and TNF-$\alpha$ (in many immune responses), including T-cell activation. In particular, IL-6 is the primary inducer of the acute-phase response in liver. IL-6 also enhances the differentiation of B-cells and their consequent production of immunoglobulin. Unlike IL-1, IL-2 and TNF-$\alpha$, IL-6 does not induce cytokine expression; its main effects, therefore, are to augment the responses of immune cells to other cytokines.

IL-8 is an interleukin that belongs to an ever-expanding family of proteins that exert chemoattractant activity to leukocytes and fibroblasts. IL-8 is produced by monocytes, neutrophils, and NK cells and is chemoattractant for neutrophils, basophiles and T-cells. In addition, IL-8 activates neutrophils to degranulate.

TNF-$\alpha$, like IL-1 is a major immune response modifying cytokine produced primarily by activated macrophages. Like other growth factors, TNF-a induces expression of a number of nuclear proto-oncogenes as well as of several interleukins.

TNF-$\beta$ is characterized by its ability to kill a number of different cell types, as well as the ability to induce terminal differentiation in others. One significant non-proliferative response to TNF-$\beta$ is an inhibition of lipoprotein lipase present on the surface of vascular endothelial cells. The predominant site of TNF-$\beta$ synthesis is T-lymphocytes, in particular the special class of T-cells called cytotoxic T-lymphocytes (CTL cells). The induction of TNF-b expression results from elevations in IL-2 as well as the interaction of antigen with T-cell receptors.

IFN-$\alpha$, IFN-$\beta$ and IFN-$\omega$ are known as type I interferons: they are predominantly responsible for the antiviral activities of the interferons. In contrast, IFN-$\gamma$ is a type II or immune interferon. Although IFN-$\gamma$, has antiviral activity it is significantly less active at this function than the type I IFNs. IFN-$\gamma$ is secreted primarily by CD8+ T-cells. Nearly all cells express receptors for IFN-$\gamma$ and respond to IFN-$\gamma$ binding by increasing the surface expression of class I MHC proteins, thereby promoting the presentation of antigen to T-helper (CD4+) cells. IFN-$\gamma$ also increases the presentation of class II MHC proteins on class II cells further enhancing the ability of cells to present antigen to T-cells.

CSFs are cytokines that stimulate the proliferation of specific pluripotent stem cells of the bone marrow in adults. Granulocyte-CSF (G-CSF) is specific for proliferative effects on cells of the granulocyte lineage. Macrophage-CSF (M-CSF) is specific for cells of the macrophage lineage. Granulocyte-macrophage-CSF (GM-CSF) has proliferative effects on both classes of lymphoid cells. IL-3 (secreted primarily from T-cells) is also known as multi-CSF, since it stimulates stem cells to produce all forms of hematopoietic cells.

In this invention, 36 genes (or proteins) have been selected as appropriate representative of the growth factors and cytokines being a vital function of the cell (table 1). They comprise : AREG, BMP2, CCL2, CSF1, CTGF, FGF2, FGF8, GMCSF, IFNG, IGF1, IGFBP2, IGFBP3, IGFBP5, IL2, IL3, IL8, IL10, IL11, IL12, IL15, ILIA, IL1B, IL4, IL6, MEK1, MEK2, PDGFA, PRSS11, TGFA, TGFB1, TNFA, TNFB, VEGF, VEGFB, VEGFC and VEGFD.

### 5. Cell signaling/receptor

This is accomplished by a variety of signaling molecules that are secreted or expressed on the surface of a given cell and bind to receptors expressed by other cells, thereby integrating and coordinating the functions of the many individual cells that make up organisms as complex as human beings.

The binding of most signaling molecules to their receptors initiates a series of intracellular reactions that regulate virtually all aspects of cell behavior, including metabolism, movement, proliferation, survival, and differentiation. Interest in this area is further heightened by the fact that many cancers arise as a result of a breakdown in the signaling pathways that control normal cell proliferation and survival.

Cells must be ready to respond to essential signals in their environment. These are often chemicals in the extracellular fluid (ECF) from: a) distant locations in a multicellular organism - endocrine signaling by hormones; b) nearby cells - paracrine stimulation by cytokines; c) even secreted by themselves ( = autocrine stimulation).

They may also respond to molecules on the surface of adjacent cells (e.g. producing contact inhibition).

Signaling molecules may trigger: a) an immediate change in the metabolism of the cell (e.g., increased glycogenolysis when a liver cell detects adrenaline); b) an immediate change in the electrical charge across the plasma membrane (e.g., the source of action potentials); c) a change in the gene expression - transcription - within the nucleus.

Two categories of signaling molecules (steroids and nitric oxide) diffuse into the cell where they bind internal receptors.

The others, e.g., proteins, bind to receptors displayed at the surface of the cell. These are transmembrane proteins whose extracellular portion has the binding site for the signaling molecule (the ligand); intracellular portion activates proteins in the cytosol that in different ways eventually regulate gene transcription in the nucleus.

They comprise: G-Protein-Coupled Receptors (GPCRs), Cytokine Receptors, Receptor Tyrosine Kinases (RTKs), JAK-STAT Pathways, Transforming Growth Factor-beta (TGF-b) Receptors, Tumor Necrosis Factor-a Receptors and the NF-kB Pathway, The T-Cell Receptor for Antigen (TCR)

In this invention, 67 genes (or proteins) have been selected as appropriate representatives of the cell signaling and receptor pathway being a vital function of the cell (table 1). They comprise : ADRA1a, ADRA1b, ADRA1d, ADRA2c, ADRB2, Calcyon, CCR2, CHRNA2, CHRNA3, CHRNA4, CHRNA5, CHRNA7, CHRNB1, CHRNB2, CHRNB3, CHRNB4, CHRND, CHRNE, CHRM1, CHRM2, CHRM3, CHRM4, CSF1R, Drd1a, Drd2, Drd3, DRIP78, DTR, EGFR, EAR1, ESR2, FGFR, Gpr88, Hrh1, Hrh2, Hrh3, Hrh4, Htr1a , Htr1b , Htr1d, Htr1f, Htr2a, Htr2b, Htr2c, Htr3a, Htr3b, Htr4, Htr5a, Htr5b, Htr6, Htr7, IGF1R, IL11RA, MSR1, NCK1, NCORI, NCOR2, NGFR, PGR, PLAUR, ROR1, TBXA2R, TNFRSF1A, TNFRSF1B, VEGFR1, VEGFR2 and VEGFR3.

### 6. Chromosomal processing

In eukaryotic cells, the genetic material is organized in a complex structure composed of DNA and proteins, and is localized in the nucleus. This structure is called chromatin. In each cell, about two meters of DNA are contained in the nucleus. In addition to its high degree of compaction, DNA must be easily accessible to allow its interaction with the protein machinery allowing its replication, repair and recombination. The fundamental unit of chromatin is called nucleosome and is composed of DNA and histones. It constitutes the first level of compaction of DNA in the nucleus. This structure is regularly repeated to form nucleofilaments, which can adopt further compaction levels. The chromatin is divided in euchromatin and heterochromatin. The heterochromatin keeps the same structure along the cell cycle while the euchromatin appears less condensed during the interphase.

The histones H3, H4, H2A and H2B are very conserved basic proteins. They are the targets of numerous post-translational modifications, which could affect the accessibility to DNA and protein/protein interactions with the nucleosome.

The assembly of DNA into chromatin starts with the formation of tetramers (H3-H4)2 histones newly synthesized, which fix two dimers H2A-H2B (2). The newly synthesized histones are specifically modified, the most conserved modification being the acetylation of histone H4 on lysines 5 and 12. The maturation step requires ATP in order to allow a regular spacing of nucleosomes and the histones newly incorporated are desactetylated. The acetylation state results from equilibrium between two antagonist activities: the activity histone-acetyltransferase (HAT) and the activity histone-desacetylase (HDAC).

The chromatin can be submitted to variations at the DNA level (methylation) and at the histone level (post-translational modification, existence of variants such as CENPA, variant of histone 3). These modifications are able to induce difference in the structure and activity of chromatin. For instance, CENPA is associated to centromeric regions.

Chaperon histones may form complexes with histones to favor their assembly. For example, CAF-1 (Chromatin Assembly Factor 1) can react with acetylated histones H3 and H4 and take part to the assembly of chromatin and to the DNA replication. The interaction between CAF-1 and PCNA (Proliferating Cell Nuclear Antigen) establish a molecular bound between the chromatin assembly and the processes of DNA replication and repair.

In this invention, 7 genes (or proteins) have been selected as representative of the chromosomes processing being a vital function of the cell (table 1). They comprise : CENPA, CENPF, H2B/S, H3FF, H4FM, KNSL5 and KNSL6.

### 7. DNA Replication/repair

Replication of nuclear chromosomes involves polymerase alpha and polymerase delta. Polymerase alpha (lagging strand replicase) Contains primase activity and has no proofreading 3'-5' exonuclease activity. Polymerase delta (leading strand replicase) lacks primase activity, has 3'-5' exonuclease activity. Proliferating cell nuclear antigen (PCNA) enhances processivity. DNA polymerases cannot replicate the extreme 5'-ends of chromosomes due to RNA priming (primer gap). The ends of chromosomes, or telomeres, consist of short repeated sequences that are synthesized by a polymerase called telomerase. Telomerase is a reverse transcriptase that adds de novo telomeric repeats onto chromosome ends or telomeres, compensating for the telomere loss that occurs due to the "end replication problem". Telomerase contains a catalytic subunit or TERT (telomerase reverse transcriptase) and an RNA component, Terc (telomerase RNA), which contains the sequence that telomerase uses as template for the addition of the new telomeric repeats and is therefore essential for enzyme activity. The maintenance of telomere length by telomerase is essential for chromosomal stability and cell viability and plays an important role in both tumor formation and aging. The loss of telomere repeats has been causally linked to replicative senescence by the demonstration that overexpression of the enzyme telomerase can result in the elongation or maintenance of telomeres and immortalisation of somatic cells with a diploid and apparently normal karyotype. Experimental evidence indicates that short telomeres accumulate prior to senescence and that replicative senescence is not triggered by the first telomere to reach a critical minimal threshold length. These observations are compatible with limited repair of short telomeres by telomerase-dependent or telomerase-independent DNA repair pathways. Deficiencies in telomere repair may result in accelerated senescence and aging as well as genetic instability that facilitates malignant transformation.

DNA replication is extremely accurate, but errors in polymerization occur, environmental factors, such as chemicals and ultraviolet radiation, can alter DNA. Such damage to DNA can block replication or transcription, and can result in a high frequency of mutations. To maintain the integrity of their genomes, cells have therefore evolved mechanisms to repair damaged DNA. These mechanisms of DNA repair can be divided into two general classes: (1) direct reversal of the chemical reaction responsible for DNA damage, and (2) removal of the damaged bases followed by their replacement with newly synthesized DNA. Where DNA repair fails, additional mechanisms have evolved to enable cells to cope with the damage.

In this invention, 13 genes (or proteins) are included into the array analysis as appropriate characteristic of the DNA replication and repair processes (table 1). They comprise: ADPRT, CROC1A, FHIT, GADD153, PLK, POLA2, RRM1, SLK, TERC, TERT, TOP2, TRF1 and TYMS.

### 8. Intermediate metabolism

The immediate source of energy for most cells is glucose but carbohydrates, fats and even proteins may in certain cells or at certain times be used as a source of energy (ATP).

Dietary carbohydrates from which humans gain energy enter the body in complex forms, such as disaccharides and the polymers starch (amylose and amylopectin) and glycogen. The first step in the metabolism of digestible carbohydrate is the conversion of the higher polymers to simpler, mono-saccharides soluble forms that can be transported across the intestinal wall and delivered to the tissues. The resultant glucose and other simple carbohydrates are transported across the intestinal wall to the hepatic portal vein and then to liver parenchymal cells and other tissues. There they are converted to fatty acids, amino acids, and glycogen, or else oxidized by the various catabolic pathways of cells. Oxidation of glucose is known as glycolysis. Glucose is oxidized to either lactate or pyruvate. Under aerobic conditions, the dominant product in most tissues is pyruvate and the pathway is known as aerobic glycolysis.

$$\text{Glucose} + 2\text{ ADP} + 2\text{ NAD}^+ + 2\text{ Pi} \text{ -----> } 2\text{ Pyruvate} + 2\text{ ATP} + 2\text{ NADH} + 2\text{ H}^+$$

When oxygen is depleted, as for instance during prolonged vigorous exercise, the dominant glycolytic product in many tissues is lactate and the process is known as anaerobic glycolysis.

Aerobic glycolysis of glucose to pyruvate requires two equivalents of ATP to activate the process, with the subsequent production of four equivalents of ATP and two equivalents of NADH. Thus, conversion of one mole of glucose to two moles of pyruvate is accompanied by the net production of two moles each of ATP and NADH.

The main enzymes involved in glycolysis are hexokinase, phosphohexose isomerase, phosphofructokinase-1, aldolase, glyceraldehyde-3-phosphate dehydrogenase, Phosphoglycerate kinase, enolase and pyruvate kinase.

Utilization of dietary lipids requires that they first be absorbed through the intestine. As these molecules are oils they would be essentially insoluble in the aqueous intestinal environment. Solubilization (emulsification) of dietary lipid is accomplished via bile salts that are synthesized in the liver and secreted from the gallbladder. The emulsified fats can then be degraded by pancreatic lipases (lipase and phospholipase A2). These enzymes, secreted into the intestine from the pancreas, generate free fatty acids and a mixture of mono- and diacylglycerols from dietary triacylglycerols. Following absorption of the products of pancreatic lipase by the intestinal mucosal cells, the resynthesis of triacylglycerols occurs. The triacylglycerols are then solubilized in lipoprotein complexes (complexes of lipid and protein) called chylomicrons. Triacylglycerols synthesized in the liver are packaged into VLDLs and released into the blood directly. Chylomicrons from the intestine are then released into the blood via the lymph system for delivery to the various tissues for storage or production of energy through oxidation.

Fatty acids must be activated in the cytoplasm before being oxidized in the mitochondria. Activation is catalyzed by fatty acyl-CoA ligase (also called acyl-CoA synthetase or thiokinase).

$$\text{Fatty acid} + ATP + CoA \text{ -------> } Acyl\text{-}CoA + PPi + AMP$$

Oxidation of fatty acids occurs in the mitochondria. The process of fatty acid oxidation is termed b-oxidation since it occurs through the sequential removal of 2-carbon units by oxidation at the b-carbon position of the fatty acyl-CoA molecule. The oxidation of fatty acids yields significantly more energy per carbon atom than does the oxidation of carbohydrates. The main enzymes involved in the oxidation of fatty acids are acyl-CoA synthetase, enoyl-CoA hydratase, 3-hydorxyacyl-CoA dehydrogenase and thiolase.

One might predict that the pathway for the synthesis of fatty acids would be the reversal of the oxidation pathway. However, this would not allow distinct regulation of the two pathways to occur even given the fact that the pathways are separated within different cellular compartments. The pathway for fatty acid synthesis occurs in the cytoplasm, whereas, oxidation occurs in the mitochondria. The other major difference is the use of nucleotide cofactors. Oxidation of fats involves the reduction of FADH+ and NAD+. Synthesis of fats involves the oxidation of NADPH. Both oxidation and synthesis of fats utilize an activated two carbon intermediate, acetyl-CoA. However, the acetyl-CoA in fat synthesis exists temporarily bound to the enzyme complex as malonyl-CoA. The synthesis of malonyl-CoA is the first committed step of fatty acid synthesis and the enzyme that catalyzes this reaction, acetyl-CoA carboxylase (ACC), is the major site of regulation of fatty acid synthesis.

All tissues have some capability for synthesis of the non-essential amino acids, amino acid remodeling, and conversion of non-amino acid carbon skeletons into amino acids and other derivatives that contain nitrogen. However, the liver is the major site of nitrogen metabolism in the body. In times of dietary surplus, the potentially toxic nitrogen of amino acids is eliminated via transamination, deamination, and urea formation; the carbon skeletons are generally conserved as carbohydrate, via gluconeogenesis, or as fatty acid via fatty acid synthesis pathways. In this respect amino acids fall into three categories: glucogenic, ketogenic, or glucogenic and ketogenic. Glucogenic amino acids are those that give rise to a net production of pyruvate or TCA cycle intermediates, such as $\alpha$-ketoglutarate or oxaloacetate, all of which are precursors to glucose via gluconeogenesis. All amino acids except lysine and leucine are at least partly glucogenic. Lysine and leucine are the only amino acids that are solely ketogenic, giving rise only to acetylCoA or acetoacetylCoA, neither of which can bring about net glucose production. A small group of amino acids comprised of isoleucine, phenylalanine, threonine, tryptophan, and tyrosine give rise to both glucose and fatty acid precursors and are thus characterized as being glucogenic and ketogenic. Finally, it should be recognized that amino acids have a third possible fate. During times of starvation the reduced carbon skeleton is used for energy production, with the result that it is oxidized to $CO_2$ and $H_2O$.

In this invention, 10 genes (or proteins) are included into the array analysis as appropriate characteristic of the intermediate metabolism pathway (table 1). They comprise: CKB, ETFB, G6PD, GAA, GLB1, MVK, eNOS, iNOS, ODC and PKM2.

**9. The Extracellular Matrix**

The extracellular matrix (ECM) is a complex structural entity surrounding and supporting cells that are found within mammalian tissues. The ECM is often referred to as the connective tissue. The ECM is composed of 3 major classes of biomolecules:

1. Fibrous structural proteins: collagen and elastin.
2. Specialized proteins: e.g. fibrillin, fibronectin, and laminin.
3. Proteoglycans: these are composed of a protein core to which is attached long chains of repeating disaccharide units termed of glycosaminoglycans (GAGs) forming extremely complex high molecular weight components of the ECM.

The differences between the various types of extracellular matrix result from variations in the proportion of components.

The major structural protein of the extracellular matrix is collagen, which is the single most abundant protein in animal tissues. There are at least 12 types of collagen. Types I, II and III are the most abundant and form fibrils of similar structure. Type IV collagen forms a two-dimensional reticulum and is a major component of the basal lamina. Collagens are predominantly synthesized by fibroblasts but epithelial cells also synthesize these proteins.

Connective tissues also contain elastic fibbers, which are particularly abundant in organs that regularly stretch and then return to their original shape. Elastic fibbers are composed principally of a protein called elastin, which is crosslinked into a network by covalent bonds. This network of crosslinked elastin chains behaves like a rubber band, stretching under tension and then snapping back when the tension is released.

The role of fibronectin is to attach cells to a variety of extracellular matrices. Fibronectin attaches cells to all matrices except type IV that involves laminin as the adhesive molecule. Fibrillin is an integral constituent of the non-collagen us microfibrils of the extracellular matrix. The ECM includes Matrix MetalloProteinase (MMP).

In this invention, 23 genes (or proteins) are included into the array analysis as an appropriate characteristic of the extracellular proteins (table 1). They comprise : BSG, COL1A1, COL3AI, COL6A2, COL15A1, DPT, ELN, FN1, FMOD, MMP1, MMP9, MMP10, MMP11, MMP12, MMP13, MMP14, MMP15, MMP2, MMP3, MMP7, OPN, TIMP 1 and TIMP2.

**10. Cell structure**

Cytoskeleton (intracellular)

Cellular responses to extracellular signals, including growth factors, frequently include changes in cell movement and cell shape. For example, growth factor-induced alterations in cell motility (as well as in cell proliferation) play critical roles in processes such as wound healing and embryonic development. In particular, many types of cell movement are based on the dynamic assembly and disassembly of actin filaments underlying the plasma membrane. Remodelling of the actin cytoskeleton therefore represents a key element of the response of many cells to growth factors and other extracellular stimuli.

A network of actin filaments and other cytoskeletal proteins underlies the plasma membrane and determines cell shape. Actin bundles also attach to the plasma membrane and anchor the cell at regions of cell-cell and cell-substratum contact.

Membrane cytoskeleton comprises the following proteins: microfilament (actin/myosin), spectrins (alpha, beta), dystrophin, ankyrin, adduxin, myosin, tropomyosin, dematin, glycophorin, fibronectin receptor, talin, vinculin, $\alpha$-actinin, fimbrin, villin, myosin I, spectrin, filamin, keratin, vimemtin, cytokeratin.

Microtubules are formed by the reversible polymerization of tubulin. They display dynamic instability and undergo continual cycles of assembly and disassembly as a result of GTP hydrolysis following tubulin polymerization. Interior cytoskeleton comprises the following components: tubulin (microtubules), actin (microfilaments), stress fibers (microfilaments, myosin, a-actinin, tropomyosin, caldesmon), vinculin, talin, kinetochore, centrosome, spindle pole body, centriol, centractin, pericentrin.

Intermediate filaments are polymers of more than 50 different proteins that are expressed in various types of cells and can often identify the origin of the cell. They are not involved in cell movement, but provide mechanical support to cells and tissues. Intermediate filaments (IF) comprise: keratins, cytokeratins, nestin, vimentin, desmin, glial fibrillary acidic protein, peripherin, lamins. Intermediate filament-associated proteins (IFAP) comprise: epinemin, filaggrin, plectin, peripherin, restin, lamin.

Actin binding proteins (ABP) comprise : fragmin, b-actinin, gelsolin, villin, brevin, severin, filamin, spectrin, fodrin, $\alpha$-actinin, gelactin, fascin, vinculin, talin, fimbrin, tau, profilin, capping proteins.

In this invention, 10 genes (or proteins) are included into the array analysis as an appropriate characteristic of the cell structure proteins(table 1). They comprise : CDC42, EMS1, GSN, MP1, ON, PAK, SLP2, SM22, TB10 and TGM1.

### 11. Protein metabolism (synthesis/degradation)

Protein synthesis

Translation is the RNA directed synthesis of polypeptides. This process requires all three classes of RNA. Although the chemistry of peptide bond formation is relatively simple, the processes leading to the ability to form a peptide bond are exceedingly complex. The template for correct addition of individual amino acids is the mRNA, yet both tRNAs and rRNAs are involved in the process. The tRNAs carry activated amino acids into the ribosome which is composed of rRNA and ribosomal proteins. The ribosome is associated with the mRNA ensuring correct access of activated tRNAs and containing the necessary enzymatic activities to catalyze peptide bond formation. Following assembly of both the small and large subunits onto the mRNA, and given the presence of charged tRNAs, protein synthesis can take place.

Translation proceeds in an ordered process. First accurate and efficient initiation occurs, then chain elongation and finally accurate and efficient termination must occur. All three of these processes require specific proteins, some of which are ribosome associated and some of which are separate from the ribosome, but may be temporarily associated with it. Initiation of translation requires a specific initiator tRNA, tRNAmeti, that is used to incorporate the initial methionine residue into all proteins. The initiation of translation requires recognition of an AUG codon. A specific sequence context surrounding the initiator AUG aids ribosomal discrimination. This context is A/GCCA/GCCAUGA/G in most mRNAs. The specific non-ribosomally associated proteins required for accurate translational initiation are termed initiation factors.

The initiation factors eIF-1 and eIF-3 bind to the 40S ribosomal subunit favoring antiassociation to the 60S subunit. The prevention of subunit reassociation allows the preinitiation complex to form. The first step in the formation of the preinitiation complex is the binding of GTP to eIF-2 to form a binary complex. eIF-2 is composed of three subunits, a, b and g. The binary complex then binds to the activated initiator tRNA, met-tRNAmet forming a ternary complex that then binds to the 40S subunit forming the 43S preinitiation complex. The preinitiation complex is stabilized by the earlier association of eIF-3 and eIF-1 to the 40S subunit. The cap structure of eukaryotic mRNAs is bound by specific eIFs prior to association with the preinitiation complex. Cap binding is accomplished by the initiation factor eIF-4F. This factor is actually a complex of 3 proteins; eIF-4E, A and G. The protein eIF-4E is a 24 kDa protein which physically recognizes and binds to the cap structure. eIF-4A is a 46 kDa protein which binds and hydrolyses ATP and exhibits RNA helicase activity. Unwinding of mRNA secondary structure is necessary to allow access of the ribosomal subunits. eIF-4G aids in binding of the mRNA to the 43 S preinitiation complex. Once the mRNA is properly aligned onto the preinitiation complex and the initiator met-tRNAmet is bound to the initiator AUG codon (a process facilitated by eIF-1) the 60S subunit associates with the complex. The association of the 60S subunit requires the activity of eIF-5 which binds first to the preinitiation complex. The energy needed to stimulate the formation of the 80S initiation complex comes from the hydrolysis of the GTP bound to eIF-2. The GDP bound form of eIF-2 then binds to eIF-2B which stimulates the exchange of GTP for GDP on eIF-2. When GTP is exchanged eIF-2B dissociates from eIF-2. This is termed the eIF-2 cycle (see diagram below). This cycle is absolutely required in order for eukaryotic translational initiation to occur. The GTP exchange reaction can be affected by phosphorylation of the a-subunit of eIF-2. At this stage the initiator met-tRNAmet is bound to the mRNA within a site of the ribosome termed the P-site, for peptide site. The other site within the ribosome to which incoming charged tRNAs bind is termed the A-site, for amino acid site.

The process of elongation, like that of initiation requires specific non-ribosomal proteins. Elongation of polypeptides occurs in a cyclic manner such that at the end of one complete round of amino acid addition the A site will be empty and ready to accept the incoming aminoacyl-tRNA dictated by the next codon of the mRNA. This means that not only does the incoming amino acid need to be attached to the peptide chain but also the ribosome must move down the mRNA to the next codon. Each incoming aminoacyl-tRNA is brought to the ribosome by an eEF-1a-GTP complex. When the correct tRNA is deposited into the A site the GTP is hydrolyzed and the eEF-la-GDP complex dissociates. In order for additional translocation events the GDP must be exchanged for GTP. This is carried out by eEF-1bg similarly to the GTP exchange that occurs with eIF-2 catalyzed by eIF-2B. The peptide attached to the tRNA in the P site is transferred to the amino group at the aminoacyl-tRNA in the A site. This reaction is catalyzed by peptidyltransferase. This process is termed transpeptidation. The elongated peptide now resides on a tRNA in the A site. The A site needs to be freed in order to accept the next aminoacyl-tRNA. The process of moving the peptidyl-tRNA from the A site to the P site is termed, translocation. Translocation is catalyzed by eEF-2 coupled to GTP hydrolysis. In the process of translocation the ribosome is moved along the mRNA such that the next codon of the mRNA resides under the A site. Following translocation eEF-2 is released from the ribosome. The cycle can now begin again. Like initiation and elongation, translational termination requires specific protein factors identified as releasing factors. The signals for termination are termination codons present in the mRNA. There are 3 termination codons, UAG, UAA and UGA. The eRF binds to the A site of the ribosome in conjunction with GTP. The binding of eRF to the ribosome stimulates the peptidyltransferase activity to transfer the peptidyl group to water instead of an aminoacyl-tRNA. The resulting uncharged tRNA left in the P site is expelled with concomitant hydrolysis of GTP. The inactive ribosome then releases its mRNA and the 80S complex dissociates into the 40S and 60S subunits ready for another round of translation.

Protein degradation

The levels of proteins within cells are determined not only by rates of synthesis, but also by rates of degradation. The half-lives of proteins within cells vary widely, from minutes to several days, and differential rates of protein degradation are an important aspect of cell regulation. Many rapidly degraded proteins function as regulatory molecules, such as transcription factors. The rapid turnover of these proteins is necessary to allow their levels to change quickly in response to external stimuli. Other proteins are rapidly degraded in response to specific signals, providing another mechanism for the regulation of intracellular enzyme activity.

The major pathway of selective protein degradation in eukaryotic cells uses ubiquitin as a marker that targets cytosolic and nuclear proteins for rapid proteolysis. Ubiquitin is a 76-amino-acid polypeptide that is highly conserved in all eukaryotes. Proteins are marked for degradation by the attachment of ubiquitin to the amino group of the side chain of a lysine residue. Additional ubiquitins are then added to form a multiubiquitin chain. Such polyubiquinated proteins are recognized and degraded by a large, multisubunit protease complex, called the proteasome. Ubiquitin is released in the process, so it can be reused in another cycle. It is noteworthy that both the attachment of ubiquitin and the degradation of marked proteins require energy in the form of ATP.

Some enzymes involved in the degradation of proteins are: trypsin, trypsin inhibitors, chymotrypsin, proprotein convertase, cathepsins, kallikrein (hormone processing), calpain, metalloproteinases, hippostasin, granzyme, renin, elastase, C 1 inhibitor.

In this invention, 15 genes (or proteins) are included into the array analysis as an appropriate characteristic of the protein metabolism (table 1). They comprise : ADAM1, BAT1, CANX, CTSB, CTSD, CTSH, CTSL, CTSS, CTSZ, EF1A, EIF-4A, EIF-4E, EIF3S6, RPL3 and RPL10.

## 12. Oxidative metabolism

Oxidative stress is imposed on cells as a result of one of three factors: 1) an increase in oxidant generation, 2) a decrease in antioxidant protection, or 3) a failure to repair oxidative damage. Cell damage is induced by reactive oxygen species (ROS). ROS are either free radicals, reactive anions containing oxygen atoms, or molecules containing oxygen atoms that can either produce free radicals or are chemically activated by them. Examples are hydroxyl radical, superoxide, hydrogen peroxide, and peroxynitrite. The main source of ROS *in vivo* is aerobic respiration, although ROS are also produced by peroxisomal β-oxidation of fatty acids, microsomal cytochrome P450 metabolism of xenobiotic compounds, stimulation of phagocytosis by pathogens or lipopolysaccharides, arginine metabolism, and tissue specific enzymes. Under normal conditions, ROS are cleared from the cell by the action of superoxide dismutase (SOD), catalase, or glutathione (GSH) peroxidase. The main damage to cells results from the ROS-induced alteration of macromolecules such as polyunsaturated fatty acids in membrane lipids, essential proteins, and DNA. Additionally, oxidative stress and ROS have been implicated in disease states, such as Alzheimer's disease, Parkinson's disease, cancer, and aging.

In this invention, 5 genes (or proteins) are included into the array analysis as an appropriate characteristic of the oxidative metabolism (table 1). They comprise : SOD2, GSTT1, MSRA, GPX and GSTP1.

## 13. Transcription

The intricate task of regulating gene expression in the many differentiated cell types of multicellular organisms is accomplished primarily by the combined actions of multiple different transcriptional regulatory proteins. In addition, the packaging of DNA into chromatin and its modification by methylation impart further levels of complexity to the control of eukaryotic gene expression.

Despite the development of *in vitro* systems and the characterization of several general transcription factors, much remains to be learned concerning the mechanism of polymerase II transcription in eukaryotic cells. Transcription/Transcription factors include:

- RNA polymerases, transcription factors, activator/ repressor
- STAT = signal transducer and activator of transcription, PIAS = protein inhibitor of activated STAT
- Homeobox / forkhead motif proteins, TATA-binding protein (TBP), SOX = family of SRY-related genes, which encode transcriptional factors involved in development. The Sox gene family consists of a large number of embryonically expressed genes related via the possession of a 79-amino-acid DNA-binding domain known as the HMG box.

Polycomb group (PcG) proteins were first described in Drosophila as factors responsible for maintaining the transcriptionally repressed state of Hox/homeotic genes in a stable and heritable manner throughout development. A growing number of vertebrate genes related to the Drosophila PcG proteins have recently been identified.

In this invention, 18 genes (or proteins) are included into the array analysis as abn appropriate characteristic of the transcription pathways (table 1). They comprise : DP1, DP2, E2F1, E2F2, E2F3, E2F4, E2F5, EGR1, EGR2, EGR3, EPC1, JUND, MAX, MYBL2, STAT5, TFAP2A, TFAP2B and TFAP2C.

## B. Specific functions

Specific cellular functions are associated with particular cell types, the stage of differentiation of a cell, pathological conditions of changes in the cell environment. We described here under 5 of such particular functions which are part of this invention. We provide a description of their roles and some characteristics associated genes.

### 1. Cell differentiation

Structural and functional modification of an unspecialized cell into a specialized one. Roughly half of the genes expressed in a cell or tissue follow a tissue specific expression pattern. The diversity of expression patterns reflect the functional and structural differences that are necessary between distinct organ tissues are reflected through a certain number of genes that are present on the micro-array design.

In particular, for the Senechip, genes such as keratins, filaggrin and neuregulin are important markers of cell differentiation of epidermal tissues.

In this invention, 18 genes (or proteins) are included into the array analysis as an appropriate characteristic of the cell differentiation process and 66 genes as an appropriate characteristic of the neuronal cell differentiation process (table 1). They comprise : CST6, FLG, ID1, ID2, IVL, KRT1, CYT2A, KRT6A, KRT10, KRT14, KRT16, KRT17, KRT19, NRG1, OPG, PSOR1, SPRR1B, TH, TBXAS1, CD47, Rnpep, Ph2C, Pcbp4, PENK2, Txn, Baiap2, Sv2b, CBL20, Tac 1, Arha2, Ndufv1, Nlgn2, Rps23, Mxi1, Gbp2, Nudel, Sv2b, VL30, Sult4a1, Masp2, Cript, Ascl1, PP2A/B, Dbnl, p56, Rbbp9, Rps26, Myo5b, Tgm2, Rpl32, Gsk3a, Cops7a, Uchl1, Col2a1, Tsc2, Rmt7, Napa, Homer2, Plcb3, PAIHC3, TRPRS, Cdc37, Sdc4, ntt4r, Csrp3, Phyh, Aloxe3, Cst3, Atp2a2, GTP, Adcyap1, Mtmr2, Gnb1, Sap18, Slc2a1, Gda, Fth1, Casp1, Rab4a, Myr5, Cd59, Apeg1, TCEB2 and Fzd1.

### 2. Oncogene/tumor suppressor

Most, if not all, cancer cells contain genetic damage that appears to be the responsible event leading to tumorigenesis. The genetic damage present in a parental tumorigenic cell is maintained (i.e. not correctable) such that it is a heritable trait of all cells of subsequent generations. Genetic damage found in cancer cells is of two types:

1. Dominant and the genes have been termed proto-oncogenes. The distinction between the terms proto-oncogene and oncogene relates to the activity of the protein product of the gene. A proto-oncogene is a gene whose protein product has the capacity to induce cellular transformation given it sustains some genetic insult. An oncogene is a gene that has sustained some genetic damage and, therefore, produces a protein capable of cellular transformation. The process of activation of proto-oncogenes to oncogenes can include retroviral transduction or retroviral integration (see below), point mutations, insertion mutations, gene amplification, chromosomal translocation and/ or protein-protein interactions. Proto-oncogenes can be classified into many different groups based upon their normal function within cells or based upon sequence homology to other known proteins. As predicted, proto-oncogenes have been identified at all levels of the various signal transduction cascades that control cell growth, proliferation and differentiation. Oncogene include:

- Retroviral oncogenes (abl,akt, cbl, crk, erb, ets, fes, fgr, fms, fos, fps, jun, kit, maf, mos, mpl, myb, myc, qin, raf, ras, rel, eos, sea, sis, ski, src)
- Cellular oncogenes (ras, neu, met, ret, trk, ros, dbl, vav, cot, ovc, tre, hst, fgf, mas)
- Oncogenes activated by translocation (c-myc,bcl-2, bc1-3,bc1-6,hox11,IL-3,lyl-1,PRAD-1, rhom-1, rhom-2, tal-1, tal-2, tan-1)

2. Recessive and the genes variously termed tumor suppressors, growth suppressors, recessive oncogenes or anti-oncogenes. Tumor suppressor genes were first identified by making cell hybrids between tumor and normal cells. On some occasions a chromosome from the normal cell reverted the transformed phenotype. Several familial cancers have been shown to be associated with the loss of function of a tumor suppressor gene. They include the retinoblastoma susceptibility gene (RB), Wilms' tumors (WT1), neurofibromatosis type-1 (NF1), familial adenomatosis polyposis coli (APC or FAP), and those identified through loss of heterozygosity such as in colorectal carcinomas (called DCC for deleted in colon carcinoma) and p53, which was originally thought to be a proto-oncogene. However, the wild-type p53 protein suppresses the activity of mutant alleles of p53, which are the oncogenic forms of p53.

In this invention, a selective list of 19 genes (or proteins) are included into the array as an appropriate characteristic of the oncogene /tumor suppressor process (table 1). They comprise : BIN1, BRCA2, EWSR1, FES, FOS, ING1, L6, MAP17, MYC, NF1, p53, RAF1, RB1, RET, RRAS, S100A11, SHC, SNCG and TGFBR2.

### 3. Stress response

Heat Shock Proteins are proteins expressed in cells that have been subjected to elevated temperatures or other forms of environmental stress. The heat-shock proteins (abbreviated Hsp), which are highly conserved in both prokaryotic and eukaryotic cells, are thought to stabilize and facilitate the refolding of proteins that have been partially denatured as a result of exposure to elevated temperature. However, many members of the heat-shock protein family are expressed and have essential cellular functions under normal growth conditions. These proteins serve as molecular chaperones, which are needed for polypeptide folding and transport under normal conditions as well as in cells subjected to environmental stress.

Corticotropin-releasing hormone (CRH) is the principal regulator of the stress response. CRH stimulates production of ACTH via specific CRH receptors located on pituitary corticotropes. In addition to pituitary and central nervous system effects, peripheral effects of CRH have been observed involving the immune and cardiovascular systems.

Treatment and challenges to the cell system often result in readjustment of the level of expression of these defense enzymes in response to the type of stress such as drug or chemical treatment, UV or physical stress.

In this invention, 14 genes (or proteins) are included into the array analysis as an appropriate characteristic of the stress response (table 1). They comprise : AOP2, HMOX, HSP27, HSP40, HSP70, HSP70B, HSP90-alpha,, HSP90-beta, JNK1, JNKK1, JNK2, JNK3, MT2A and SRI.

### 4. Lipid metabolism

Many of the lipids involved as second messengers in cell signaling pathways arise from the arachidonic acid (AA) pathway. AA is an unsaturated fatty acid that is a normal constituent of membrane phospholipids and is released from the phospholipids by the actions of phospholipase $A_2$ ($PLA_2$). Prostaglandins (PG) arise from a cyclic endoperoxide generated by the enzyme system PG synthetase, a complex of enzymes that includes cyclooxygenase (COX). There is a constitutive (COX-1) and an inducible cyclooxygenase (COX-2). The cyclic endoperoxide intermediate is also a precursor of prostacyclin ($PGI_2$) and thromboxane ($TXA_3$). Other groups of compounds in this class, leukotrienes (LT) and lipoxins (LP), are derived directly from AA without the mediation of a cyclic endoperoxide. Lipoxygenase acts on AA to produce 5-hydroperoxyeicosatetraenoic acid (5-HPETE) that is converted to $LTA_4$. $LTA_4$ is the precursor of $LTB_4$, that induces inflammation by its chemotactic and degranulating actions on polymorphonuclear lymphocytes (PML), and of $LTC_4$, $LTD_4$, and $LTE_4$, the amino acid containing LTs that induce vasoconstriction and bronchoconstriction and are involved in asthma and anaphylaxis.

In this invention, 11 genes (or proteins) are included into the array analysis as characteristic of the lipid metabolism (table 1). They comprise : ANX1, APOB, APOE, APOJ, COX1, COX2, PLA2G4A, PLA2G2A, PLA2G6, PPARA and PPARG.

### 5. Proteasome

Intracellular proteolysis occurs via two pathways: a lysosomal pathway and a non-lysosomal ATP-dependent pathway. The latter, which is known to degrade most cell proteins including regulatory proteins, requires first covalent linking of proteins to multiple molecules of the polypeptide ubiquitin. This modification has the effect to mark the protein for rapid degradation by the proteasome, a 26S (200 kD) complex which, in mammalian cells, contains a 2OS (673 kD) proteasome or multicatalytic protease complex (MCP) as the key proteolytic component and a 19S complex containing several ATPases and a binding site for ubiquitin chains. The role of this 19S particle, which "caps" each extremity of the 20S proteasome, is to unfold the protein substrates to inject them into the 20S proteasome and to stimulate the proteolytic activity.

Despite the difference in subunit composition, the proteasome from archaebacteria to eukaryotes have the same basic architecture, which under the electron microscope appears as a cylinder shaped particle, made up of four stacked rings with dimensions of approximately 15 nm in height and 11 nm in diameter. Eukaryotic proteasomes have a more complex structure than the proteasome from the archaebacterium *Thermoplasma acidophilum,* which contains only two different subunits, alpha and beta of 25.8 kD and 22.3 kD respectively.

This simpler structure of the bacterial proteasome has allowed the recent elucidation of its 3D structure by X-ray crystallography. Results reveal that the T. acidophilum 20S proteasome is composed of 28 subunits: 14 $\alpha$-subunits and 14-$\beta$-subunits that form a four stacked ring. The two outer rings consist of seven alpha subunits and the two inner consist of seven beta-subunits. This alpha7beta7beta7alpha7 assembly forms a central channel with three chambers: two antechambers located on opposite sides of a central chamber. Binding studies with the peptide aldehyde acetyl-Leu-Leu-norleucinal (Calpain inhibitor I) reveal 14 catalytic sites within the central chamber. The specificity of the proteasome seems to be rather unspecific but the size of the hydrolysis products is always between 6 and 9 residues. This corresponds to the length between adjacent catalytic sites in the central chamber, which probably means that the substrate must be channeled into a single 20S molecule during the hydrolysis process. This generation of peptides of defined length is of biological relevance in the context of the implication of the eukaryotic proteasome in the antigen presentation by MHC molecules during the T-cell immune response.

In this invention, 41 genes (or proteins) are included into the array analysis as an appropriate characteristic of the proteasome pathways (table 1). They comprise : PSMA1, PSMA2, PSMA3, PSMA4, PSMA5, PSMA6, PSMA7, PSMB1, PSMB2, PSMB3, PSMB4, PSMB5, PSMB6, PSMB7, PSMB8, PSMB9, PSMB10, PSMC1, PSMC2, PSMC3, PSMC4, PSMC5, PSMC6, PSMD1, PSMD2, PSMD3, PSMD4, PSMD5, PSMD6, PSMD7, PSMD8, PSMD9, PSMD10, PSMD11, PSMD12, PSMD13, PSMD14, PSME1, PSME2, PSME3 and UBE2C.

**6. Blood circulation**

Because of the importance of blood in regulating pH and the transport of oxygen, nutrients, carbon dioxide, and wastes, maintaining the integrity of the process is crucial to life. When ruptures in the system do occur, the process of blood clotting is initiated as an emergency measure to halt the loss of blood. Biochemically, blood clotting is an example of signal amplification caused by the simultaneous activation and inhibition of many enzymes. When injury to a blood vessel occurs, three major events happen to rapidly stop the loss of blood:

1) Clumping of blood platelets at the site of injury to create a physical plug.
2) Vasoconstriction occurs to reduce blood flow through the area.
3) Aggregation of fibrin into an insoluble clot that covers the rupture and stops loss of blood.

The clot is dissolved after actual repair of the blood vessel.

The initial phase of platelet aggregation is a complex formed between the platelets and underlying collagen fibrils exposed in the ruptured vessel. An additional circulating protein, von Willebrand factor (vWF), mediates binding of platelets to collagen and each other resulting in activation of the platelets and release of various activators.

In the normal, undamaged vascular endothelium, platelet aggregation does not occur since collagen fibrils are not exposed and other activating factors (like ADP) are not present in sufficient amounts. Besides exposure of the collagen fibrils in the underlying matrix of the vessel, other membrane proteins in this matrix are exposed to the circulating blood. It is these matrix and membrane proteins that serve as receptors for the various zymogens and protein co-factors that are released by the activated platelets (or that were already present in the blood).

Ultimately, the final blood clot is formed by the conversion of fibrinogen to fibrin eventually resulting in insoluble, cross-linked fibrin polymers. Two activation pathways (initiated by complexes with exposed membrane matrix proteins), historically termed the extrinsic and intrinsic pathways, supply the protease (Factor Xa) that activates the thrombin catalyzed production of fibrin.

Similar mechanisms of activation and inactivation described for clot formation apply to clot dissolving (fibrinolysis). Plasmin, which exists in circulating blood as plasminogen, is the protease responsible for degrading the fibrin clots. The activator of plasminogen, another protease termed tissue plasminogen activator (tPA), binds with high affinity to the fibrin clot along with plasminogen. The tPA-plasminogen-fibrin complex results in proteolytic activation of plasminogen to plasmin, which then begins digesting the fibrin. To keep one plasmin molecule from degrading the whole clot, after plasmin degrades a region of the clot, the resulting digested peptides dissociate from the clot and take the plasmin-tPA complex with them. Again similar to the antiprotease factors described above, anti-plasmin and anti-tPA proteins (PAI1, PAI2) have been described that perform the same function as the anticoagulation proteins.

In this invention, 8 genes (or proteins) are included into the array analysis as characteristic of the blood circulation pathway (table 1). They comprise : EDN1, F3, THBD, PAI1, PAI2, PLAU, TPA and VWF.

The "Generalchip" as described herein, i.e. containing at least 9 cellular functions derived from the vital functions indicated allows to determine the status of a cell and changes during a biological process. Such a general picture of the changes is also obtained when used in combination with detection of specific functions.

According to a preferred embodiment, the step of detecting and optionally quantifying the pattern of hybridization on the array is performed on a single capture nucleotide species and/or the values for the quantification on the arrays are taken as the average of three experimental data.

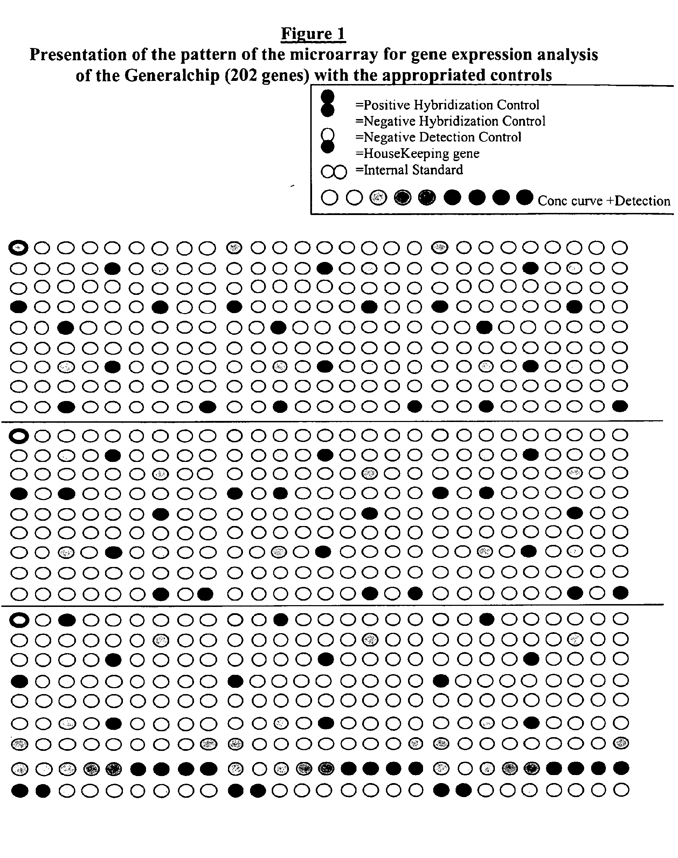

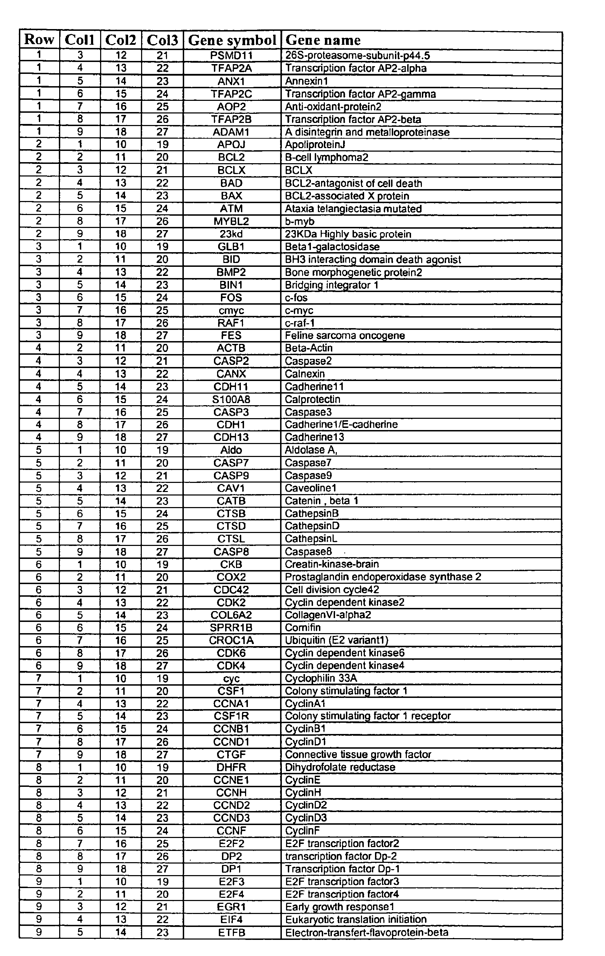

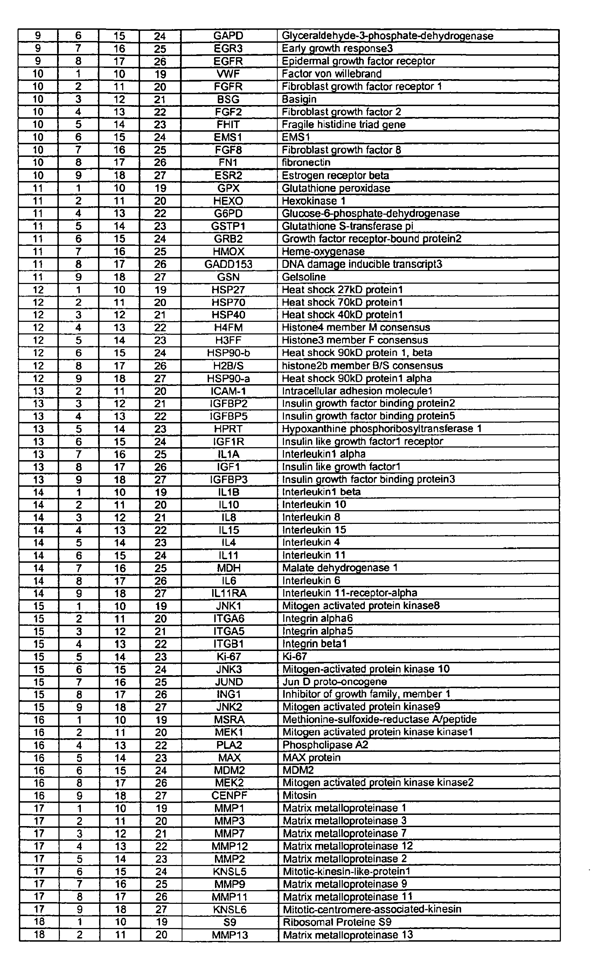

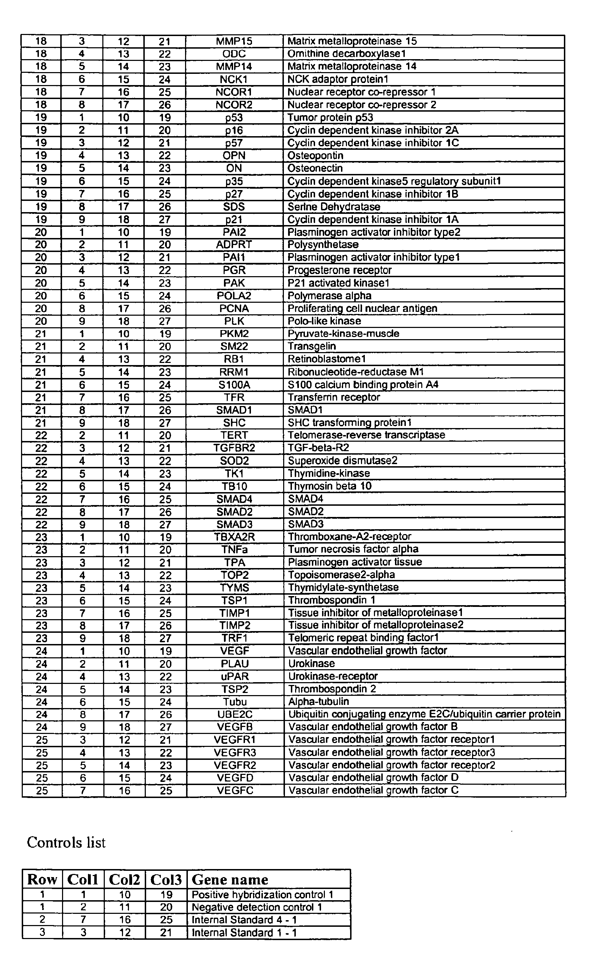

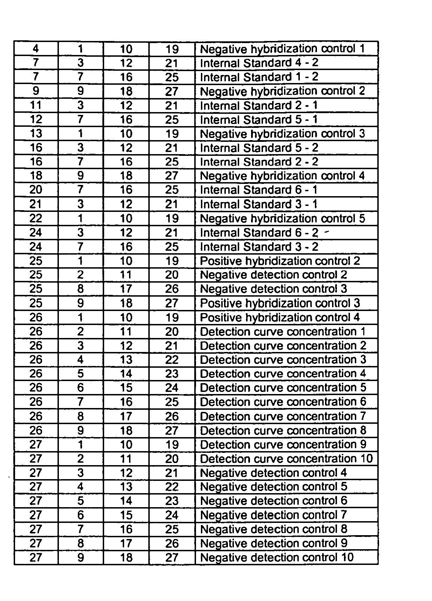

According to another preferred embodiment, the number of nucleic acids or proteins to be detected is maximum of 999. Specifically, the analysis of the vital functions may be performed on the array (Generalchip) as presented in figure 1. The arrays allows the simultaneous analysis of 202 different genes belonging to 13 vital functions. Each gene detection is performed in triplicates. The value for the presence of each gene is the average of the three values and the mean is calculated together with the standard deviation. Each of the value is then corrected using internal standards which have been added in the analysis in a given concentration. Thereafter, a correction for house keeping genes is also performed as an option for variations within the arrays. This process gives absolute values for the genes present in a test experiment compared to a control condition or to reference sample. The genes which are significantly modified in a given experimental condition are presented in table 2.

According to a preferred embodiment the present methods are used to compare cellular conditions, wherein at least one gene for each of the 9 vital cellular functions is expressed differentially, said method further comprising the step of comparing the transcriptome of cells or tissues in a given biological condition with a reference or control condition. This control condition may differ from the sample condition in respect of the cellular microenvironment, in respect of exposure to a physiological stimulus, hormones, growth factors, cytokines, chemokines, inflammatory agents, toxins, metabolites, pH, chemical and/or pharmaceutical agents, hypoxia, anoxia, ischemia, imbalance of any plasma-borne nutrient, osmotic stress, temperature, mechanical stress, irradiation, cell-extracellular matrix interactions, cell-cell interactions, accumulations of foreign or pathological extracellular components, intracellular and extracellular pathogens, or a genetic perturbation.

According to a preferred embodiment the control condition differs in that the sample cells have been exposed to a physiological stimulus, which may be a mechanical, temperature, chemical, toxic or pharmaceutical stress.

The present invention therefore enables a quick determination of the effect of different influences on the general cellular condition/performance.

According to another preferred embodiment the array provides at least 20 different capture probes for at least one nucleic acid for each of the 9 vital cellular functions.

The vital functions of a cell may be any function that fulfil the above definition. Yet, according to a preferred embodiment the vital functions on the array are represented by at least 2 genes of the table 1, and more preferably are derived from table 1.

According to another preferred embodiment at least one gene for each of the 9 vital functions is a gene which effects a regulatory activity in the function.

The cell to be investigated may be any prokaryotic or eucaryotic cell but is preferably a cell selected from the group consisting of cardiomyocytes, endothelial cells, sensory neurons, motor neurons, CNS neurons, astrocytes, glial cells, Schwann cells, mast cells, eosinophils, smooth muscle cells, skeletal muscle cells, pericytes, lymphocytes, tumor cells, monocytes, macrophages, foamy macrophages, dentritic cells, granulocytes, melanocytes, keratinocytes, synovial cells/synovial fibroblasts and epithelial cells.

The array to be used may be any conventional "biochip structure" on which corresponding biological samples may be spotted. According to one embodiment the array comprises polynucleotide sequences and/or peptidic sequences.

According to a preferred embodiment the biological test sample and the control experimental conditions are analyzed on the same support.

According to another preferred embodiment at least one gene of 5 vital functions is expressed differentially together with at least 5 genes of a specific function.

According to another preferred embodiment the two dimensional array provides capture probes for at least one gene of each of the 5 vital functions together with at least 5 genes of a specific function.

According to an alternative embodiment the present invention provides a kit for the determination of the general condition of a cell, which kit comprises an array, containing on predetermined locations thereof a maximum of 2999 nucleic acids or proteins belonging to or representative for at least 5 of the following vital cellular functions: apoptosis, cell adhesion, cell cycle, growth factors and cytokines, cell signaling, chromosomal processing, DNA repair/synthesis, intermediate metabolism, extracellular matrix, cell structure, protein metabolism, oxidative metabolism, transcription and house keeping genes.

According to a further alternative embodiment the array in the kit comprises on predetermined locations thereof a maximum of 2999 nucleic acids or proteins belonging to or representative for at least 5 of the following vital cellular functions: apoptosis, cell adhesion, cell cycle, growth factors and cytokines, cell signaling, chromosomal processing, DNA repair/synthesis, intermediate metabolism, extracellular matrix, cell structure, protein metabolism, oxidative metabolism, transcription and house keeping genes, and at least one nucleic acid or protein, belonging to or representative for at least one of the following specific functions : cell differentiation, oncogene/tumor suppressor, stress response, lipid metabolism proteasome, circulation, wherein the array comprises at least 20 different spot compositions and a maximum of 2999 different spots.

The method and the kit of the present invention may be used for the determination of the current status of a cell.

To this end, in case e.g. a cell (e.g. a skin cell) is exposed to UV light, the radiation induces damages in the DNA of the cells. In such a case, the results on micro-array provides evidence of activation of DNA repair genes since DNA is the first target of the UV stress. In addition, the use of a "General-chip" covering in this case 13 vital functions will give a good overview of the overall cell response. For example, an inhibition of 4 genes associated with the cell cycle especially the cyclins, will indicate that DNA is repaired before cell division.

In case of determining, whether a cell, e.g. a keratinocyte, is undergoing cell differentiation, it will be determined, whether the KRT1, KRT10 and FLG genes are induced as previously described (Poumay and Pittelkow 1995, J. Invest. Dermatol., 104, 271-76).

As regards the determination of cell cycle a Cyclin B reduced expression will reflect a cell cycle slow down or arrest imposed by e.g. a confluent status of the culture, which in turn induces the differentiation process.

The differentiation process is associated with a rearrangement of the expression level of multiple genes (50% from the category) responsible of the extracellular matrix composition.

E.g. general functions, such as cell division and migration may be linked to some oncogenic status of the cell.

TNF has since long been associated with the potential of tumor clearance. TNF induces the general cell adhesion modifications (ICAM-1 gene) which in principle would allow adhesion of leukocytes to the cells. This is further accompanied by the increased expression of interleukins (IL1$\alpha$- and IL-8) potentially mobilising an immune response against the transformed cells.

Alternatively, the method and the kit of the present invention may be used for the determination of changes of gene expression occurring in particular conditions a cell is subjected. Proceeding accordingly will allow to e.g. elucidate the role of particular genes in a given physiological event, such as stress, ageing, stem cell differentiation, haematopoiesis, neuronal functional status, diabetes, obesity, transformation process such as carcinogenesis, protein turnover or circulatory disorders as atherosclerosis.

Decomposing the analysis process in clearly defined functional classes enables to reconstitute an integrated biological interpretation of a biological process in a complete cell.

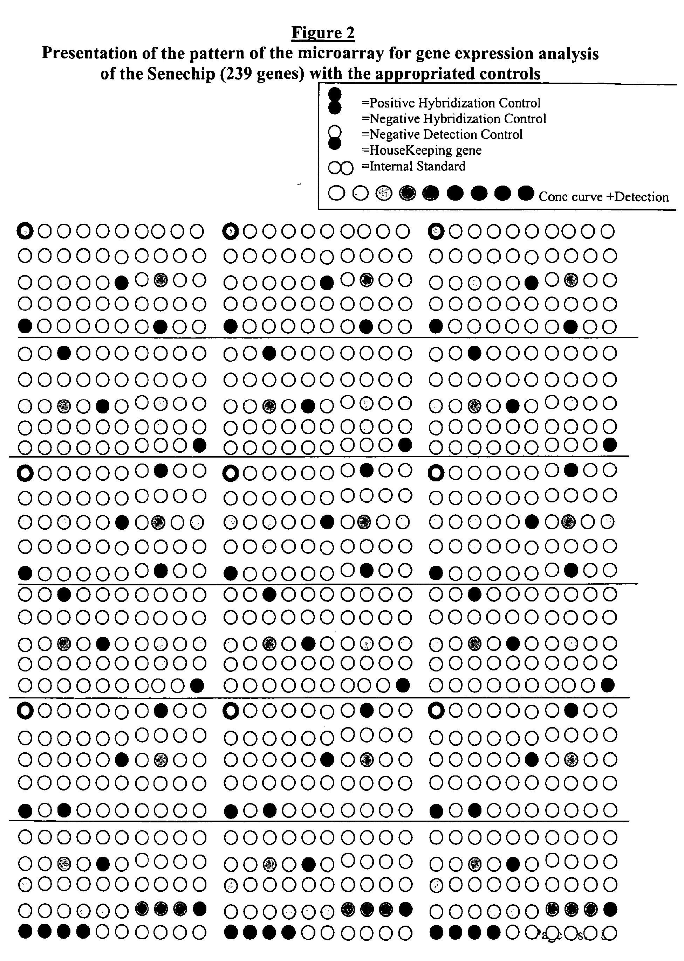

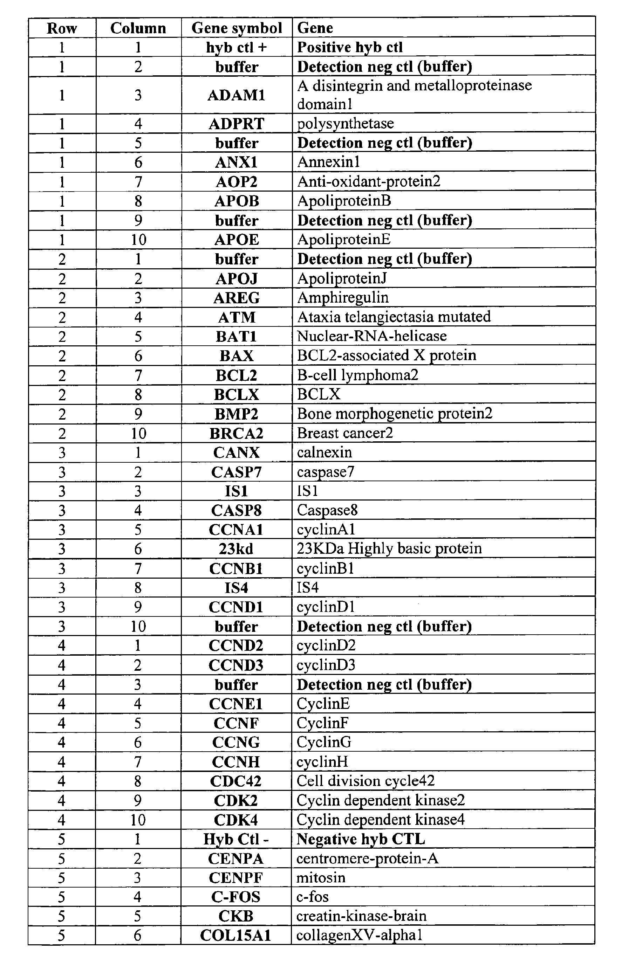

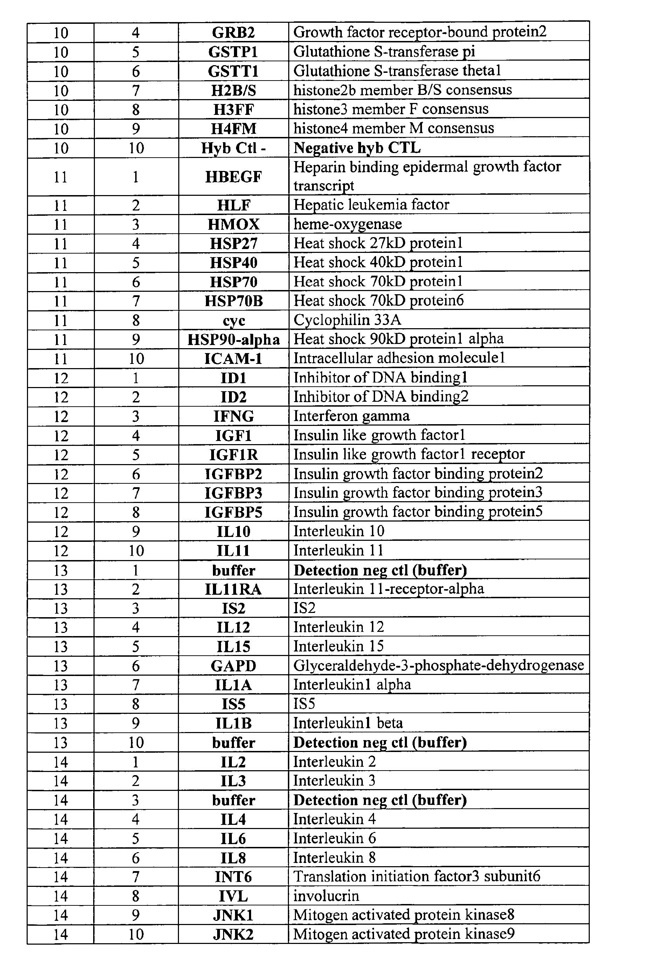

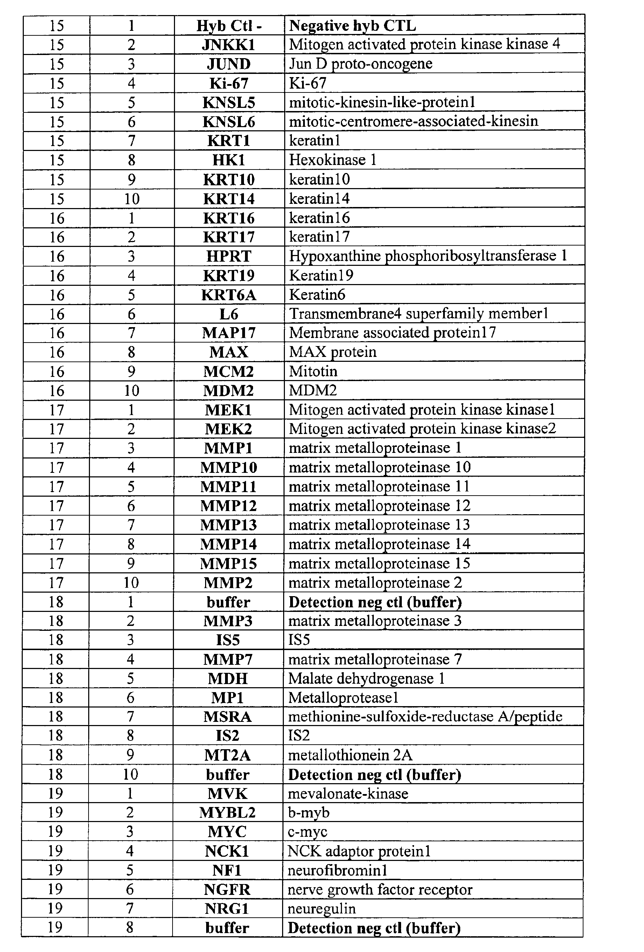

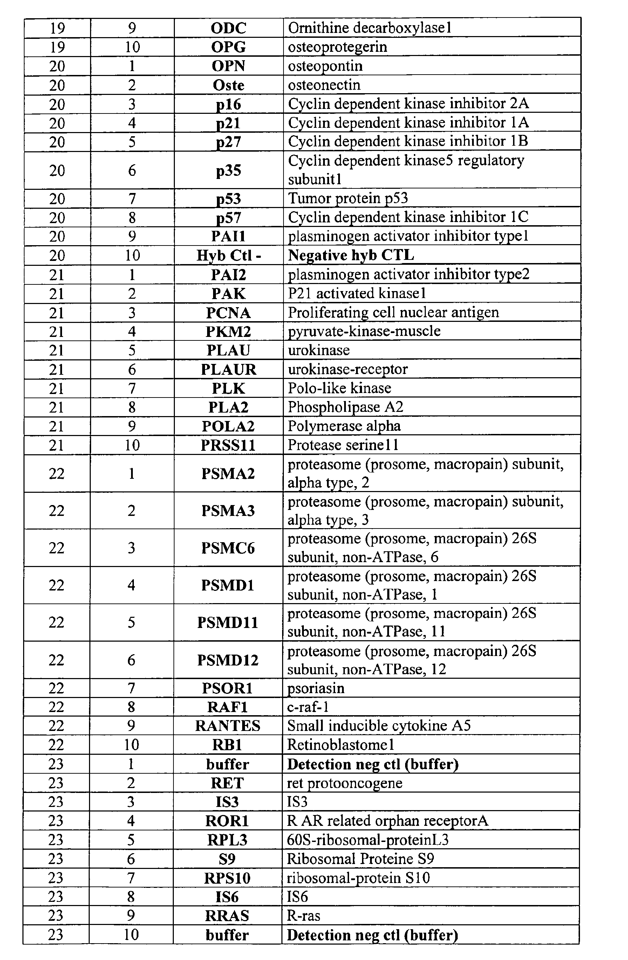

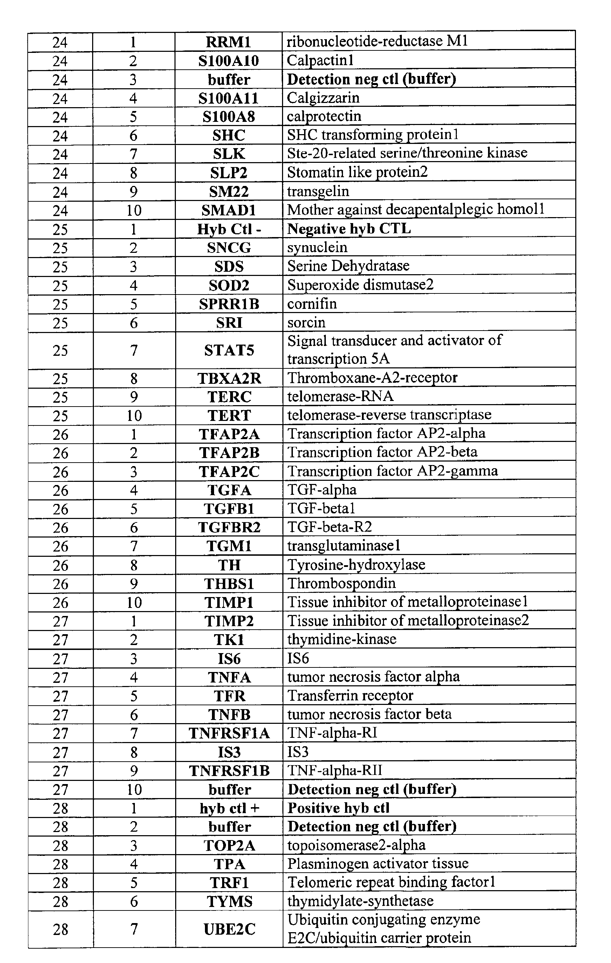

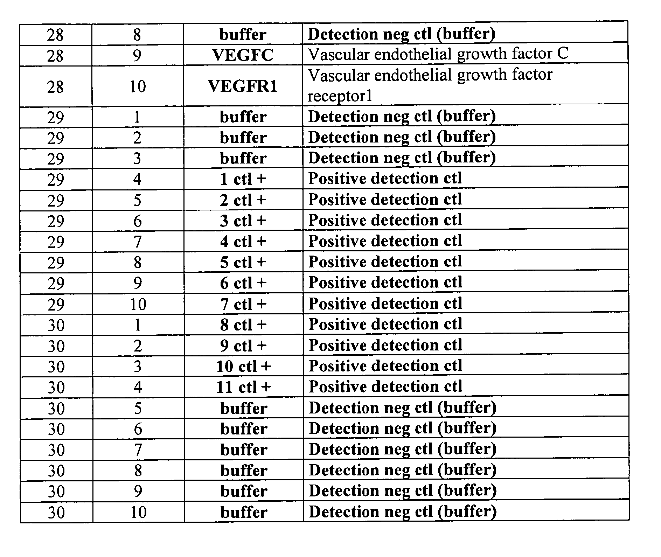

In another particular embodiment, the analysis of variations within cells due to stress or/and ageing is performed on arrays which design is presented in figure 2. The arrays can detect and quantify 239 different genes. The genes belong to 13 vital functions and to 15 genes belonging to the specific stress and ageing process. The method of using the arrays and analysis of the genes are the same as for the "Generalchip". The genes which are significantly modified in a given experimental condition are presented in table 2.

According to another embodiment, the cells, tissues or organisms are contacted with a substance of interest and the effect of the substance on the status/performance of the cell is monitored. The two dimensional analysis of the spots intensity allows a quantification of the changes of the gene products within the cells compared to cells not contacted with the given compound. The invention is particularly useful to follow cellular reactions in the presence of biological or chemical compounds. Variations in the level of the genes or gene products are determined and give a first overview of the changes occurring in the biological organisms, cells or tissues, in reaction to the presence of the compound. Thereafter, specific analysis based on data mining linking the various cellular functions and pathways provide the necessary information on the mechanism behind the presence of the given compound. Compounds comprise: biological molecules such as cytokines, growth hormones, or any biological molecules affecting cells. It also comprises chemical compounds such as drugs, toxic molecules, compounds from plants or animal extracts, chemicals resulting from organic synthesis including combinatory chemistry.

In one embodiment, biological and control experimental conditions differ in respect of the cellular microenvironment, or in respect of exposure to hormones, growth factors, cytokines, chemokines, inflammatory agents, toxins, metabolites, pH, pharmaceutical agents, hypoxia, anoxia, ischemia, imbalance of any plasma-borne nutrient, osmotic stress, temperature, mechanical stress, irradiation, cell-extracellular matrix interactions, cell-cell interactions, accumulations of foreign or pathological extracellular components, intracellular and extracellular pathogens, or a genetic perturbation.

In one embodiment, screening compounds affect cellular vital functions.

In another embodiment, screening compounds affects cellular specific functions.

In one embodiment, cells, tissues or organisms are incubated in particular physical, chemical or biological conditions and the analysis is performed according to the present methods. The particular physical conditions means only conditions in which a physical parameter has been changed such as pH, temperature, pressure. The particular chemical conditions mean any conditions in which the concentration of one or several chemicals have been changed as compared to a control or reference condition including salts, oxygen, nutriments, proteins, glucides (carbohydrates), and lipids. The particular biological conditions mean any changes in the living cells, tissues or organisms including ageing, stress, transformation (cancer), pathology, which affect cells, tissues or organisms.

In one specific embodiment, the genes detected as associated with a function are genes which encode for regulatory activity in the function.

In another embodiment, the genes detected as associated with a function are regulated on the transcriptional level.

The genes as mentioned here to be spotted on the array may be derived from public databases (i.e. the gene ontology project at http://www.geneontology.org/).

The following examples illustrate the invention without limiting it thereto.

**Examples**

Example 1:

Detection of gene expression on "Generalchip": example of activity of a cell under UV stress Cultures of human skin fibroblasts (AG04431, Coriell Institute for Medical Research (USA)) at early cumulative population doublings were submitted to UVB stress. Cells were exposed twice a day during five days to a UVB radiation of 250 mJ/cm2 using three Philips TL 20W/01 lamps (Philips, The Netherlands). Control samples were submitted to the same conditions without UVB illumination. Cells were lysed 72 hours after the last stress and mRNA was harvested before retro-transcription according to the following instructions.

1. RNA extraction:

Poly(A$^+$) RNA (mRNA) was extracted using FastTrack columns (InVitrogen). Poly(A+) RNA was resuspended in RNAse-free water.

The concentration and purity of RNA was determined by diluting an aliquot of the preparation in TE (10mM Tris-HCl pH 8, 1mM EDTA) and measuring (reading) its absorbance (in a spectrophotometer) at 260 nM and 280 nm.

While the A260 value allows to evaluate the RNA concentration, the A260/A280 ratio gives an indication of the RNA purity. For a RNA to be used, its ratio must be comprised between 1.8 and 2.

The overall quality of the RNA preparation was determined by electrophoresis on a denaturing 1% agarose gel (Sambrook et al., eds. (1989) Molecular Cloning - A Laboratory Manual, 2nd ed. Cold Spring Harbor, NY: Cold Spring Harbor Laboratory Press).

2. cDNA synthesis:

1 µl of poly(A$^+$) RNA sample (0.5 µg/µl) was mixed with 2 µl oligo(dT)$_{12-18}$ (0.5 µg/µl, Roche), 3.5 µl H$_2$O, and 3 µl of a solution of 3 different synthetic well-defined poly(A+) RNAs. These latter served as internal standards to assist in quantification and estimation of experimental variation introduced during the subsequent steps of analysis. After an incubation of 10 minutes at 70°C and 5 minutes on ice, 9 µl of reaction mix were added. Reaction mix consisted in 4 µl Reverse Transcription Buffer 5X (Gibco BRL), 1 µl RNAsin Ribonuclease Inhibitor (40 U/ml, Promega), and 2 µl of a 10X dNTP mix, made of dATP, dTTP, dGTP (5 mM each, Roche), dCTP (800 µM, Roche), and Biotin-11-dCTP (800 µM, NEN).

After 5 min at room temperature, 1.5 µl Superscript II (200 U/ml, Gibco BRL) was added and incubation was performed at 42°C for 90 minutes. Addition of Superscript and incubation were repeated once. The mixture was then placed at 70°C for 15 minutes and 1 µl Ribonuclease H (2U/µl) was added for 20 minutes at 37°C. Finally, a 3-min denaturation step was performed at 95°C. The biotinylated cDNA, was kept at -20°C.

3. Hybridization of (using) biotinylated cDNA:

A "Generalchip" used in this study is composed of 202 genes representative of the vital cellular functions as provided in table 1. The design of the chip is presented in figure 1. Each capture molecule for the gene is present in triplicate. The arrays also contain several different controls including positive and negative detection control, positive and negative hybridization control, three different internal standards all dispersed at different locations among the genes to be analyzed on the micro-array. In this example each spots was covered with a capture probe being a polynucleotide species, which allow the specific binding of one target polynucleotide, corresponding to a specific gene listed in table 1. All sequences have been designed to be gene specific and have been prepared using human cDNA clones.

Hybridization chambers were from Biozym (Landgraaf, The Netherlands). Hybridization mixture consisted in biotinylated cDNA (the total amount of labeled cDNA), 6.5 µl HybriBuffer A (Eppendorf, Hamburg, Germany), 26 µl HybriBuffer B (Eppendorf, Hamburg, Germany), 8 µl H$_2$O, and 2 µl of positive hybridization control.

Hybridization was carried out overnight at 60°C. The micro-arrays were then washed 4 times for 2 min with washing buffer (Eppendorf, Hamburg, Germany).

The micro-arrays were than incubated for 45 min at room temperature with the Cy3-conjugated IgG Anti biotin (Jackson Immuno Research laboratories, Inc #200-162-096) diluted 1/1000 X Conjugate-Cy3 in the blocking reagent and protect from light.

The micro-arrays were washed again 4 times for 2 minutes with washing buffer and 2 times for 2 minutes with distilled water before being dried under a flux of $N_2$.

4. Scanning and data analysis:

The hybridized micro-arrays were scanned using a laser confocal scanner "ScanArray" (Packard, USA) at a resolution of 10 μm. To maximize the dynamic range of the assay the same arrays were scanned at different photomultiplier tube (PMT) settings. After image acquisition, the scanned 16-bit images were imported to the software, 'ImaGene4.0' (BioDiscovery, Los Angeles, CA, USA), which was used to quantify the signal intensities. Data mining and determination of significantly expressed gene in the test compared to the reference arrays was performed according to the method described by Delongueville et al (Biochem Pharmacol. 2002 Jul 1; 64(1): 137-49). Briefly, the spots intensities were first corrected for the local background and than the ratios between the test and the reference arrays were calculated. To account variation in the different experimental steps, the data obtained from different hybridizations were normalized in two ways. First the values are corrected using a factor calculated from the intensity ratios of the internal standard reference and the test sample. The presence of 3 internal standard probes at different locations on the micro-array allows measurement of a local background and evaluation of the micro-array homogeneity, which is going to be considered in the normalization (Schuchhardt et al., Nucleic Acids Res. 28 (2000), E47). However, the internal standard control does not account for the quality of the mRNA samples, therefore a second step of normalization was performed based on the expression levels of housekeeping genes. This process involves calculating the average intensity for a set of housekeeping genes, the expression of which is not expected to vary significantly. The variance of the normalized set of housekeeping genes is used to generate an estimate of expected variance, leading to a predicted confidence interval for testing the significance of the ratios obtained (Chen et al, J. Biomed. Optics 1997, 2, 364-74). Ratios outside the 95% confidence interval were determined to be significantly changed by the treatment.

Example 2:

Detection of gene expression: comparison between proliferating (subconfluent) and growth-arrested differentiating (confluent) epidermal keratinocytes

Culture of human adult epidermic keratinocytes in autocrine conditions (no peptide in the culture medium) were used in this example. Subconfluent cells (control sample) were compared to confluent cells (test sample) Poumay and Pittelkow (1995) J. Invest. Dermatol. 104, 271-276: Cell density and culture factors regulate keratinocyte commitment to differentiation and expression of suprabasal K1/K10 keratins). The cell density induces epidermic differentiation.

The experimental protocol is the same as in example 1. However, the array used for the hybridization of biotinylated cDNA is the Senechip (EAT, Naumur, Belgium). It is composed of 239 genes and several different controls including positive and negative detection control, positive and negative hybridization control, three different internal standards all dispersed at different locations among the genes to be analyzed on the micro-array (Fig. 2). Each capture molecule for the gene is present in triplicate. In this example each spots was covered with a capture probe being a polynucleotide species which allow the specific binding of one target polynucleotide corresponding to a specific gene listed in table 1.

In example 2, the procedure was applied to the data obtained when performing gene expression profiling of keratinocytes that were grown to confluence.

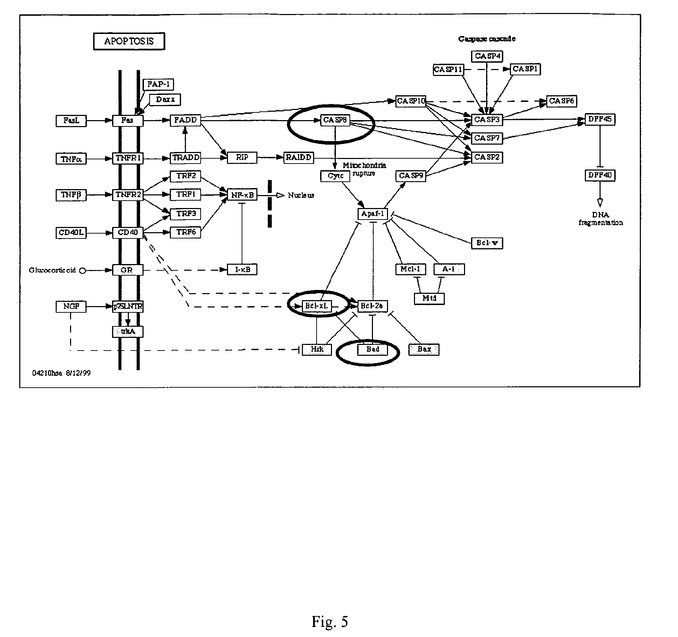

In the apoptosis gene list from example 2, three genes, show statistically significant transcript level changes (see table 2 for change ratios), namely CASP8, Bcl-xL and Bad (cf. Fig. 5).

BCL-X and Bad, which are both members of the apoptosis inhibitory cascade are down regulated whilst CASP8, the prominent caspase (a protease) has an increased expression level in cells that have attained confluence.

The integration of this information is further illustrated in the Cell cycle genes category where numerous genes including all Cyclins (CycA,B,C,D,E,H and E) show statistically significant reduction of transcription clearly illustrating that keratinocytes have a reduced growth process when reaching confluence.

The analysis of the results within the two maps lead to the conclusion that when keratinocytes reach confluence, growth is arrested and conditions for undergoing apoptosis are met.

This analysis can be extended to the other vital and specific gene categories allowing to reconstitute an integrated picture of the biological process.

Finally, the positioning of genes in the context of specific predefined categories allows for a very straightforward interpretation and understanding of which are the processes taking place in the hybridized cell or sample and to understand how multiple processes interrelate.

In doing so, the information recovered by the micro-arrays described in the invention allow for an improved rendering and unification of a higher order or 3D organization of the cell.

Example 3:

Detection of gene expression : example of activity of a cell after TNF-$\alpha$ activation

Culture of human endothelial HUV-EC-C cells (ATCC n°CRL-1730) at cumulative population doublings of G44.5 were submitted to TNF-$\alpha$ stimulation. Cell were first rinsed once with a cell medium that do not contain growth factors (F12K from Gibco) and then incubated during 3 hours with TNF-$\alpha$ (TNF-$\alpha$ from R&D at 10 ng/ml in ethanol 0.01 %). Control samples were submitted to the same conditions without TNF-$\alpha$ activation (ethanol 0.01%). A pool of 3 T75 ($\pm$ 5.10$^6$ cells) were lysed and mRNA was harvested before retrotranscription according to the experimental protocol described in example 1.

Table 2 Values of genes expression which are statistically significant in the study of either the cell vital function in the generalchi or in association with the stress and ageing process on the senechip
Example 1. Detection of gene expression on a 2D array : activity of a cell after a UV stress
Example 2. Detection of gene expression on a 2D array : comparison between proliferating (subconfluent) and growth-arrested differentiating (confluent) epidermal keratinocytes
Example 3. Detection of gene expression on a 2D array : activation of a cell after TNF-apha treatment

| Gene symbol | Gene bank # | A. Vital function | B. Specific function | Example 1 | Example 2 | Example 3 |
|---|---|---|---|---|---|---|
| BAD | NM_004322 | Apoptosis | | 0,53 | | |
| BAX | NM_004324 | Apoptosis | | | 0,17 | |
| BCL2 | NM_000633 | Apoptosis | | | | |
| BCLX | NM_001191 | Apoptosis | | 0,47 | 0,24 | |
| BID | NM_001196 | Apoptosis | | | | 2,62 |
| CASP2 | NM_001224 | Apoptosis | | | | |
| CASP3 | NM_004346 | Apoptosis | | | | |
| CASP7 | NM_001227 | Apoptosis | | | | 4,30 |
| CASP8 | X98172 | Apoptosis | | | 1,6 | |
| CASP9 | NM_001229 | Apoptosis | | | | |
| CATB | NM_001904 | Cell adhesion | | | | |
| CD36 | NM_000361 | Cell adhesion | | | | |
| CDH1 | NM004360 | Cell adhesion | | | | |
| CDH5 | NM_001795 | Cell adhesion | | | | |
| CDH11 | NM_001797 | Cell adhesion | | | | |
| CDH13 | U59289 | Cell adhesion | | 0,39 | | |
| DSG1 | AF097935 | Cell adhesion | | | 25,62 | |
| ICAM-1 | J03132 | Cell adhesion | | 0,49 | 0,57 | 13,52 |
| ITGA4 | NM_000885 | Cell adhesion | | | | |
| ITGA5 | NM_002205 | Cell adhesion | | | | |
| ITGA6 | NM_000210 | Cell adhesion | | | | |
| ITGB1 | NM_002211 | Cell adhesion | | | | |
| ITGB2 | NM_000211 | Cell adhesion | | | | |
| ITGB3 | NM_000212 | Cell adhesion | | | | |
| PECAM1 | NM_000442 | Cell adhesion | | | | |
| SELE | NM_000450 | Cell adhesion | | | | |
| SELL | NM_000655 | Cell adhesion | | | | |
| RANTES | NM_002985 | Cell adhesion | | | 1,68 | |
| TSP1 | NM_003246 | Cell adhesion | | | 0,12 | |
| TSP2 | NM_003247 | Cell adhesion | | | | |
| VCAM1 | NM_001078 | Cell adhesion | | | | |
| ATM | U26455 | Cell cycle | | | | |
| CAV1 | NM_001753 | Cell cycle | | | 0,65 | |
| CCNA1 | NM_003914 | Cell cycle | | | 0,65 | |
| CCNB1 | NM_031966 | Cell cycle | | | 0,05 | |
| CCND1 | NM_053056 | Cell cycle | | 0,61 | | |
| CCND2 | NM_001759 | Cell cycle | | | 0,59 | |
| CCND3 | NM_001760 | Cell cycle | | 0,51 | 0,55 | |
| CCNE1 | NM_001238 | Cell cycle | | | 0,56 | |
| CCNF | NM_001761 | Cell cycle | | 0,38 | 0,54 | |
| CCNG | U53328 | Cell cycle | | | | |
| CCNH | NM_001239 | Cell cycle | | 0,62 | 0,54 | |
| CDK2 | NM_001798 | Cell cycle | | | | |
| CDK4 | U79269 | Cell cycle | | | 0,54 | |
| CDK6 | NM001259 | Cell cycle | | | | 3,34 |
| DHFR | NM_000791 | Cell cycle | | | | |
| FE65 | L77864 | Cell cycle | | | | |
| GRB2 | NM_002086 | Cell cycle | | | | |
| HLF | M95585 | Cell cycle | | | | |
| MCM2 | D21063 | Cell cycle | | | 0,42 | |
| MDM2 | NM_002392 | Cell cycle | | | | |
| MKI67 | NM_002417 | Cell cycle | | 0,39 | 0,65 | |
| p16 | L27211 | Cell cycle | | 1,78 | | |
| p21 | U03106 | Cell cycle | | | | |
| p27 | NM_004064 | Cell cycle | | | | |
| p35 | NM_003885 | Cell cycle | | | 0,52 | |
| p53 | AF307851 | Cell cycle | | | 0,63 | |
| p57 | NM_000076 | Cell cycle | | 1,84 | 2,25 | |
| PCNA | NM002592 | Cell cycle | | | 0,61 | |
| RB1 | NM_000321 | Cell cycle | | | 0,47 | |
| SMAD1 | U59423 | Cell cycle | | | 0,62 | |
| SMAD2 | U68018 | Cell cycle | | | | |
| SMAD3 | U68019 | Cell cycle | | | | 1,67 |
| SMAD4 | U44378 | Cell cycle | | | | |
| S100A10 | M81457 | Cell cycle | | | | |
| S100A4 | NM_002961 | Cell cycle | | | | |
| S100A8 | NM_002964 | Cell cycle | | | 2,61 | |

| Gene | Accession | Function | | | Value |
|------|-----------|----------|---|---|-------|
| TK1 | NM_003258 | Cell cycle | | | 0,43 |
| CST6 | U62800 | Cell differentiation | | | |
| FLG | M60502 | Cell differentiation | | | 23,12 |
| ID1 | X77956 | Cell differentiation | | | 1,66 |
| ID2 | M97796 | Cell differentiation | | | 1,58 |
| IVL | M13903 | Cell differentiation | | | 2,71 |
| KRT1 | NM_006121 | Cell differentiation | | | 46,37 |
| CYT2A | M99063 | Cell differentiation | | | |
| KRT6A | NM_005554 | Cell differentiation | | | |
| KRT10 | NM_000421 | Cell differentiation | | | 18,13 |
| KRT14 | NM_000526 | Cell differentiation | | | |
| KRT16 | AF061812 | Cell differentiation | | | 2,29 |
| KRT17 | X62571 | Cell differentiation | | | |
| KRT19 | NM_002276 | Cell differentiation | | | |
| NRG1 | M94165 | Cell differentiation | | | 0,16 |
| OPG | U94332 | Cell differentiation | | | |
| PSOR1 | M86757 | Cell differentiation | | | 1,93 |
| SPRR1B | NM_003125 | Cell differentiation | | | 2,67 |
| TH | NM_000360 | Cell differentiation | | | 2,48 |
| TBXAS1 | NM_012687 | Neuronal cell differentiation | | | |
| CD47 | NM_019195 | Neuronal cell differentiation | | | |
| Rnpep | NM_031097 | Neuronal cell differentiation | | | |
| Ph2C | NM_022606 | Neuronal cell differentiation | | | |
| Pcbp4 | BC010694 | Neuronal cell differentiation | | | |
| PENK2 | NM_017139 | Neuronal cell differentiation | | | |
| Txn | X14878 | Neuronal cell differentiation | | | |
| Baiap2 | NM_057196 | Neuronal cell differentiation | | | |
| Sv2b | NM_057207 | Neuronal cell differentiation | | | |
| CBL20 | NM_139104 | Neuronal cell differentiation | | | |
| Tac 1 | NM_012666 | Neuronal cell differentiation | | | |
| Arha2 | NM_057132 | Neuronal cell differentiation | | | |
| Ndufv1 | XM_215176 | Neuronal cell differentiation | | | |
| Nlgn2 | NM_053992 | Neuronal cell differentiation | | | |
| Rps23 | NM_078617 | Neuronal cell differentiation | | | |
| Mxi1 | NM_013160 | Neuronal cell differentiation | | | |
| Gbp2 | NM_133624 | Neuronal cell differentiation | | | |
| Nudel | NM_133320 | Neuronal cell differentiation | | | |
| Sv2b | NM_057207 | Neuronal cell differentiation | | | |
| VL30 | M91235 | Neuronal cell differentiation | | | |
| Sult4a1 | NM_031641 | Neuronal cell differentiation | | | |
| Masp2 | XM_216574 | Neuronal cell differentiation | | | |
| Cript | NM_019907 | Neuronal cell differentiation | | | |
| Ascl1 | NM_022384 | Neuronal cell differentiation | | | |
| PP2A/B | AF180350 | Neuronal cell differentiation | | | |
| Dbnl | NM_031352 | Neuronal cell differentiation | | | |
| p56 | X80349 | Neuronal cell differentiation | | | |
| Rbbp9 | NM_019219 | Neuronal cell differentiation | | | |

| | | | Neuronal cell differentiation | | | | |
|---|---|---|---|---|---|---|---|
| Rps26 | NM_013224 | | Neuronal cell differentiation | | | | |
| Myo5b | NM_017083 | | Neuronal cell differentiation | | | | |
| Tgm2 | NM_019386 | | Neuronal cell differentiation | | | | |
| Rpl32 | NM_013226 | | Neuronal cell differentiation | | | | |
| Gsk3a | NM_017344 | | Neuronal cell differentiation | | | | |
| Cops7a | NM_012003 | | Neuronal cell differentiation | | | | |
| Uchl1 | NM_017237 | | Neuronal cell differentiation | | | | |
| Col2a1 | NM_012929 | | Neuronal cell differentiation | | | | |
| Tsc2 | NM_012680 | | Neuronal cell differentiation | | | | |
| Rmt7 | AF465614 | | Neuronal cell differentiation | | | | |
| Napa | NM_080585 | | Neuronal cell differentiation | | | | |
| Homer2 | NM_053309 | | Neuronal cell differentiation | | | | |
| Plcb3 | M99567 | | Neuronal cell differentiation | | | | |
| PAIHC3 | NM_017351 | | Neuronal cell differentiation | | | | |
| TRPRS | XM_234566 | | Neuronal cell differentiation | | | | |
| Cdc37 | NM_053743 | | Neuronal cell differentiation | | | | |
| Sdc4 | NM_012649 | | Neuronal cell differentiation | | | | |
| ntt4r | L06434 | | Neuronal cell differentiation | | | | |
| Csrp3 | NM_057144 | | Neuronal cell differentiation | | | | |
| Phyh | NM_053674 | | Neuronal cell differentiation | | | | |
| Aloxe3 | XM_213336 | | Neuronal cell differentiation | | | | |
| Cst3 | X16957 | | Neuronal cell differentiation | | | | |
| Atp2a2 | NM_017290 | | Neuronal cell differentiation | | | | |
| GTP | K03248 | | Neuronal cell differentiation | | | | |
| Adcyap1 | NM_016989 | | Neuronal cell differentiation | | | | |
| Mtmr2 | XM_235822 | | Neuronal cell differentiation | | | | |
| Gnb1 | NM_030987 | | Neuronal cell differentiation | | | | |
| Sap18 | XM_214170 | | Neuronal cell differentiation | | | | |
| Slc2a1 | NM_138827 | | Neuronal cell differentiation | | | | |
| Gda | NM_031776 | | Neuronal cell differentiation | | | | |
| Fth1 | NM_012848 | | Neuronal cell differentiation | | | | |
| Casp1 | NM_012762 | | Neuronal cell differentiation | | | | |
| Rab4a | NM_009003 | | Neuronal cell differentiation | | | | |
| Myr5 | NM_012984 | | Neuronal cell differentiation | | | | |
| Cd59 | NM_012925 | | Neuronal cell differentiation | | | | |
| Apeg1 | NM_012905 | | Neuronal cell differentiation | | | | |
| TCEB2 | NM_031129 | | Neuronal cell differentiation | | | | |

| | | | | | | |
|---|---|---|---|---|---|---|
| Fzd1 | NM_021266 | | Neuronal cell differentiation | | | |
| AREG | NM_001657 | Growth factors and cytokines | | | 0,08 | |
| BMP2 | NM_001200 | Growth factors and cytokines | | | 0,31 | 5,48 |
| CCL2 | NM_002982 | Growth factors and cytokines | chemokine | | | |
| CSF1 | M37435 | Growth factors and cytokines | | 0,61 | | 9,51 |
| CTGF | U14750 | Growth factors and cytokines | | | | |
| FGF2 | NM_002006 | Growth factors and cytokines | | | | |
| FGF8 | U36223 | Growth factors and cytokines | | 0,43 | | |
| GMCSF | M11220 | Growth factors and cytokines | | | | |
| IFNG | X13274 | Growth factors and cytokines | | | | |
| IGF1 | X57025 | Growth factors and cytokines | | | | |
| IGFBP2 | M35410 | Growth factors and cytokines | | 1,47 | 1,57 | |
| IGFBP3 | X64875 | Growth factors and cytokines | | | | |
| IGFBP5 | M65062 | Growth factors and cytokines | | | | |
| IL2 | U25676 | Growth factors and cytokines | | | | |
| IL3 | M20137 | Growth factors and cytokines | | | 0,63 | |
| IL8 | NM_000584 | Growth factors and cytokines | | | | 6,83 |
| IL10 | NM_000572 | Growth factors and cytokines | | | | |
| IL11 | NM_000641 | Growth factors and cytokines | | 2,04 | 1,93 | |
| IL12 | M65291 | Growth factors and cytokines | | | | |
| IL15 | NM_000585 | Growth factors and cytokines | | | | 3,72 |
| IL1A | NM000575 | Growth factors and cytokines | | | | |
| IL1B | M15330 | Growth factors and cytokines | | | 0,24 | 2,71 |
| IL4 | NM_000589 | Growth factors and cytokines | | | | |
| IL6 | NM000600 | Growth factors and cytokines | | | | 1,81 |
| MEK1 | L11284 | Growth factors and cytokines | | | 0,50 | |
| MEK2 | NM_030662 | Growth factors and cytokines | | 1,7 | | |
| PDGFA | NM_002607 | Growth factors and cytokines | | | | |
| PRSS11 | NM_002775 | Growth factors and cytokines | | | | |
| TGFA | NM_003236 | Growth factors and cytokines | | | 0,26 | |
| TGFB1 | NM_000660 | Growth factors and cytokines | | | | |
| TNFA | NM_000594 | Growth factors and cytokines | | | | |
| TNFB | NM_000595 | Growth factors and cytokines | | | 2,19 | |
| VEGF | AF022375 | Growth factors and cytokines | | | | 1,75 |
| VEGFB | U43368 | Growth factors and cytokines | | | | |
| VEGFC | NM_005429 | Growth factors and cytokines | | | 0,14 | |
| VEGFD | NM_004469 | Growth factors and cytokines | | | | |
| BIN1 | NM_004305 | | Tumor suppressor | | | |

| Gene | Accession | Function | | | |
|------|-----------|----------|---|---|---|
| BRCA2 | NM_000059 | Tumor suppressor | | 0,31 | |
| EWSR1 | NM_005243 | Oncogenesis | | 1,51 | |
| FES | X52192 | Oncogenesis | 0,41 | 2 | |
| FOS | NM_005252 | Oncogenesis | 0,36 | 0,25 | |
| ING1 | NM005537 | Tumor suppressor | | | |
| L6 | M90657 | Tumor antigen | | 0,28 | |
| MAP17 | U21049 | Oncogenesis | | | |
| MYC | NM_012333 | Oncogenesis | | 0,53 | |
| NF1 | NM_000267 | Tumor supressor | | 0,50 | |
| RAF1 | X03484 | Oncogenesis | 0,61 | | 2,97 |
| RET | NM_000323 | Oncogenesis | | | |
| RRAS | NM_006270 | Oncogenesis | | 0,42 | |
| S100A11 | D38583 | Oncogenesis | | | |
| SHC | U73377 | Oncogenesis | | 0,49 | |
| SNCG | NM_003087 | Oncogenesis | | 1,92 | |
| TGFBR2 | D50683 | Tumor supressor | | 0,31 | |
| ADRA1a | NM_017191 | Cell signaling/receptor | | | |
| ADRA1b | NM_016991 | Cell signaling/receptor | | | |
| ADRA1d | NM_024483 | Cell signaling/receptor | | | |
| ADRA2c | NM_138506 | Cell signaling/receptor | | | |
| ADRB2 | NM_012492 | Cell signaling/receptor | | | |
| Calcyon | NM_138915 | Cell signaling/receptor | | | |
| CCR2 | NM_000647 | Cell signaling/receptor | | | |
| CHRNA2 | NM_133420 | Cell signaling/receptor | | | |
| CHRNA3 | NM_052805 | Cell signaling/receptor | | | |
| CHRNA4 | NM_024354 | Cell signaling/receptor | | | |
| CHRNA5 | NM_017078 | Cell signaling/receptor | | | |
| CHRNA7 | NM_012832 | Cell signaling/receptor | | | |
| CHRNB1 | NM_012528 | Cell signaling/receptor | | | |
| CHRNB2 | NM_019297 | Cell signaling/receptor | | | |
| CHRNB3 | NM_133597 | Cell signaling/receptor | | | |
| CHRNB4 | NM_052806 | Cell signaling/receptor | | | |
| CHRN D | NM_019298 | Cell signaling/receptor | | | |
| CHRN E | NM_017194 | Cell signaling/receptor | | | |
| CHRM1 | NM_080773 | Cell signaling/receptor | | | |
| CHRM2 | NM_031016 | Cell signaling/receptor | | | |
| CHRM3 | NM_012527 | Cell signaling/receptor | | | |
| CHRM4 | M16409 | Cell signaling/receptor | | | |
| CSF1R | NM_005211 | Cell signaling/receptor | | | |
| Drd1a | NM_012546 | Cell signaling/receptor | | | |
| Drd2 | X56065 | Cell signaling/receptor | | | |
| Drd3 | X53944 | Cell signaling/receptor | | | |
| DRIP78 | NM_053690 | Cell signaling/receptor | | | |
| DTR | M60278 | Cell signaling/receptor | | 0,36 | |
| EGFR | NM_005228 | Cell signaling/receptor | 0,42 | | |
| EAR1 | NM_021724 | Cell signaling/receptor | | | |
| ESR2 | X99101 | Cell signaling/receptor | | | |
| FGFR | NM_000604 | Cell signaling/receptor | 0,44 | | |
| Gpr88 | NM_031696 | Cell signaling/receptor | | | |
| Hrh1 | NM_017018 | Cell signaling/receptor | | | |
| Hrh2 | S57565 | Cell signaling/receptor | | | |
| Hrh3 | AB015646 | Cell signaling/receptor | | | |
| Hrh4 | AF358860 | Cell signaling/receptor | | | |
| Htr1a | NM_012585 | Cell signaling/receptor | | | |
| Htr1b | X62944 | Cell signaling/receptor | | | |
| Htr1d | NM_012852 | Cell signaling/receptor | | | |
| Htr1f | NM_021857 | Cell signaling/receptor | | | |
| Htr2a | M64867 | Cell signaling/receptor | | | |
| Htr2b | NM_017250 | Cell signaling/receptor | | | |
| Htr2c | NM_012765 | Cell signaling/receptor | | | |
| Htr3a | NM_024394 | Cell signaling/receptor | | | |
| Htr3b | NM_022189 | Cell signaling/receptor | | | |
| Htr4 | NM_012853 | Cell signaling/receptor | | | |
| Htr5a | NM_013148 | Cell signaling/receptor | | | |
| Htr5b | L10073 | Cell signaling/receptor | | | |
| Htr6 | NM_024365 | Cell signaling/receptor | | | |
| Htr7 | NM_022938 | Cell signaling/receptor | | | |
| IGF1R | NM_000875 | Cell signaling/receptor | | | |
| IL11RA | U32324 | Cell signaling/receptor | 2,61 | | |
| MSR1 | NM_002445 | Cell signaling/receptor | | | |
| NCK1 | NM_006153 | Cell signaling/receptor | | | |
| NCOR1 | NM_006311 | Cell signaling/receptor | | | |
| NCOR2 | NM_006312 | Cell signaling/receptor | 1,62 | | 0,61 |
| NGFR | M14764 | Cell signaling/receptor | | 0,53 | |
| PGR | NM_000926 | Cell signaling/receptor | | | |

| Gene | Accession | Category | Val1 | Val2 | Val3 |
|---|---|---|---|---|---|
| PLAUR | NM_002659 | Cell signaling/receptor | | | |
| ROR1 | U04897 | Cell signaling/receptor | | | |
| TBXA2R | D38081 | Cell signaling/receptor | 1,79 | 2,04 | |
| TNFRSF1A | X55313 | Cell signaling/receptor | | | |
| TNFRSF1B | NM_001066 | Cell signaling/receptor | | 2,01 | |
| VEGFR1 | NM_002019 | Cell signaling/receptor | | | |
| VEGFR2 | NM_002253 | Cell signaling/receptor | | | 0,45 |
| VEGFR3 | NM_002020 | Cell signaling/receptor | | | |
| CENPA | U14518 | Chromosomal processing | | 0,08 | |
| CENPF | U30872 | Chromosomal processing | | 0,26 | |
| H2B/S | NM_080593 | Chromosomal processing | | | |
| H3FF | NM_003533 | Chromosomal processing | | | |
| H4FM | NM_003495 | Chromosomal processing | 0,58 | | |
| KNSL5 | NM_004856 | Chromosomal processing | | 0,09 | |
| KNSL6 | NM_006845 | Chromosomal processing | 2,96 | 0,28 | |
| EDN1 | NM_001955 | Ciculation | | | |
| F3 | NM_001993 | Ciculation | | | |
| THBD | NM_000361 | Ciculation | | | |
| PAI1 | M14083 | Ciculation | | 1,55 | 1,88 |
| PAI2 | J02685 | Ciculation | | 0,23 | 2,46 |
| PLAU | NM_002658 | Ciculation | | 0,13 | 2,51 |
| TPA | NM_000930 | Ciculation | | 0,41 | |
| VWF | NM_000552 | Ciculation | | | |
| AOP2 | NM_004905 | **Stress response** | | 2,48 | 2,28 |
| HMOX | NM_002133 | **Stress response** | | | |
| HSP27 | AB020027 | **Stress response** | | 2,92 | |
| HSP40 | D49547 | **Stress response** | | 1,56 | |
| HSP70 | AB023420 | **Stress response** | | | |
| HSP70B | NM_002155 | **Stress response** | | | |
| HSP90-alpha | X15183 | **Stress response** | | | |
| HSP90-beta | NM_007355 | **Stress response** | | | |
| JNK1 | L26318 | **Stress response** | | | |
| JNKK1 | NM_003010 | **Stress response** | | | |
| JNK2 | U09759 | **Stress response** | | 0,59 | |
| JNK3 | NM_002753 | **Stress response** | | | |
| MT2A | V00594 | **Stress response** | | 0,53 | |
| SRI | NM_003130 | **Stress response** | | 0,49 | |
| ADPRT | J03473 | DNA repair/synthesis | | 1,8 | |
| CROC1A | NM_003349 | DNA repair/synthesis | 0,57 | | |
| FHIT | NM_002012 | DNA repair/synthesis | | | |
| GADD153 | S40706 | DNA repair/synthesis | | | |
| PLK | U01038 | DNA repair/synthesis | | 0,56 | |
| POLA2 | NM_002689 | DNA repair/synthesis | 2,05 | 1,8 | 0,61 |
| RRM1 | NM_001033 | DNA repair/synthesis | | | |
| SLK | NM_014720 | DNA repair/synthesis | | | |
| TERC | U86046 | DNA repair/synthesis | | 1,92 | |
| TERT | AF018167 | DNA repair/synthesis | 1,79 | 1,75 | |
| TOP2 | NM_001067 | DNA repair/synthesis | | 0,12 | |
| TRF1 | U40705 | DNA repair/synthesis | | | |
| TYMS | NM_001071 | DNA repair/synthesis | | 0,27 | |
| ANX1 | NM_000700 | lipid metabolism | | 1,59 | |
| APOB | NM_000384 | lipid metabolism | | | |
| APOE | M12529 | lipid metabolism | | 2,48 | |
| APOJ | J02908 | lipid metabolism | | 1,96 | |
| COX1 | NM_000962 | lipid metabolism | | 0,53 | |
| COX2 | NM_000963 | lipid metabolism | | | 3,41 |
| PLA2G4A | NM_024420 | lipid metabolism | | | |
| PLA2G2A | NM_000300 | lipid metabolism | | | |
| PLA2G6 | NM_003560 | lipid metabolism | | | |
| PPARA | NM_005036 | lipid metabolism | | | |
| PPARG | NM_005037 | lipid metabolism | | | |
| CKB | M16364 | Intermediate metabolism | | 1,91 | |
| ETFB | NM_001985 | Intermediate metabolism | | | |
| G6PD | NM_000402 | Intermediate metabolism | | 2,35 | |
| GAA | NM_000152 | Intermediate metabolism | | 2,09 | |
| GLB1 | M34423 | Intermediate metabolism | | 0,63 | |
| MVK | M88468 | Intermediate metabolism | | 2 | |
| eNOS | NM_000603 | Intermediate metabolism | | | |
| iNOS | NM_000625 | Intermediate metabolism | | | |
| ODC | NM_002539 | Intermediate metabolism | | 0,17 | |
| PKM2 | M26252 | Intermediate metabolism | | 0,66 | |
| BSG | NM_001728 | Extracellular matrix | | | |
| COL1A1 | NM_000088 | Extracellular matrix | | 2,31 | |
| COL3A1 | NM_000090 | Extracellular matrix | | | |
| COL6A2 | NM_001849 | Extracellular matrix | 0,65 | 1,85 | |

| Gene | Accession | Function | | | | |
|---|---|---|---|---|---|---|
| COL15A1 | NM_001855 | Extracellular matrix | | | | |
| DPT | XM_001897 | Extracellular matrix | | 0,56 | | |
| ELN | NM_000501 | Extracellular matrix | | 2,54 | | |
| FN1 | X02761 | Extracellular matrix | | 0,62 | | |
| FMOD | NM_002023 | Extracellular matrix | | | | |
| MMP1 | NM_002421 | Extracellular matrix | | | | |
| MMP9 | NM_004994 | Extracellular matrix | 2,11 | | | 0,59 |
| MMP10 | NM_002425 | Extracellular matrix | | 0,24 | | |
| MMP11 | NM_005940 | Extracellular matrix | 2,12 | | | |
| MMP12 | NM_002426 | Extracellular matrix | | | | |
| MMP13 | NM_002427 | Extracellular matrix | | | | |
| MMP14 | NM_004995 | Extracellular matrix | | 0,36 | | |
| MMP15 | NM_002428 | Extracellular matrix | | 0,63 | 1,57 | |
| MMP2 | NM_004530 | Extracellular matrix | | 0,53 | | |
| MMP3 | NM_002422 | Extracellular matrix | | 0,22 | | |
| MMP7 | NM_002423 | Extracellular matrix | | 0,34 | 0,62 | |
| OPN | NM_000582 | Extracellular matrix | | | | |
| TIMP1 | NM_003254 | Extracellular matrix | | 1,83 | | |
| TIMP2 | NM_003255 | Extracellular matrix | | | | |
| CDC42 | NM_001791 | Cell structure | | | | |
| EMS1 | NM_005231 | Cell structure | | | | |
| GSN | X04412 | Cell structure | | | | |
| MP1 | AF061243 | Cell structure | | 0,46 | | |
| ON | NM_003118 | Cell structure | | 0,29 | | |
| PAK | NM_002576 | Cell structure | | 0,45 | | |
| SLP2 | AF282596 | Cell structure | | 0,48 | | |
| SM22 | M95787 | Cell structure | | 1,92 | | |
| TB10 | NM_021103 | Cell structure | | | | |
| TGM1 | NM_000359 | Cell structure | | 2,22 | | |
| ADAM1 | XM_090479 | Protein metabolism | | | | |
| BAT1 | Z37166 | Protein metabolism | | | | |
| CANX | NM_001746 | Protein metabolism | | 0,62 | | |
| CTSB | NM_001904 | Protein metabolism | | | | |
| CTSD | NM_001904 | Protein metabolism | | 1,98 | | |
| CTSH | NM_004390 | Protein metabolism | | 1,93 | | |
| CTSL | NM_001912 | Protein metabolism | | | | |
| CTSS | M90696 | Protein metabolism | | | | |
| CTSZ | AF136273 | Protein metabolism | | 0,55 | | |
| EF1A | AY043301 | Protein metabolism | | | | |
| EIF-4A | NM_001416 | Protein metabolism | | 0,57 | | |
| EIF-4E | NM_001968 | Protein metabolism | | | | |
| EIF3S6 | NM_001568 | Protein metabolism | | | | |
| RPL3 | NM_000967 | Protein metabolism | | | | |
| RPS10 | NM_001014 | Protein metabolism | | | | |
| PSMA1 | NM_002786 | Proteasome | | | | |
| PSMA2 | NM_002787 | Proteasome | | 0,53 | | |
| PSMA3 | NM_002788 | Proteasome | | | | |
| PSMA4 | NM_002789 | Proteasome | | | | |
| PSMA5 | NM_002790 | Proteasome | | | | |
| PSMA6 | NM_002791 | Proteasome | | | | |
| PSMA7 | NM_002792 | Proteasome | | | | |
| PSMB1 | NM_002793 | Proteasome | | | | |
| PSMB2 | NM_002794 | Proteasome | | | | |
| PSMB3 | NM_002795 | Proteasome | | | | |
| PSMB4 | NM_002796 | Proteasome | | | | |
| PSMB5 | NM_002797 | Proteasome | | | | |
| PSMB6 | NM_002798 | Proteasome | | | | |
| PSMB7 | NM_002799 | Proteasome | | | | |
| PSMB8 | NM_004159 | Proteasome | | | | |
| PSMB9 | NM_002800 | Proteasome | | | | |
| PSMB10 | NM_002801 | Proteasome | | | | |
| PSMC1 | NM_002802 | Proteasome | | | | |
| PSMC2 | NM_002803 | Proteasome | | | | |
| PSMC3 | NM_002804 | Proteasome | | | | |
| PSMC4 | NM_006503 | Proteasome | | | | |
| PSMC5 | NM_002805 | Proteasome | | | | |
| PSMC6 | NM_002806 | Proteasome | | 0,60 | | |
| PSMD1 | NM_002807 | Proteasome | | 0,49 | | |
| PSMD2 | NM_002808 | Proteasome | | | | |
| PSMD3 | NM_002809 | Proteasome | | | | |
| PSMD4 | NM_002810 | Proteasome | | | | |
| PSMD5 | NM_005047 | Proteasome | | | | |
| PSMD6 | NM_014814 | Proteasome | | | | |
| PSMD7 | NM_002811 | Proteasome | | | | |

| Gene | Accession | Function | Category | C1 | C2 | C3 | C4 |
|---|---|---|---|---|---|---|---|
| PSMD8 | NM_002812 | Proteasome | | | | | |
| PSMD9 | NM_002813 | Proteasome | | | | | |
| PSMD10 | NM_002814 | Proteasome | | | | | |
| PSMD11 | NM_002815 | Proteasome | | | | | 2,61 |
| PSMD12 | NM_002816 | Proteasome | | | | | |
| PSMD13 | NM_002817 | Proteasome | | | | | |
| PSMD14 | NM_005805 | Proteasome | | | | | |
| PSME1 | NM_006263 | Proteasome | | | | | |
| PSME2 | NM_002818 | Proteasome | | | | | |
| PSME3 | NM_005789 | Proteasome | | | | | |
| UBE2C | NM_007019 | Proteasome | | | | 0,14 | |
| SOD2 | NM_000636 | Oxidative metabolism | | | | 0,46 | |
| GSTT1 | NM_000853 | Oxidative metabolism | | 0,56 | | 1,58 | |
| MSRA | AF183420 | Oxidative metabolism | | | | 0,37 | |
| GPX | M21304 | Oxidative metabolism | | | | 0,31 | |
| GSTP1 | NM_000852 | Oxidative metabolism | | 0,54 | | | 11,25 |
| DP1 | NM_007111 | Transcription | | 0,51 | | | |
| DP2 | NM_006286 | Transcription | | | | | |
| E2F1 | NM_005225 | Transcription | | | | 1,73 | |
| E2F2 | NM_004091 | Transcription | | 0,32 | | | |
| E2F3 | NM_001949 | Transcription | | | | | |
| E2F4 | NM_001950 | Transcription | | | | | |
| E2F5 | U31556 | Transcription | | | | | |
| EGR1 | NM_001964 | Transcription | | 0,42 | | | |
| EGR2 | NM_000399 | Transcription | | | | | |
| EGR3 | NM_004430 | Transcription | | | | | |
| EPC1 | AF286904 | Transcription | | | | | |
| JUND | NM_005354 | Transcription | | | | | |
| MAX | NM_002382 | Transcription | | | | 0,51 | |
| MYBL2 | X13293 | Transcription | | 0,43 | | 0,51 | |
| STAT5 | L41142 | Transcription | | | | 0,63 | |
| TFAP2A | M36711 | Transcription | | | | | |
| TFAP2B | X95694 | Transcription | | 0,51 | | 1,55 | |
| TFAP2C | NM_003222 | Transcription | | | | | |
| ACTB | NM_001101 | Housekeeping gene | Cell structure | | | | |
| GAPD | NM002046 | Housekeeping gene | Intermediate metabolism | | | | |
| L10a | NM_031065 | Housekeeping gene | Tumor suppressor | | | | |
| RPS13 | X53378 | Housekeeping gene | Protein metabolism | | | | |
| RPL31 | NM_022506 | Housekeeping gene | Protein metabolism | | | | |
| Rps2 | NM_031838 | Housekeeping gene | Protein metabolism | | | | |
| S9 | NM_001013 | Housekeeping gene | Protein metabolism | | | | |
| SDS | NM_006843 | Housekeeping gene | Intermediate metabolism | | | | |
| SOD3 | NM_012880 | Housekeeping gene | Oxidative metabolism | | | | |
| TFR | NM_003234 | Housekeeping gene | Protein metabolism | | | | |
| Tubu | NM_006082 | Housekeeping gene | Cell structure | | | | |
| 23kd | X56932 | Housekeeping gene | Protein metabolism | | | | |
| Aldo | NM_000034 | Housekeeping gene | Intermediate metabolism | | | | 2,54 |
| cyc | AF042385 | Housekeeping gene | Protein metabolism | | | | |
| HEXO | M75126 | Housekeeping gene | Intermediate metabolism | | | | |
| HPRT | NM_000194 | Housekeeping gene | Intermediate metabolism | | | | |
| MDH | NM_005917 | Housekeeping gene | Intermediate metabolism | | | | |
| PLA2 | M86400 | Housekeeping gene | lipid metabolism | | | | |

**Claims**

1. A method for a quantitative determination of the overall status of cell(s) comprising the steps of:

   providing an array containing on predetermined locations thereof a maximum of 2999 nucleic acids or proteins belonging to or being representative for at least 9 of the following vital cellular functions: apoptosis, cell adhesion, cell cycle, growth factors and cytokines, cell signaling, chromosomal processing, DNA repair/synthesis, intermediate metabolism, extracellular matrix, cell structure, protein metabolism, oxidative metabolism, transcription and house keeping genes, said functions being represented by at least 4 genes or proteins,
   contacting a sample component derived from a particular cell of interest with the array,
   detecting whether and where binding of the sample components to any of the predetermined locations on the array occurred, and
   optionally quantifying the intensity of the spots detected,

wherein the pattern and/or the intensity of the binding events is indicative for the cellular status.

2. The method according to claim 1, wherein the step of detecting and optionally quantifying the pattern of hybridization on the array is performed on a single capture nucleotide species.

3. The method according to any of the claims 1 or 2, wherein the values for the quantification on the arrays are taken as the average of three experimental data.

4. The method according to any of claim 1, wherein the number of nucleic acids or proteins to be detected is maximum of 999.

5. The method according to claim 1, wherein at least one nucleic acid for each of the 9 vital cellular functions is expressed differentially, said method further comprising the step of comparing the transcriptome of cells or tissues in a given biological condition with at least one reference or control condition.

6. The method according to claim 5, wherein said control condition differs from the sample condition in respect of the cellular microenvironment, in respect of exposure to a physiological stimulus, hormones, growth factors, cytokines, chemokines, inflammatory agents, toxins, metabolites, pH, chemical and/or pharmaceutical agents, hypoxia, anoxia, ischemia, imbalance of any plasma-borne nutrient, osmotic stress, temperature, mechanical stress, irradiation, cell-extracellular matrix interactions, cell-cell interactions, accumulations of foreign or pathological extracellular components, intracellular and extracellular pathogens, or a genetic perturbation.

7. The method according to claim 6, wherein the control condition differs in that the sample cells have been exposed to a physiological stimulus, preferably a mechanical, temperature, chemical, toxic or pharmaceutical stress.

8. The method according to claims 1 and 2, wherein the array provides at least 20 different capture probes for at least one nucleic acid for each of the 9 vital cellular functions.

9. The method according to any of the claims 1 to 8, wherein the vital functions are represented by at least 2 genes of the table 1.

10. The method according to claim 1 to 9, wherein at least one gene for each of the 9 vital functions is a gene which effects a regulatory activity in the function.

11. The method according to claim 1, wherein said cell is selected from the group consisting of cardiomyocytes, endothelial cells, sensory neurons, motor neurons, CNS neurons, astrocytes, glial cells, Schwann cells, mast cells, eosinophils, smooth muscle cells, skeletal muscle cells, pericytes, lymphocytes, tumor cells, monocytes, macrophages, foamy macrophages, dentritic cells, granulocytes, melanocytes, keratinocytes, synovial cells/synovial fibroblasts and epithelial cells.

12. A method according to any of claims 1 to 11, wherein the array comprises polynucleotide sequences or peptidic sequences.

13. The method according to any of the preceding claims, wherein the two arrays for the biological and the control experimental conditions are analyzed on the same support.

14. A method for the quantitative determination of a cellular status of cell(s) comprising the steps of,
   providing an array, containing on predetermined locations thereof nucleic acids or proteins belonging to or representative for at least 5 of the following vital cellular functions: apoptosis, cell adhesion, cell cycle, growth factors and cytokines, cell signaling, chromosomal processing, DNA repair/synthesis, intermediate metabolism, extracellular matrix, cell structure, protein metabolism, oxidative metabolism, transcription and house keeping genes and at least one nucleic acid or protein, belonging to or representative for at least one of the following specific functions: cell differentiation, oncogene/tumor suppressor, stress response, lipid metabolism, proteasome, circulation, wherein the array comprises at least 20 different spot compositions and a maximum of 2999 different spots,
   contacting a sample component derived from the cell(s) of interest with the array,
   detecting and/or quantifying the intensity of spots present on an array specific of the expression of,
   said method comprising the steps of comparing the transcriptome of cells or tissues in the given biological condition with at least one reference or control condition.

15. The method according to claim 14, wherein the two dimensional array provides capture probes for at least one gene of each of the 5 vital functions together with at least 5 genes of a specific function.

16. The method according to any of the claims 14 to 15, wherein the vital functions are represented by at least 2 genes of the table 1.

17. The method according to claim 14 to 16, wherein at least one gene of each of the 5 vital functions is a gene which effects a regulatory activity in the function.

18. The method according to claim 14, wherein said cell is an eucaryotic cell selected from the group consisting of cardiomyocytes, endothelial cells, sensory neurons, motor neurons, CNS neurons, astrocytes, glial cells, Schwann cells, mast cells, eosinophils, smooth muscle cells, skeletal muscle cells, pericytes, lymphocytes, tumor cells, monocytes, macrophages, foamy macrophages, dentritic cells, granulocytes, melanocytes, keratinocytes, synovial cells/synovial fibroblasts and epithelial cells.

19. The method according to claim 14, wherein the two dimensional array provides a quantification of nucleic acids or proteins which are essential to obtain an overview of the modifications occurring in a three dimensional cell.

20. The method according to claim 14, wherein said biological and control experimental conditions differ in respect of the cellular microenvironment, or in respect of exposure to a physiological stimulus, hormones, growth factors, cytokines, chemokines, inflammatory agents, toxins, metabolites, pH, pharmaceutical agents, hypoxia, anoxia, ischemia, imbalance of any plasma-borne nutrient, osmotic stress, temperature, mechanical stress, irradiation, cell-extracellular matrix interactions, cell-cell interactions, accumulations of foreign or pathological extracellular components, intracellular and extracellular pathogens, or a genetic perturbation.

21. The method according to claim 20, wherein the biological experimental conditions and control experimental conditions differ in that under the biological experimental conditions, the cells are exposed to a physiological stimulus, preferably a mechanical, temperature, chemical, toxic or pharmaceutical stress.

22. The method according to any of claims 14 to 21, wherein the biological sample to be tested and the control are analyzed on arrays bearing the same capture molecules for the said analyzed genes.

23. The method according to any one of claims 14 to 22, wherein the biological sample to be tested and the control are analyzed on arrays present on the same support.

24. The method according to any of the claims 14 to 23, wherein the variations in the gene expression in the test compared to the control sample is obtained by examination of the ratios of the values obtained on the two arrays.

25. The method according to any of the claims 14 to 24, wherein the array comprises polynucleotide sequences or peptidic sequences.

26. A method according to any of the claims 14 to 26, wherein the two arrays for the biological and the control experimental conditions are analyzed on the same support.

27. A method according to claim 14, wherein at least one gene of each of 5 vital functions is a gene which encode for regulatory activity in the function.

28. A kit for a quantitative determination of the overall status of cell(s), including an array, containing on predetermined locations thereof a maximum of 2999 nucleic acids or proteins belonging to or representative for at least 5 of the following vital cellular functions: apoptosis, cell adhesion, cell cycle, growth factors and cytokines, cell signaling, chromosomal processing, DNA repair/synthesis, intermediate metabolism, extracellular matrix, cell structure, protein metabolism, oxidative metabolism, transcription and house keeping genes.

29. A kit for a quantitative determination of the overall status of cell(s), including an array, containing on predetermined locations thereof a maximum of 2999 nucleic acids or proteins belonging to or representative for at least 5 of the following vital cellular functions: apoptosis, cell adhesion, cell cycle, growth factors and cytokines, cell signaling, chromosomal processing, DNA repair/synthesis, intermediate metabolism, extracellular matrix, cell structure, protein metabolism, oxidative metabolism, transcription and house keeping genes, and at least one nucleic acid or protein, belonging to or representative for at least one of the following specific functions : cell differentiation, oncogene/tumor suppressor, stress response, lipid metabolism proteasome, circulation, wherein the array comprises at least 20 different spot compositions and a maximum of 2999 different spots.

30. Use of a method according to any of the claims 1 to 27 or a kit according to claim 28 to 29 for the analysis of gene expression changes occurring in particular conditions a cell is subjected.

31. The use according to claim 30, wherein the condition a cell is subjected is selected from stress, ageing, stem cell differentiation, haematopoiesis, neuronal functional status, diabetes, obesity, transformation process such as carcinogenesis, protein turnover or circulatory disorders as atherosclerosis.

# Figure 1
## Presentation of the pattern of the microarray for gene expression analysis of the Generalchip (202 genes) with the appropriated controls

| Row | Col1 | Col2 | Col3 | Gene symbol | Gene name |
|---|---|---|---|---|---|
| 1 | 3 | 12 | 21 | PSMD11 | 26S-proteasome-subunit-p44.5 |
| 1 | 4 | 13 | 22 | TFAP2A | Transcription factor AP2-alpha |
| 1 | 5 | 14 | 23 | ANX1 | Annexin1 |
| 1 | 6 | 15 | 24 | TFAP2C | Transcription factor AP2-gamma |
| 1 | 7 | 16 | 25 | AOP2 | Anti-oxidant-protein2 |
| 1 | 8 | 17 | 26 | TFAP2B | Transcription factor AP2-beta |
| 1 | 9 | 18 | 27 | ADAM1 | A disintegrin and metalloproteinase |
| 2 | 1 | 10 | 19 | APOJ | ApoliproteinJ |
| 2 | 2 | 11 | 20 | BCL2 | B-cell lymphoma2 |
| 2 | 3 | 12 | 21 | BCLX | BCLX |
| 2 | 4 | 13 | 22 | BAD | BCL2-antagonist of cell death |
| 2 | 5 | 14 | 23 | BAX | BCL2-associated X protein |
| 2 | 6 | 15 | 24 | ATM | Ataxia telangiectasia mutated |
| 2 | 8 | 17 | 26 | MYBL2 | b-myb |
| 2 | 9 | 18 | 27 | 23kd | 23KDa Highly basic protein |
| 3 | 1 | 10 | 19 | GLB1 | Beta1-galactosidase |
| 3 | 2 | 11 | 20 | BID | BH3 interacting domain death agonist |
| 3 | 4 | 13 | 22 | BMP2 | Bone morphogenetic protein2 |
| 3 | 5 | 14 | 23 | BIN1 | Bridging integrator 1 |
| 3 | 6 | 15 | 24 | FOS | c-fos |
| 3 | 7 | 16 | 25 | cmyc | c-myc |
| 3 | 8 | 17 | 26 | RAF1 | c-raf-1 |
| 3 | 9 | 18 | 27 | FES | Feline sarcoma oncogene |
| 4 | 2 | 11 | 20 | ACTB | Beta-Actin |
| 4 | 3 | 12 | 21 | CASP2 | Caspase2 |
| 4 | 4 | 13 | 22 | CANX | Calnexin |
| 4 | 5 | 14 | 23 | CDH11 | Cadherine11 |
| 4 | 6 | 15 | 24 | S100A8 | Calprotectin |
| 4 | 7 | 16 | 25 | CASP3 | Caspase3 |
| 4 | 8 | 17 | 26 | CDH1 | Cadherine1/E-cadherine |
| 4 | 9 | 18 | 27 | CDH13 | Cadherine13 |
| 5 | 1 | 10 | 19 | Aldo | Aldolase A, |
| 5 | 2 | 11 | 20 | CASP7 | Caspase7 |
| 5 | 3 | 12 | 21 | CASP9 | Caspase9 |
| 5 | 4 | 13 | 22 | CAV1 | Caveoline1 |
| 5 | 5 | 14 | 23 | CATB | Catenin , beta 1 |
| 5 | 6 | 15 | 24 | CTSB | CathepsinB |
| 5 | 7 | 16 | 25 | CTSD | CathepsinD |
| 5 | 8 | 17 | 26 | CTSL | CathepsinL |
| 5 | 9 | 18 | 27 | CASP8 | Caspase8 |
| 6 | 1 | 10 | 19 | CKB | Creatin-kinase-brain |
| 6 | 2 | 11 | 20 | COX2 | Prostaglandin endoperoxidase synthase 2 |
| 6 | 3 | 12 | 21 | CDC42 | Cell division cycle42 |
| 6 | 4 | 13 | 22 | CDK2 | Cyclin dependent kinase2 |
| 6 | 5 | 14 | 23 | COL6A2 | CollagenVI-alpha2 |
| 6 | 6 | 15 | 24 | SPRR1B | Cornifin |
| 6 | 7 | 16 | 25 | CROC1A | Ubiquitin (E2 variant1) |
| 6 | 8 | 17 | 26 | CDK6 | Cyclin dependent kinase6 |
| 6 | 9 | 18 | 27 | CDK4 | Cyclin dependent kinase4 |
| 7 | 1 | 10 | 19 | cyc | Cyclophilin 33A |
| 7 | 2 | 11 | 20 | CSF1 | Colony stimulating factor 1 |
| 7 | 4 | 13 | 22 | CCNA1 | CyclinA1 |
| 7 | 5 | 14 | 23 | CSF1R | Colony stimulating factor 1 receptor |
| 7 | 6 | 15 | 24 | CCNB1 | CyclinB1 |
| 7 | 8 | 17 | 26 | CCND1 | CyclinD1 |
| 7 | 9 | 18 | 27 | CTGF | Connective tissue growth factor |
| 8 | 1 | 10 | 19 | DHFR | Dihydrofolate reductase |
| 8 | 2 | 11 | 20 | CCNE1 | CyclinE |
| 8 | 3 | 12 | 21 | CCNH | CyclinH |
| 8 | 4 | 13 | 22 | CCND2 | CyclinD2 |
| 8 | 5 | 14 | 23 | CCND3 | CyclinD3 |
| 8 | 6 | 15 | 24 | CCNF | CyclinF |
| 8 | 7 | 16 | 25 | E2F2 | E2F transcription factor2 |
| 8 | 8 | 17 | 26 | DP2 | transcription factor Dp-2 |
| 8 | 9 | 18 | 27 | DP1 | Transcription factor Dp-1 |
| 9 | 1 | 10 | 19 | E2F3 | E2F transcription factor3 |
| 9 | 2 | 11 | 20 | E2F4 | E2F transcription factor4 |
| 9 | 3 | 12 | 21 | EGR1 | Early growth response1 |
| 9 | 4 | 13 | 22 | EIF4 | Eukaryotic translation initiation |
| 9 | 5 | 14 | 23 | ETFB | Electron-transfer-flavoprotein-beta |

| 9 | 6 | 15 | 24 | GAPD | Glyceraldehyde-3-phosphate-dehydrogenase |
|---|---|----|----|------|------------------------------------------|
| 9 | 7 | 16 | 25 | EGR3 | Early growth response3 |
| 9 | 8 | 17 | 26 | EGFR | Epidermal growth factor receptor |
| 10 | 1 | 10 | 19 | VWF | Factor von willebrand |
| 10 | 2 | 11 | 20 | FGFR | Fibroblast growth factor receptor 1 |
| 10 | 3 | 12 | 21 | BSG | Basigin |
| 10 | 4 | 13 | 22 | FGF2 | Fibroblast growth factor 2 |
| 10 | 5 | 14 | 23 | FHIT | Fragile histidine triad gene |
| 10 | 6 | 15 | 24 | EMS1 | EMS1 |
| 10 | 7 | 16 | 25 | FGF8 | Fibroblast growth factor 8 |
| 10 | 8 | 17 | 26 | FN1 | fibronectin |
| 10 | 9 | 18 | 27 | ESR2 | Estrogen receptor beta |
| 11 | 1 | 10 | 19 | GPX | Glutathione peroxidase |
| 11 | 2 | 11 | 20 | HEXO | Hexokinase 1 |
| 11 | 4 | 13 | 22 | G6PD | Glucose-6-phosphate-dehydrogenase |
| 11 | 5 | 14 | 23 | GSTP1 | Glutathione S-transferase pi |
| 11 | 6 | 15 | 24 | GRB2 | Growth factor receptor-bound protein2 |
| 11 | 7 | 16 | 25 | HMOX | Heme-oxygenase |
| 11 | 8 | 17 | 26 | GADD153 | DNA damage inducible transcript3 |
| 11 | 9 | 18 | 27 | GSN | Gelsoline |
| 12 | 1 | 10 | 19 | HSP27 | Heat shock 27kD protein1 |
| 12 | 2 | 11 | 20 | HSP70 | Heat shock 70kD protein1 |
| 12 | 3 | 12 | 21 | HSP40 | Heat shock 40kD protein1 |
| 12 | 4 | 13 | 22 | H4FM | Histone4 member M consensus |
| 12 | 5 | 14 | 23 | H3FF | Histone3 member F consensus |
| 12 | 6 | 15 | 24 | HSP90-b | Heat shock 90kD protein 1, beta |
| 12 | 8 | 17 | 26 | H2B/S | histone2b member B/S consensus |
| 12 | 9 | 18 | 27 | HSP90-a | Heat shock 90kD protein1 alpha |
| 13 | 2 | 11 | 20 | ICAM-1 | Intracellular adhesion molecule1 |
| 13 | 3 | 12 | 21 | IGFBP2 | Insulin growth factor binding protein2 |
| 13 | 4 | 13 | 22 | IGFBP5 | Insulin growth factor binding protein5 |
| 13 | 5 | 14 | 23 | HPRT | Hypoxanthine phosphoribosyltransferase 1 |
| 13 | 6 | 15 | 24 | IGF1R | Insulin like growth factor1 receptor |
| 13 | 7 | 16 | 25 | IL1A | Interleukin1 alpha |
| 13 | 8 | 17 | 26 | IGF1 | Insulin like growth factor1 |
| 13 | 9 | 18 | 27 | IGFBP3 | Insulin growth factor binding protein3 |
| 14 | 1 | 10 | 19 | IL1B | Interleukin1 beta |
| 14 | 2 | 11 | 20 | IL10 | Interleukin 10 |
| 14 | 3 | 12 | 21 | IL8 | Interleukin 8 |
| 14 | 4 | 13 | 22 | IL15 | Interleukin 15 |
| 14 | 5 | 14 | 23 | IL4 | Interleukin 4 |
| 14 | 6 | 15 | 24 | IL11 | Interleukin 11 |
| 14 | 7 | 16 | 25 | MDH | Malate dehydrogenase 1 |
| 14 | 8 | 17 | 26 | IL6 | Interleukin 6 |
| 14 | 9 | 18 | 27 | IL11RA | Interleukin 11-receptor-alpha |
| 15 | 1 | 10 | 19 | JNK1 | Mitogen activated protein kinase8 |
| 15 | 2 | 11 | 20 | ITGA6 | Integrin alpha6 |
| 15 | 3 | 12 | 21 | ITGA5 | Integrin alpha5 |
| 15 | 4 | 13 | 22 | ITGB1 | Integrin beta1 |
| 15 | 5 | 14 | 23 | Ki-67 | Ki-67 |
| 15 | 6 | 15 | 24 | JNK3 | Mitogen-activated protein kinase 10 |
| 15 | 7 | 16 | 25 | JUND | Jun D proto-oncogene |
| 15 | 8 | 17 | 26 | ING1 | Inhibitor of growth family, member 1 |
| 15 | 9 | 18 | 27 | JNK2 | Mitogen activated protein kinase9 |
| 16 | 1 | 10 | 19 | MSRA | Methionine-sulfoxide-reductase A/peptide |
| 16 | 2 | 11 | 20 | MEK1 | Mitogen activated protein kinase kinase1 |
| 16 | 4 | 13 | 22 | PLA2 | Phospholipase A2 |
| 16 | 5 | 14 | 23 | MAX | MAX protein |
| 16 | 6 | 15 | 24 | MDM2 | MDM2 |
| 16 | 8 | 17 | 26 | MEK2 | Mitogen activated protein kinase kinase2 |
| 16 | 9 | 18 | 27 | CENPF | Mitosin |
| 17 | 1 | 10 | 19 | MMP1 | Matrix metalloproteinase 1 |
| 17 | 2 | 11 | 20 | MMP3 | Matrix metalloproteinase 3 |
| 17 | 3 | 12 | 21 | MMP7 | Matrix metalloproteinase 7 |
| 17 | 4 | 13 | 22 | MMP12 | Matrix metalloproteinase 12 |
| 17 | 5 | 14 | 23 | MMP2 | Matrix metalloproteinase 2 |
| 17 | 6 | 15 | 24 | KNSL5 | Mitotic-kinesin-like-protein1 |
| 17 | 7 | 16 | 25 | MMP9 | Matrix metalloproteinase 9 |
| 17 | 8 | 17 | 26 | MMP11 | Matrix metalloproteinase 11 |
| 17 | 9 | 18 | 27 | KNSL6 | Mitotic-centromere-associated-kinesin |
| 18 | 1 | 10 | 19 | S9 | Ribosomal Proteine S9 |
| 18 | 2 | 11 | 20 | MMP13 | Matrix metalloproteinase 13 |

| 18 | 3 | 12 | 21 | MMP15 | Matrix metalloproteinase 15 |
|---|---|---|---|---|---|
| 18 | 4 | 13 | 22 | ODC | Ornithine decarboxylase1 |
| 18 | 5 | 14 | 23 | MMP14 | Matrix metalloproteinase 14 |
| 18 | 6 | 15 | 24 | NCK1 | NCK adaptor protein1 |
| 18 | 7 | 16 | 25 | NCOR1 | Nuclear receptor co-repressor 1 |
| 18 | 8 | 17 | 26 | NCOR2 | Nuclear receptor co-repressor 2 |
| 19 | 1 | 10 | 19 | p53 | Tumor protein p53 |
| 19 | 2 | 11 | 20 | p16 | Cyclin dependent kinase inhibitor 2A |
| 19 | 3 | 12 | 21 | p57 | Cyclin dependent kinase inhibitor 1C |
| 19 | 4 | 13 | 22 | OPN | Osteopontin |
| 19 | 5 | 14 | 23 | ON | Osteonectin |
| 19 | 6 | 15 | 24 | p35 | Cyclin dependent kinase5 regulatory subunit1 |
| 19 | 7 | 16 | 25 | p27 | Cyclin dependent kinase inhibitor 1B |
| 19 | 8 | 17 | 26 | SDS | Serine Dehydratase |
| 19 | 9 | 18 | 27 | p21 | Cyclin dependent kinase inhibitor 1A |
| 20 | 1 | 10 | 19 | PAI2 | Plasminogen activator inhibitor type2 |
| 20 | 2 | 11 | 20 | ADPRT | Polysynthetase |
| 20 | 3 | 12 | 21 | PAI1 | Plasminogen activator inhibitor type1 |
| 20 | 4 | 13 | 22 | PGR | Progesterone receptor |
| 20 | 5 | 14 | 23 | PAK | P21 activated kinase1 |
| 20 | 6 | 15 | 24 | POLA2 | Polymerase alpha |
| 20 | 8 | 17 | 26 | PCNA | Proliferating cell nuclear antigen |
| 20 | 9 | 18 | 27 | PLK | Polo-like kinase |
| 21 | 1 | 10 | 19 | PKM2 | Pyruvate-kinase-muscle |
| 21 | 2 | 11 | 20 | SM22 | Transgelin |
| 21 | 4 | 13 | 22 | RB1 | Retinoblastome1 |
| 21 | 5 | 14 | 23 | RRM1 | Ribonucleotide-reductase M1 |
| 21 | 6 | 15 | 24 | S100A | S100 calcium binding protein A4 |
| 21 | 7 | 16 | 25 | TFR | Transferrin receptor |
| 21 | 8 | 17 | 26 | SMAD1 | SMAD1 |
| 21 | 9 | 18 | 27 | SHC | SHC transforming protein1 |
| 22 | 2 | 11 | 20 | TERT | Telomerase-reverse transcriptase |
| 22 | 3 | 12 | 21 | TGFBR2 | TGF-beta-R2 |
| 22 | 4 | 13 | 22 | SOD2 | Superoxide dismutase2 |
| 22 | 5 | 14 | 23 | TK1 | Thymidine-kinase |
| 22 | 6 | 15 | 24 | TB10 | Thymosin beta 10 |
| 22 | 7 | 16 | 25 | SMAD4 | SMAD4 |
| 22 | 8 | 17 | 26 | SMAD2 | SMAD2 |
| 22 | 9 | 18 | 27 | SMAD3 | SMAD3 |
| 23 | 1 | 10 | 19 | TBXA2R | Thromboxane-A2-receptor |
| 23 | 2 | 11 | 20 | TNFa | Tumor necrosis factor alpha |
| 23 | 3 | 12 | 21 | TPA | Plasminogen activator tissue |
| 23 | 4 | 13 | 22 | TOP2 | Topoisomerase2-alpha |
| 23 | 5 | 14 | 23 | TYMS | Thymidylate-synthetase |
| 23 | 6 | 15 | 24 | TSP1 | Thrombospondin 1 |
| 23 | 7 | 16 | 25 | TIMP1 | Tissue inhibitor of metalloproteinase1 |
| 23 | 8 | 17 | 26 | TIMP2 | Tissue inhibitor of metalloproteinase2 |
| 23 | 9 | 18 | 27 | TRF1 | Telomeric repeat binding factor1 |
| 24 | 1 | 10 | 19 | VEGF | Vascular endothelial growth factor |
| 24 | 2 | 11 | 20 | PLAU | Urokinase |
| 24 | 4 | 13 | 22 | uPAR | Urokinase-receptor |
| 24 | 5 | 14 | 23 | TSP2 | Thrombospondin 2 |
| 24 | 6 | 15 | 24 | Tubu | Alpha-tubulin |
| 24 | 8 | 17 | 26 | UBE2C | Ubiquitin conjugating enzyme E2C/ubiquitin carrier protein |
| 24 | 9 | 18 | 27 | VEGFB | Vascular endothelial growth factor B |
| 25 | 3 | 12 | 21 | VEGFR1 | Vascular endothelial growth factor receptor1 |
| 25 | 4 | 13 | 22 | VEGFR3 | Vascular endothelial growth factor receptor3 |
| 25 | 5 | 14 | 23 | VEGFR2 | Vascular endothelial growth factor receptor2 |
| 25 | 6 | 15 | 24 | VEGFD | Vascular endothelial growth factor D |
| 25 | 7 | 16 | 25 | VEGFC | Vascular endothelial growth factor C |

Controls list

| Row | Col1 | Col2 | Col3 | Gene name |
|---|---|---|---|---|
| 1 | 1 | 10 | 19 | Positive hybridization control 1 |
| 1 | 2 | 11 | 20 | Negative detection control 1 |
| 2 | 7 | 16 | 25 | Internal Standard 4 - 1 |
| 3 | 3 | 12 | 21 | Internal Standard 1 - 1 |

| 4 | 1 | 10 | 19 | Negative hybridization control 1 |
|---|---|----|----|----------------------------------|
| 7 | 3 | 12 | 21 | Internal Standard 4 - 2 |
| 7 | 7 | 16 | 25 | Internal Standard 1 - 2 |
| 9 | 9 | 18 | 27 | Negative hybridization control 2 |
| 11 | 3 | 12 | 21 | Internal Standard 2 - 1 |
| 12 | 7 | 16 | 25 | Internal Standard 5 - 1 |
| 13 | 1 | 10 | 19 | Negative hybridization control 3 |
| 16 | 3 | 12 | 21 | Internal Standard 5 - 2 |
| 16 | 7 | 16 | 25 | Internal Standard 2 - 2 |
| 18 | 9 | 18 | 27 | Negative hybridization control 4 |
| 20 | 7 | 16 | 25 | Internal Standard 6 - 1 |
| 21 | 3 | 12 | 21 | Internal Standard 3 - 1 |
| 22 | 1 | 10 | 19 | Negative hybridization control 5 |
| 24 | 3 | 12 | 21 | Internal Standard 6 - 2 - |
| 24 | 7 | 16 | 25 | Internal Standard 3 - 2 |
| 25 | 1 | 10 | 19 | Positive hybridization control 2 |
| 25 | 2 | 11 | 20 | Negative detection control 2 |
| 25 | 8 | 17 | 26 | Negative detection control 3 |
| 25 | 9 | 18 | 27 | Positive hybridization control 3 |
| 26 | 1 | 10 | 19 | Positive hybridization control 4 |
| 26 | 2 | 11 | 20 | Detection curve concentration 1 |
| 26 | 3 | 12 | 21 | Detection curve concentration 2 |
| 26 | 4 | 13 | 22 | Detection curve concentration 3 |
| 26 | 5 | 14 | 23 | Detection curve concentration 4 |
| 26 | 6 | 15 | 24 | Detection curve concentration 5 |
| 26 | 7 | 16 | 25 | Detection curve concentration 6 |
| 26 | 8 | 17 | 26 | Detection curve concentration 7 |
| 26 | 9 | 18 | 27 | Detection curve concentration 8 |
| 27 | 1 | 10 | 19 | Detection curve concentration 9 |
| 27 | 2 | 11 | 20 | Detection curve concentration 10 |
| 27 | 3 | 12 | 21 | Negative detection control 4 |
| 27 | 4 | 13 | 22 | Negative detection control 5 |
| 27 | 5 | 14 | 23 | Negative detection control 6 |
| 27 | 6 | 15 | 24 | Negative detection control 7 |
| 27 | 7 | 16 | 25 | Negative detection control 8 |
| 27 | 8 | 17 | 26 | Negative detection control 9 |
| 27 | 9 | 18 | 27 | Negative detection control 10 |

## Figure 2
### Presentation of the pattern of the microarray for gene expression analysis of the Senechip (239 genes) with the appropriated controls

| Row | Column | Gene symbol | Gene |
|-----|--------|-------------|------|
| 1 | 1 | hyb ctl + | **Positive hyb ctl** |
| 1 | 2 | buffer | **Detection neg ctl (buffer)** |
| 1 | 3 | ADAM1 | A disintegrin and metalloproteinase domain1 |
| 1 | 4 | ADPRT | polysynthetase |
| 1 | 5 | buffer | **Detection neg ctl (buffer)** |
| 1 | 6 | ANX1 | Annexin1 |
| 1 | 7 | AOP2 | Anti-oxidant-protein2 |
| 1 | 8 | APOB | ApoliproteinB |
| 1 | 9 | buffer | **Detection neg ctl (buffer)** |
| 1 | 10 | APOE | ApoliproteinE |
| 2 | 1 | buffer | **Detection neg ctl (buffer)** |
| 2 | 2 | APOJ | ApoliproteinJ |
| 2 | 3 | AREG | Amphiregulin |
| 2 | 4 | ATM | Ataxia telangiectasia mutated |
| 2 | 5 | BAT1 | Nuclear-RNA-helicase |
| 2 | 6 | BAX | BCL2-associated X protein |
| 2 | 7 | BCL2 | B-cell lymphoma2 |
| 2 | 8 | BCLX | BCLX |
| 2 | 9 | BMP2 | Bone morphogenetic protein2 |
| 2 | 10 | BRCA2 | Breast cancer2 |
| 3 | 1 | CANX | calnexin |
| 3 | 2 | CASP7 | caspase7 |
| 3 | 3 | IS1 | IS1 |
| 3 | 4 | CASP8 | Caspase8 |
| 3 | 5 | CCNA1 | cyclinA1 |
| 3 | 6 | 23kd | 23KDa Highly basic protein |
| 3 | 7 | CCNB1 | cyclinB1 |
| 3 | 8 | IS4 | IS4 |
| 3 | 9 | CCND1 | cyclinD1 |
| 3 | 10 | buffer | **Detection neg ctl (buffer)** |
| 4 | 1 | CCND2 | cyclinD2 |
| 4 | 2 | CCND3 | cyclinD3 |
| 4 | 3 | buffer | **Detection neg ctl (buffer)** |
| 4 | 4 | CCNE1 | CyclinE |
| 4 | 5 | CCNF | CyclinF |
| 4 | 6 | CCNG | CyclinG |
| 4 | 7 | CCNH | cyclinH |
| 4 | 8 | CDC42 | Cell division cycle42 |
| 4 | 9 | CDK2 | Cyclin dependent kinase2 |
| 4 | 10 | CDK4 | Cyclin dependent kinase4 |
| 5 | 1 | Hyb Ctl - | **Negative hyb CTL** |
| 5 | 2 | CENPA | centromere-protein-A |
| 5 | 3 | CENPF | mitosin |
| 5 | 4 | C-FOS | c-fos |
| 5 | 5 | CKB | creatin-kinase-brain |
| 5 | 6 | COL15A1 | collagenXV-alpha1 |

| 10 | 4 | GRB2 | Growth factor receptor-bound protein2 |
|---|---|---|---|
| 10 | 5 | GSTP1 | Glutathione S-transferase pi |
| 10 | 6 | GSTT1 | Glutathione S-transferase theta1 |
| 10 | 7 | H2B/S | histone2b member B/S consensus |
| 10 | 8 | H3FF | histone3 member F consensus |
| 10 | 9 | H4FM | histone4 member M consensus |
| 10 | 10 | Hyb Ctl - | **Negative hyb CTL** |
| 11 | 1 | HBEGF | Heparin binding epidermal growth factor transcript |
| 11 | 2 | HLF | Hepatic leukemia factor |
| 11 | 3 | HMOX | heme-oxygenase |
| 11 | 4 | HSP27 | Heat shock 27kD protein1 |
| 11 | 5 | HSP40 | Heat shock 40kD protein1 |
| 11 | 6 | HSP70 | Heat shock 70kD protein1 |
| 11 | 7 | HSP70B | Heat shock 70kD protein6 |
| 11 | 8 | cyc | Cyclophilin 33A |
| 11 | 9 | HSP90-alpha | Heat shock 90kD protein1 alpha |
| 11 | 10 | ICAM-1 | Intracellular adhesion molecule1 |
| 12 | 1 | ID1 | Inhibitor of DNA binding1 |
| 12 | 2 | ID2 | Inhibitor of DNA binding2 |
| 12 | 3 | IFNG | Interferon gamma |
| 12 | 4 | IGF1 | Insulin like growth factor1 |
| 12 | 5 | IGF1R | Insulin like growth factor1 receptor |
| 12 | 6 | IGFBP2 | Insulin growth factor binding protein2 |
| 12 | 7 | IGFBP3 | Insulin growth factor binding protein3 |
| 12 | 8 | IGFBP5 | Insulin growth factor binding protein5 |
| 12 | 9 | IL10 | Interleukin 10 |
| 12 | 10 | IL11 | Interleukin 11 |
| 13 | 1 | buffer | **Detection neg ctl (buffer)** |
| 13 | 2 | IL11RA | Interleukin 11-receptor-alpha |
| 13 | 3 | IS2 | IS2 |
| 13 | 4 | IL12 | Interleukin 12 |
| 13 | 5 | IL15 | Interleukin 15 |
| 13 | 6 | GAPD | Glyceraldehyde-3-phosphate-dehydrogenase |
| 13 | 7 | IL1A | Interleukin1 alpha |
| 13 | 8 | IS5 | IS5 |
| 13 | 9 | IL1B | Interleukin1 beta |
| 13 | 10 | buffer | **Detection neg ctl (buffer)** |
| 14 | 1 | IL2 | Interleukin 2 |
| 14 | 2 | IL3 | Interleukin 3 |
| 14 | 3 | buffer | **Detection neg ctl (buffer)** |
| 14 | 4 | IL4 | Interleukin 4 |
| 14 | 5 | IL6 | Interleukin 6 |
| 14 | 6 | IL8 | Interleukin 8 |
| 14 | 7 | INT6 | Translation initiation factor3 subunit6 |
| 14 | 8 | IVL | involucrin |
| 14 | 9 | JNK1 | Mitogen activated protein kinase8 |
| 14 | 10 | JNK2 | Mitogen activated protein kinase9 |

| 15 | 1 | Hyb Ctl - | Negative hyb CTL |
|----|----|----|----|
| 15 | 2 | JNKK1 | Mitogen activated protein kinase kinase 4 |
| 15 | 3 | JUND | Jun D proto-oncogene |
| 15 | 4 | Ki-67 | Ki-67 |
| 15 | 5 | KNSL5 | mitotic-kinesin-like-protein1 |
| 15 | 6 | KNSL6 | mitotic-centromere-associated-kinesin |
| 15 | 7 | KRT1 | keratin1 |
| 15 | 8 | HK1 | Hexokinase 1 |
| 15 | 9 | KRT10 | keratin10 |
| 15 | 10 | KRT14 | keratin14 |
| 16 | 1 | KRT16 | keratin16 |
| 16 | 2 | KRT17 | keratin17 |
| 16 | 3 | HPRT | Hypoxanthine phosphoribosyltransferase 1 |
| 16 | 4 | KRT19 | Keratin19 |
| 16 | 5 | KRT6A | Keratin6 |
| 16 | 6 | L6 | Transmembrane4 superfamily member1 |
| 16 | 7 | MAP17 | Membrane associated protein17 |
| 16 | 8 | MAX | MAX protein |
| 16 | 9 | MCM2 | Mitotin |
| 16 | 10 | MDM2 | MDM2 |
| 17 | 1 | MEK1 | Mitogen activated protein kinase kinase1 |
| 17 | 2 | MEK2 | Mitogen activated protein kinase kinase2 |
| 17 | 3 | MMP1 | matrix metalloproteinase 1 |
| 17 | 4 | MMP10 | matrix metalloproteinase 10 |
| 17 | 5 | MMP11 | matrix metalloproteinase 11 |
| 17 | 6 | MMP12 | matrix metalloproteinase 12 |
| 17 | 7 | MMP13 | matrix metalloproteinase 13 |
| 17 | 8 | MMP14 | matrix metalloproteinase 14 |
| 17 | 9 | MMP15 | matrix metalloproteinase 15 |
| 17 | 10 | MMP2 | matrix metalloproteinase 2 |
| 18 | 1 | buffer | Detection neg ctl (buffer) |
| 18 | 2 | MMP3 | matrix metalloproteinase 3 |
| 18 | 3 | IS5 | IS5 |
| 18 | 4 | MMP7 | matrix metalloproteinase 7 |
| 18 | 5 | MDH | Malate dehydrogenase 1 |
| 18 | 6 | MP1 | Metalloprotease1 |
| 18 | 7 | MSRA | methionine-sulfoxide-reductase A/peptide |
| 18 | 8 | IS2 | IS2 |
| 18 | 9 | MT2A | metallothionein 2A |
| 18 | 10 | buffer | Detection neg ctl (buffer) |
| 19 | 1 | MVK | mevalonate-kinase |
| 19 | 2 | MYBL2 | b-myb |
| 19 | 3 | MYC | c-myc |
| 19 | 4 | NCK1 | NCK adaptor protein1 |
| 19 | 5 | NF1 | neurofibromin1 |
| 19 | 6 | NGFR | nerve growth factor receptor |
| 19 | 7 | NRG1 | neuregulin |
| 19 | 8 | buffer | Detection neg ctl (buffer) |

| 19 | 9 | ODC | Ornithine decarboxylase1 |
|---|---|---|---|
| 19 | 10 | OPG | osteoprotegerin |
| 20 | 1 | OPN | osteopontin |
| 20 | 2 | Oste | osteonectin |
| 20 | 3 | p16 | Cyclin dependent kinase inhibitor 2A |
| 20 | 4 | p21 | Cyclin dependent kinase inhibitor 1A |
| 20 | 5 | p27 | Cyclin dependent kinase inhibitor 1B |
| 20 | 6 | p35 | Cyclin dependent kinase5 regulatory subunit1 |
| 20 | 7 | p53 | Tumor protein p53 |
| 20 | 8 | p57 | Cyclin dependent kinase inhibitor 1C |
| 20 | 9 | PAI1 | plasminogen activator inhibitor type1 |
| 20 | 10 | Hyb Ctl - | Negative hyb CTL |
| 21 | 1 | PAI2 | plasminogen activator inhibitor type2 |
| 21 | 2 | PAK | P21 activated kinase1 |
| 21 | 3 | PCNA | Proliferating cell nuclear antigen |
| 21 | 4 | PKM2 | pyruvate-kinase-muscle |
| 21 | 5 | PLAU | urokinase |
| 21 | 6 | PLAUR | urokinase-receptor |
| 21 | 7 | PLK | Polo-like kinase |
| 21 | 8 | PLA2 | Phospholipase A2 |
| 21 | 9 | POLA2 | Polymerase alpha |
| 21 | 10 | PRSS11 | Protease serine11 |
| 22 | 1 | PSMA2 | proteasome (prosome, macropain) subunit, alpha type, 2 |
| 22 | 2 | PSMA3 | proteasome (prosome, macropain) subunit, alpha type, 3 |
| 22 | 3 | PSMC6 | proteasome (prosome, macropain) 26S subunit, non-ATPase, 6 |
| 22 | 4 | PSMD1 | proteasome (prosome, macropain) 26S subunit, non-ATPase, 1 |
| 22 | 5 | PSMD11 | proteasome (prosome, macropain) 26S subunit, non-ATPase, 11 |
| 22 | 6 | PSMD12 | proteasome (prosome, macropain) 26S subunit, non-ATPase, 12 |
| 22 | 7 | PSOR1 | psoriasin |
| 22 | 8 | RAF1 | c-raf-1 |
| 22 | 9 | RANTES | Small inducible cytokine A5 |
| 22 | 10 | RB1 | Retinoblastome1 |
| 23 | 1 | buffer | Detection neg ctl (buffer) |
| 23 | 2 | RET | ret protooncogene |
| 23 | 3 | IS3 | IS3 |
| 23 | 4 | ROR1 | R AR related orphan receptorA |
| 23 | 5 | RPL3 | 60S-ribosomal-proteinL3 |
| 23 | 6 | S9 | Ribosomal Proteine S9 |
| 23 | 7 | RPS10 | ribosomal-protein S10 |
| 23 | 8 | IS6 | IS6 |
| 23 | 9 | RRAS | R-ras |
| 23 | 10 | buffer | Detection neg ctl (buffer) |

| 24 | 1 | RRM1 | ribonucleotide-reductase M1 |
|----|----|------|------|
| 24 | 2 | S100A10 | Calpactin1 |
| 24 | 3 | buffer | Detection neg ctl (buffer) |
| 24 | 4 | S100A11 | Calgizzarin |
| 24 | 5 | S100A8 | calprotectin |
| 24 | 6 | SHC | SHC transforming protein1 |
| 24 | 7 | SLK | Ste-20-related serine/threonine kinase |
| 24 | 8 | SLP2 | Stomatin like protein2 |
| 24 | 9 | SM22 | transgelin |
| 24 | 10 | SMAD1 | Mother against decapentalplegic homol1 |
| 25 | 1 | Hyb Ctl - | Negative hyb CTL |
| 25 | 2 | SNCG | synuclein |
| 25 | 3 | SDS | Serine Dehydratase |
| 25 | 4 | SOD2 | Superoxide dismutase2 |
| 25 | 5 | SPRR1B | cornifin |
| 25 | 6 | SRI | sorcin |
| 25 | 7 | STAT5 | Signal transducer and activator of transcription 5A |
| 25 | 8 | TBXA2R | Thromboxane-A2-receptor |
| 25 | 9 | TERC | telomerase-RNA |
| 25 | 10 | TERT | telomerase-reverse transcriptase |
| 26 | 1 | TFAP2A | Transcription factor AP2-alpha |
| 26 | 2 | TFAP2B | Transcription factor AP2-beta |
| 26 | 3 | TFAP2C | Transcription factor AP2-gamma |
| 26 | 4 | TGFA | TGF-alpha |
| 26 | 5 | TGFB1 | TGF-beta1 |
| 26 | 6 | TGFBR2 | TGF-beta-R2 |
| 26 | 7 | TGM1 | transglutaminase1 |
| 26 | 8 | TH | Tyrosine-hydroxylase |
| 26 | 9 | THBS1 | Thrombospondin |
| 26 | 10 | TIMP1 | Tissue inhibitor of metalloproteinase1 |
| 27 | 1 | TIMP2 | Tissue inhibitor of metalloproteinase2 |
| 27 | 2 | TK1 | thymidine-kinase |
| 27 | 3 | IS6 | IS6 |
| 27 | 4 | TNFA | tumor necrosis factor alpha |
| 27 | 5 | TFR | Transferrin receptor |
| 27 | 6 | TNFB | tumor necrosis factor beta |
| 27 | 7 | TNFRSF1A | TNF-alpha-RI |
| 27 | 8 | IS3 | IS3 |
| 27 | 9 | TNFRSF1B | TNF-alpha-RII |
| 27 | 10 | buffer | Detection neg ctl (buffer) |
| 28 | 1 | hyb ctl + | Positive hyb ctl |
| 28 | 2 | buffer | Detection neg ctl (buffer) |
| 28 | 3 | TOP2A | topoisomerase2-alpha |
| 28 | 4 | TPA | Plasminogen activator tissue |
| 28 | 5 | TRF1 | Telomeric repeat binding factor1 |
| 28 | 6 | TYMS | thymidylate-synthetase |
| 28 | 7 | UBE2C | Ubiquitin conjugating enzyme E2C/ubiquitin carrier protein |

| 28 | 8 | buffer | Detection neg ctl (buffer) |
|---|---|---|---|
| 28 | 9 | VEGFC | Vascular endothelial growth factor C |
| 28 | 10 | VEGFR1 | Vascular endothelial growth factor receptor1 |
| 29 | 1 | buffer | Detection neg ctl (buffer) |
| 29 | 2 | buffer | Detection neg ctl (buffer) |
| 29 | 3 | buffer | Detection neg ctl (buffer) |
| 29 | 4 | 1 ctl + | Positive detection ctl |
| 29 | 5 | 2 ctl + | Positive detection ctl |
| 29 | 6 | 3 ctl + | Positive detection ctl |
| 29 | 7 | 4 ctl + | Positive detection ctl |
| 29 | 8 | 5 ctl + | Positive detection ctl |
| 29 | 9 | 6 ctl + | Positive detection ctl |
| 29 | 10 | 7 ctl + | Positive detection ctl |
| 30 | 1 | 8 ctl + | Positive detection ctl |
| 30 | 2 | 9 ctl + | Positive detection ctl |
| 30 | 3 | 10 ctl + | Positive detection ctl |
| 30 | 4 | 11 ctl + | Positive detection ctl |
| 30 | 5 | buffer | Detection neg ctl (buffer) |
| 30 | 6 | buffer | Detection neg ctl (buffer) |
| 30 | 7 | buffer | Detection neg ctl (buffer) |
| 30 | 8 | buffer | Detection neg ctl (buffer) |
| 30 | 9 | buffer | Detection neg ctl (buffer) |
| 30 | 10 | buffer | Detection neg ctl (buffer) |

Fig. 3

Fig. 4

Fig. 5

EP 1 477 571 A1

Fig. 6

50

**European Patent Office**

## EUROPEAN SEARCH REPORT

Application Number

EP 04 01 0205

### DOCUMENTS CONSIDERED TO BE RELEVANT

| Category | Citation of document with indication, where appropriate, of relevant passages | Relevant to claim | CLASSIFICATION OF THE APPLICATION (Int.Cl.7) |
|---|---|---|---|
| X | WO 03/029273 A (WHITEHEAD BIOMEDICAL INST ; STAUNTON JANE (US); GOLUB TODD (US); BHATT) 10 April 2003 (2003-04-10) * the whole document * * page 5, line 7, paragraph 2 - line 13; claims 1-42; tables 1-8 * ----- | 1-31 | C12Q1/68 |
| Y | WO 98/30722 A (MACK DAVID H) 16 July 1998 (1998-07-16) * the whole document * * claims 1-108 * ----- | 1-31 | |
| D,Y | WO 02/46467 A (BERTUCCI FRANCOIS ; IPSOGEN (FR); VIENS PATRICE (FR); BIRNBAUM DANIEL) 13 June 2002 (2002-06-13) * the whole document * ----- | 1-31 | |
| D,Y | US 6 183 968 B1 (REDDY ROOPA ET AL) 6 February 2001 (2001-02-06) * the whole document * ----- | 1-31 | |
| | | | TECHNICAL FIELDS SEARCHED (Int.Cl.7) |
| | | | C12Q |

The present search report has been drawn up for all claims

| Place of search | Date of completion of the search | Examiner |
|---|---|---|
| Munich | 3 June 2004 | Sprinks, M |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

........................................................................
& : member of the same patent family, corresponding document

EPO FORM 1503 03.82 (P04C01)

51

## ANNEX TO THE EUROPEAN SEARCH REPORT
## ON EUROPEAN PATENT APPLICATION NO.

EP 04 01 0205

This annex lists the patent family members relating to the patent documents cited in the above-mentioned European search report.
The members are as contained in the European Patent Office EDP file on
The European Patent Office is in no way liable for these particulars which are merely given for the purpose of information.

03-06-2004

| Patent document cited in search report | | Publication date | Patent family member(s) | | Publication date |
|---|---|---|---|---|---|
| WO 03029273 | A | 10-04-2003 | WO | 03029273 A2 | 10-04-2003 |
| | | | US | 2004009489 A1 | 15-01-2004 |
| WO 9830722 | A | 16-07-1998 | AU | 6035698 A | 03-08-1998 |
| | | | EP | 0973939 A1 | 26-01-2000 |
| | | | JP | 2001508303 T | 26-06-2001 |
| | | | US | 6303301 B1 | 16-10-2001 |
| | | | WO | 9830722 A1 | 16-07-1998 |
| | | | US | 2004058376 A1 | 25-03-2004 |
| | | | US | 2002028454 A1 | 07-03-2002 |
| | | | US | 2002039739 A1 | 04-04-2002 |
| WO 0246467 | A | 13-06-2002 | US | 2003143539 A1 | 31-07-2003 |
| | | | AU | 3479902 A | 18-06-2002 |
| | | | CA | 2430981 A1 | 13-06-2002 |
| | | | EP | 1353947 A2 | 22-10-2003 |
| | | | WO | 0246467 A2 | 13-06-2002 |
| US 6183968 | B1 | 06-02-2001 | NONE | | |

EPO FORM P0459

For more details about this annex : see Official Journal of the European Patent Office, No. 12/82